# EUROPEAN PATENT SPECIFICATION

(11) **EP 3 412 772 B1**
(45) Date of publication and mention of the grant of the patent: **04.05.2022**
(21) Application number: 17747603.3
(22) Date of filing: 03.02.2017
(51) Int. Cl.: C12N 15/09, A01K 67/033, A01K 67/04, C12P 19/00, C12N 5/10, C12N 9/00, C12N 9/10, C12N 9/12, C12N 15/85

(54) **TRANSGENIC SILKWORM HAVING MAMMALIAN-TYPE SUGAR CHAIN ATTACHED THERETO**
TRANSGENE SEIDENRAUPE MIT DARAN GEBUNDENER SÄUGETIERARTIGER ZUCKERKETTE
VER À SOIE TRANSGÉNIQUE AUQUEL EST FIXÉE UNE CHAÎNE DE SUCRE DE TYPE MAMMIFÈRE

(30) Priority: 05.02.2016 JP 2016021352
(43) Date of publication of application: 12.12.2018
(73) Proprietor: Osaka University, Suita-shi, Osaka 565-0871 (JP)
(72) Inventor: FUJIYAMA Kazuhito, Suita-shi Osaka 565-0871 (JP); MISAKI Ryo, Suita-shi Osaka 565-0871 (JP); TATEMATSU Ken-ichiro, Tsukuba-shi Ibaraki 305-8602 (JP); SEZUTSU Hideki, Tsukuba-shi Ibaraki 305-8602 (JP)
(74) Representative: D Young & Co LLP
(86) International application number: PCT/JP2017/004105
(87) International publication number: WO 2017/135452

(56) References cited:
- WO-A1-2005/042753
- WO-A1-2005/042753
- WO-A1-2014/104269
- JP-A- 2006 137 739
- JP-A- 2008 131 940
- JP-A- 2009 225 781
- SAUMYA WICKRAMASINGHE ET AL: "Primer on genes encoding enzymes in sialic acid metabolism in mammals", BIOCHIMIE, MASSON, PARIS, FR, vol. 93, no. 10, 3 June 2011 (2011-06-03), pages 1641-1646, XP028271166, ISSN: 0300-9084, DOI: 10.1016/J.BIOCHI.2011.06.002 [retrieved on 2011-06-12]
- VISWANATHAN K ET AL: "Engineering sialic acid synthetic ability into insect cells: identifying metabolic bottlenecks and devising strategies to overcome them", BIOCHEMISTRY, ACS PUBLICATIONS, vol. 42, no. 51, 30 December 2003 (2003-12-30), pages 15215-15225, XP002334628, ISSN: 0006-2960, DOI: 10.1021/BI034994S
- ALEXANDRA CASTILHO ET AL: "Construction of a Functional CMP-Sialic Acid Biosynthesis Pathway in Arabidopsis", PLANT PHYSIOLOGY, vol. 147, no. 1, 7 March 2008 (2008-03-07) , pages 331-339, XP055634853, Rockville, Md, USA ISSN: 0032-0889, DOI: 10.1104/pp.108.117572
- H. MABASHI-ASAZUMA ET AL: "Impact of a human CMP-sialic acid transporter on recombinant glycoprotein sialylation in glycoengineered insect cells", GLYCOBIOLOGY, vol. 23, no. 2, 12 October 2012 (2012-10-12), pages 199-210, XP055633543, US ISSN: 0959-6658, DOI: 10.1093/glycob/cws143
- JARED J AUMILLER ET AL: "A new glycoengineered insect cell line with an inducibly mammalianized protein N-glycosylation pathway", GLYCOBIOLOGY, vol. 22, no. 3, 31 October 2011 (2011-10-31), pages 417-428, XP055632810, US ISSN: 0959-6658, DOI: 10.1093/glycob/cwr160
- AUMILLER JARED J ET AL: "A transgenic insect cell line engineered to produce CMP-sialic acid and sialylated glycoproteins", GLYCOBIOLOGY, OXFORD UNIVERSITY PRESS, US, vol. 13, no. 6, 20 February 2003 (2003-02-20), pages 497-507, XP002320026, ISSN: 0959-6658, DOI: 10.1093/GLYCOB/CWG051
- XU HANFU ED - MURRAY JAMES D ET AL: "The advances and perspectives of recombinant protein production in the silk gland of silkworm Bombyx mori", TRANSGENIC RESEARCH, SPRINGER NETHERLANDS, NL, vol. 23, no. 5, 12 August 2014 (2014-08-12), pages 697-706, XP035381276, ISSN: 0962-8819, DOI: 10.1007/S11248-014-9826-8 [retrieved on 2014-08-12]
- Hinderlich S., Weidemann W., Yardeni T., Horstkorte R., Huizing M. (2013): "UDP-GlcNAc 2-Epimerase/ManNAc Kinase (GNE): A Master Regulator of Sialic Acid Synthesis" In: "SialoGlyco Chemistry and Biology I. Topics in Current Chemistry 2015, Springer, Berlin, Heidelberg", 11 July 2013 (2013-07-11), SPRINGER, BERLIN., DE, XP055634318, ISSN: 0340-1022 vol. 366, pages 97-137, DOI: 10.1007/128_2013_464, * the whole document *
- HIROYUKI KAJIURA ET AL: "Sialylation potentials of the silkworm, Bombyx mori ; B. mori possesses an active [alpha]2,6-sialyltransferase", GLYCOBIOLOGY, vol. 25, no. 12, 24 August 2015 (2015-08-24), pages 1441-1453, XP055634345, US ISSN: 0959-6658, DOI: 10.1093/glycob/cwv060
- JARVIS D L ET AL: "Engineering N-glycosylation pathways in the baculovirus-insect cell system", CURRENT OPINION IN BIOTECHNOLOGY, LONDON, GB, vol. 9, no. 5, 1 October 1998 (1998-10-01) , pages 528-533, XP008123541, ISSN: 0958-1669, DOI: 10.1016/S0958-1669(98)80041-4 [retrieved on 2002-02-11]
- KOHJI ITOH ET AL: "Recent progress in development of transgenic silkworms overexpressing recombinant human proteins with therapeutic potential in silk glands", DD&T DRUG DISCOVERIES & THERAPEUTICS, vol. 10, no. 1, 26 February 2016 (2016-02-26), pages 34-39, XP055634362, Japan ISSN: 1881-7831, DOI: 10.5582/ddt.2016.01024
- TATSUYA KATO ET AL: "N-Glycan Modification of a Recombinant Protein via Coexpression of Human Glycosyltransferases in Silkworm Pupae", SCIENTIFIC REPORTS, vol. 7, no. 1, 3 May 2017 (2017-05-03), XP055472157, DOI: 10.1038/s41598-017-01630-6
- SUGANUMA MASATOSHI ET AL: "N-glycan sialylation in a silkworm-baculovirus expression system", JOURNAL OF BIOSCIENCE AND BIOENGINEERING, ELSEVIER, AMSTERDAM, NL, vol. 126, no. 1, 9 February 2018 (2018-02-09), pages 9-14, XP085409658, ISSN: 1389-1723, DOI: 10.1016/J.JBIOSC.2018.01.007
- TSUYOSHI NOMURA et al.: "Kaiko-Baculovirus Hatsugen-kei o Mochiita To Tanpakushitsu no Hatsugen to Tosa Kozo no Kaihen", Journal of the Society for Bioscience and Bioengineering, vol. 93, no. 6, 2015, pages 341-344, XP009512214, Japan ISSN: 0919-3758
- RIE SUGIMURA et al.: "2I001 Kaiko Kenshisen de Hatsugen saseta Interferon y no N-gata Tosa Kozo Kaiseki = N-Glycan analysis of interferon gamma expressed in the silk gland of silkworm", PROCEEDINGS ( ONLINE ) OF THE 2016 ANNUAL MEETING OF JAPAN SOCIETY FOR BIOSCIENCE, BIOTECHNOLOGY, AND AGROCHEMISTRY, 3 May 2016 (2016-05-03), page 796, XP009512442, ISSN: 2186-7976

### SEQUENCE LISTING

<110> Osaka University National Agriculture and Food Research Organization
<120> Transgenic silk worm having mammalian-type glycosylations
<130> PH-6819-PCT
<150> JP 2016-021352
   <151> 2016-02-05
<160> 99
<170> PatentIn version 3.5
<210> 1
   <211> 705
   <212> DNA
   <213> Bombyx mori
<220>
   <223> sericin 1 promoter
<400> 1
   ctttgtatcc ctttttacga aaattgcgag gacggaggag tatgaaattt cccacactta 60
   tagagaatac agagaagaag tgcacaatgc taatattttt ttaaaataat gcataaaaga 120
   tactttaaat caataaagaa aacagcacac acactacata ccatgtattt gacgcacaca 180
   cgcatgtata ctatttattg tcaaactttt gttcttgacg tctgtgttca aactgagaat 240
   agattaaata ttgtttgtct ttattaatat tttttaatag tgtagtcttg gcgaaatttg 300
   tgattataga agtataaaat acaatcataa tagtgtacaa acttacaatt ccaattaatt 360
   atagtcgaat ttcgactact gcgggacctc tagtattaat aattctcttt aaaaaaaaac 420
   agagcatcaa atactgcaca aatgtcaagc gggtctcaac gagccatgaa taaattagaa 480
   atcaattaat aacataaaat aggcaaacaa aataaaacca tttacataga gaacgtttgt 540
   tgaacaaaaa caataacttg tatacattgt ttgcacaaat gtttgaaccg aaaatttatt 600
   actctctacg taagcttgat caaacttcgt tttcgtataa aacgcgttgg cccaaccact 660
   ttggcatagt cgtcttatca tcgggtctct aaggatcaag cgatc 705
<210> 2
   <211> 2000
   <212> DNA
   <213> Bombyx mori
<220>
   <223> sericin 2 promoter
<400> 2
   tggaaggata taataattat ttttttttgc tatgaaaaat aataacttgt acttactccg 60
   ataagtcttt ttatttttta ttgcttagat gggtggacga gctcacagac tacctggtgc 120
   tgagtggtta ccggagccca tagacacaac gtaaatgcgc cacccacctt gagatataag 180
   ttctaaggtc taagtgtagt tacaacggct gccccacctt tcaaaccgaa acgcattact 240
   gcttaatggt agaaatgggc agtttgtaaa gagatgatca aagctgtagg ctgattttta 300
   tctagtttca gtattattaa gcattgagta aattaataaa gttacggact aagtttcatc 360
   gaagaacatt ctagtttttt tcctacctat tctgatagcc tccggaggct atttcagctt 420
   ctccttggtg tgtaggtgat ctcacggggc tcaaaccggg agtgttgcta acattggccc 480
   tagcaagagc agtacttcgc agaatctacc acgatcggaa acgcgaccca ctgagaagat 540
   ccggcgagaa actcagtgag ctgtgtctat gggttaattt actcgtcgag ccctgtttac 600
   tgtttagggc gacgtcgact gttaccattc ggtctacagg atcgagtgtg cattcttgta 660
   tcatcgttct attatcacga gtcattttgc gttttttcgg atcccctgga agtcgtcgtg 720
   gcctaagaga taagaagtcc ggtgcattcg tgttgagcga tgcacctgtg ttcgaatcct 780
   aggcgggtac caatttttct aatgaattac gtacccaaca aatgttcacg attgccttcc 840
   acggtgaagg aataacatcg tgcaataaaa gtgaaacccg caaaatccgg tgcttttaag 900
   cttttcaagc accggtcacc atcctcgttg aactcatcga tctacaagcg atctaatcta 960
   tagacccaat ccactaagat ctcaccggat cttctcagtg gttcgcattc cagtggtaga 1020
   ttcaattcgc tgctcttgct agggctagtg ttagcaaatt ccttcgggtt aagcccgaga 1080
   gctcacctat ccgtccgcgc taagctggaa aagcccctta agctgttttt tttttgtata 1140
   gcctttattg ctaatactaa acaataacta ataattttac atacagtaac aaattgtttt 1200
   aacttaaatc taatacatcg gatttcccgg ttcagtgatc agcgtgtcct gtgacacata 1260
   ggcctcttcc agctgctttc atttttctct attggtagct tttcttgacc agattgtctc 1320
   tccaatcatc ttgatatcgt ctgtccatct tctagcttgc ctggctcttt tcctttaaac 1380
   caggggtcgt gaatttcaat cctcacagga agccgggatt aggtgggaga atatagttcc 1440
   gatgttttga atgctttata ttttctgtgg tcgaaaatga tactagagct acgcgtcgac 1500
   aattgaatat tatgctaact accctctatt tattaaaaga cttttacgat tcatttcgca 1560
   cagaaccaat cgactgggtt tagaggttta gcagtttgtt gaatgaactc gttttcatct 1620
   tcacgattag aggatcccag gtgttaggta aaggatattc tagattgcag gagatttttc 1680
   ataaataatc acgcgatgga gcggtaatca gccaacatag tcgatcggca tcattattgg 1740
   agaccaaaca acacttcagt tatccaagcg cgtcttaagt cgcattcgga taatcttgaa 1800
   tagcctggaa gtgaattttt aaaaagtttg tctcgaacaa acatcaatta ctttgtaatt 1860
   gaaccgaaaa aagaggataa acattattag cattcgttgt aatgaaatat aatgttgaca 1920
   cagtttgacc gacgtgcact gtcttttgtg gcaccggcta tataaaggtg gtctgtccgt 1980
   tctgagccac acgagtcatc 2000
<210> 3
   <211> 578
   <212> DNA
   <213> Bombyx mori
<220>
   <223> sericin 3 promoter
<400> 3
   gcggagggac tttagttctc tctgcattct gtacggtatg tggagagttt ctaccatcgt      60
   accgcccgcc accagagtag agttcatcca tactacctgg agccactgcg ttcatccaca     120
   gtgcgtttcc agagatcttt tttgccacat accatccggc tttggaatga gctcccctcc     180
   acggtgcttc aaacgaggct tgtggagagt acttaacggt aggttggctt ggctaggctt     240
   gactctgccc ctggcattgc tgaagttcat ggacgacggt aaccacttac catcaggtgg     300
   gccgtatgct aatccgacta caagggcaac agcaaaaaaa gttaatttta caaaagtatc     360
   ataattccga ggctcgctaa agatgttcgt agcatatgtt acagaaacaa aaaaaaaaac     420
   aatttaaatg cgttatagaa aaacaagtgt attaaacaaa taattaatta tttattttat     480
   tggtaactgt ttattcataa agggaatatt tctccaacaa attagtataa atagccggcc     540
   tttgggcgtt tacagacaga gcaatcgaag cttcgaag                             578
<210> 4
   <211> 870
   <212> DNA
   <213> Bombyx mori
<220>
   <223> Fib H promoter
<400> 4
   acaaaactgc cacacgcatt tttttctcca ctgtaggttg tagttacgcg aaaacaaaat    60
   cgttctgtga aaattcaaac aaaaatattt tttcgtaaaa acacttatca atgagtaaag   120
   taacaattca tgaataattt catgtaaaaa aaaaatacta gaaaaggaat ttttcattac   180
   gagatgctta aaaatctgtt tcaaggtaga gatttttcga tatttcggaa aattttgtaa   240
   aactgtaaat ccgtaaaatt ttgctaaaca tatattgtgt tgttttggta agtattgacc   300
   caagctatca cctcctgcag tatgtcgtgc taattactgg acacattgta taacagttcc   360
   actgtattga caataataaa acctcttcat tgacttgaga atgtctggac agatttggct   420
   ttgtattttt gatttacaaa tgtttttttg gtgatttacc catccaaggc attctccagg   480
   atggttgtgg catcacgccg attggcaaac aaaaactaaa atgaaactaa aaagaaacag   540
   tttccgctgt cccgttcctc tagtgggaga aagcatgaag taagttcttt aaatattaca   600
   aaaaaattga acgatattat aaaattcttt aaaatattaa aagtaagaac aataagatca   660
   attaaatcat aattaatcac attgttcatg atcacaattt aatttacttc atacgttgta   720
   ttgttatgtt aaataaaaag attaatttct atgtaattgt atctgtacaa tacaatgtgt   780
   agatgtttat tctatcgaaa gtaaatacgt caaaactcga aaattttcag tataaaaagg   840
   ttcaactttt tcaaatcagc atcagttcgg                                     870
<210> 5
   <211> 634
   <212> DNA
   <213> Bombyx mori
<220>
   <223> Fib L promoter
<400> 5
   ggtacggttc gtaaagttca cctgcggcta tattcagact cgccaagtta cgtcagtcgt      60
   attgtaatga gcgatttagt gggcaacttc attctgttaa ttttgtgtca cggtgcgcgc     120
   gcatcgtaaa atttcactct catagatttt tcataacgtg cctaaagaag tataacttca     180
   ataatttaaa ttaaaaaaaa acatgcatag aataattata tgaattattt aaaatgtcat     240
   ttaccgacat tgacataaca gacgacgtta acactacaaa acattttaat tccacattgc     300
   tacatattca acagttaaat ttgcgttaat tctcgatgcg aacaaatata agaacaatcg     360
   gatcaattag atcgctttgt ttcgagcaac acttagttta actagaggcg tacacctcaa     420
   gaaatcatct tcattagaaa ctaaacctta aaatcgcatt aataaagcat agtcaatttt     480
   aactgaaatg caaaatcttt tgaacgttag atgctgtcag cgttcgttgg tacagttgtt     540
   tgatatttat tttaattgtc tttttatata taaatagtgg aacattaatc acggaatcct     600
   gtatagtata taccgattgg tcacataaca gacc                                 634
<210> 6
   <211> 1344
   <212> DNA
   <213> Bombyx mori
<220>
   <223> p25 promptor
<400> 6
   aagcttagat aattcggcat tgtgcgccac tgagtcgcat tatgctctgt aattggaaac      60
   taccaaacat tgtgtaccct ttaatgatat tctaatctat atatataaaa atgaattgct     120
   gttcgttagt ctcgctaaaa ctcgagaacg gccggaccga tttggctaat tttggtcttg     180
   aattatttgt ggaagtccag agaagattta gaaggtttaa ataaatatga aaatgctcgg     240
   aattaaataa aaataacaat tttgtttttt ctttgatgtg ttcccgtcgg acggattcct     300
   ttagtctttt atttatcgac tagcgacccg ccgcttcgct tcggaaacat taaaatacac     360
   atgataccaa aaaaattaaa taattttttt ttaaaaaaag tagcctatgt tcatcaggta     420
   caatgtcggc ttctaatgga aaaagaattt ttcaaatcgg tccagtagtt tcggagccta      480
   ttcgaaacaa acaaacaaac aaatctttcc tctttataat attagtatag atagtataga      540
   ttgaggcact acgaagtctg ccgggtcagc tagtatactc ataaataagg tcgacatctg      600
   ttgatgatgg tgatatcttc aaaattacct tagcgcaatg tagacttata cagtatttct      660
   gttttcctaa gttaattacc gctgtagcca ataccgtctt taccataagc gcacacgggg      720
   cccggtccag ggccgagtgt cgtcgagggg gcccgaaaga ccggcaagtt ctctcacacg      780
   tttattccca aaacattttt gtcgggcaca ttacactttt tccacaaatc cgtaatcaga      840
   aggtatttag caaggcatat actatgccta taatagaaga ttttgctcaa cagaaatccc      900
   gagagaaacc gttatcgaaa tcgtaaccaa aaaaccagca gcattctaat atcattaatg      960
   acatattata tcatactgta tttgattacc tataataaag ggtcatactc agtaaaaaaa     1020
   tgttaatata attcgctttt tttactttcc aaaagggcct caaattcttg tgtgtccaag     1080
   ggccccatct tagtttaaga cgtccctggc tgtagcccag ttactgccac acaaacatgc     1140
   ttaactcgcg ccgcctacgt cgaggagaac attttgcgcc ttagaaaata aaatggcgtc     1200
   gccgcggcgc aacaataaga acttaattcg tgcaattgtt tccacgacgc tatttattta     1260
   acgttattcg ttgtgaggaa caatactttg tataattaat gttgatcagt gcctaacgac     1320
   gcagttgttt attattcgcg caac                                            1344
<210> 7
   <211> 1110
   <212> DNA
   <213> Antheraea pernyi
<220>
   <223> Fib H promoter
<400> 7
   tccagcgtta ccaatgagag cgcttcaaaa ttctttacaa cttcattaga atacgtcgat      60
   ttttctctac ttcatataaa tattctatag atgtgtttgc tataacataa atacttttaa     120
   aaaaatgtct caacggttgt gaaaactgtc aaaatctgtt gcgtagttca gaaaaactaa     180
   ggaaacatac agaaaattta ttttacaaaa gtacggagat atataaaaat atttcgatta     240
   ctttagaatt acaataaaac tatttgacaa tttgattgca aatatagacc atgacaacac     300
   cacatctttg ttatctaaaa cacgtagcga caacactcct tgaacgttgt tcgaggatta     360
   ctacgataat tggcggtttt ttttccgcac cgcaagaaaa gagtagaaat gtaccgtatt     420
   taaatccagt gcggaaattt tcacgcagaa tgcgtttcca tacaattcta taggttacat     480
   atcttgcgga aataaattcg tgccaaaaag ccgaagtgcg gggactaata aagattttat     540
   ttggcattcc ttctaacctt tagatataaa tttctgtacg cgcgtatgtc actgaactcc     600
   ccctaaacgg ctggactaat tttgatgaaa tattgtttgt gtgttctagt ggatccgaga     660
   attgtttaaa ttcgcaaatc cggtaggtga acccgcggtt gacttttaga ttttttttat     720
   tatcaacaac aacgtccgcc cggcccgcta gttatgtatg tatttgtaaa tgtaatctca     780
   aaccgttcct gttggatcga catttaatat gtttaagtga attaattaac gtataacagt     840
   cataagaaaa tattgcaata aaatcccatc atttattctt tagagacaat ataaccaaac     900
   aacaataaga atcagaatgt aattactcta cattgttcat gataggggtt taactatgat     960
   attgttttaa ttctatagga ttcattactt tatcattttg tcaatattta aaattgttta    1020
   tttgaaatag ttaacgacat tacaaagttt tcgtataaaa gggcgccaaa gtctggtctc    1080
   attatcagtt cggttccagc tctcataacc                                     1110
<210> 8
   <211> 1110
   <212> DNA
   <213> Antheraea pernyi
<220>
   <223> Fib L promoter
<400> 8
   tccagcgtta ccaatgagag cgcttcaaaa ttctttacaa cttcattaga atacgtcgat      60
   ttttctctac ttcatataaa tattctatag atgtgtttgc tataacataa atacttttaa     120
   aaaaatgtct caacggttgt gaaaactgtc aaaatctgtt gcgtagttca gaaaaactaa     180
   ggaaacatac agaaaattta ttttacaaaa gtacggagat atataaaaat atttcgatta     240
   ctttagaatt acaataaaac tatttgacaa tttgattgca aatatagacc atgacaacac     300
   cacatctttg ttatctaaaa cacgtagcga caacactcct tgaacgttgt tcgaggatta     360
   ctacgataat tggcggtttt ttttccgcac cgcaagaaaa gagtagaaat gtaccgtatt     420
   taaatccagt gcggaaattt tcacgcagaa tgcgtttcca tacaattcta taggttacat     480
   atcttgcgga aataaattcg tgccaaaaag ccgaagtgcg gggactaata aagattttat     540
   ttggcattcc ttctaacctt tagatataaa tttctgtacg cgcgtatgtc actgaactcc     600
   ccctaaacgg ctggactaat tttgatgaaa tattgtttgt gtgttctagt ggatccgaga     660
   attgtttaaa ttcgcaaatc cggtaggtga acccgcggtt gacttttaga ttttttttat     720
   tatcaacaac aacgtccgcc cggcccgcta gttatgtatg tatttgtaaa tgtaatctca     780
   aaccgttcct gttggatcga catttaatat gtttaagtga attaattaac gtataacagt     840
   cataagaaaa tattgcaata aaatcccatc atttattctt tagagacaat ataaccaaac     900
   aacaataaga atcagaatgt aattactcta cattgttcat gataggggtt taactatgat      960
   attgttttaa ttctatagga ttcattactt tatcattttg tcaatattta aaattgttta    1020
   tttgaaatag ttaacgacat tacaaagttt tcgtataaaa gggcgccaaa gtctggtctc    1080
   attatcagtt cggttccagc tctcataacc                                     1110
<210> 9
   <211> 398
   <212> PRT
   <213> Homo sapiens
<220>
   <223> GalT1
<400> 9
   Met Arg Leu Arg Glu Pro Leu Leu Ser Gly Ser Ala Ala Met Pro Gly
   1               5                   10                  15
   Ala Ser Leu Gln Arg Ala Cys Arg Leu Leu Val Ala Val Cys Ala Leu
               20                  25                  30
   His Leu Gly Val Thr Leu Val Tyr Tyr Leu Ala Gly Arg Asp Leu Ser
           35                  40                  45
   Arg Leu Pro Gln Leu Val Gly Val Ser Thr Pro Leu Gln Gly Gly Ser
       50                  55                  60
   Asn Ser Ala Ala Ala Ile Gly Gln Ser Ser Gly Glu Leu Arg Thr Gly
   65                  70                  75                  80
   Gly Ala Arg Pro Pro Pro Pro Leu Gly Ala Ser Ser Gln Pro Arg Pro
                   85                  90                  95
   Gly Gly Asp Ser Ser Pro Val Val Asp Ser Gly Pro Gly Pro Ala Ser
               100                 105                 110
   Asn Leu Thr Ser Val Pro Val Pro His Thr Thr Ala Leu Ser Leu Pro
           115                 120                 125
   Ala Cys Pro Glu Glu Ser Pro Leu Leu Val Gly Pro Met Leu Ile Glu
       130                 135                 140
   Phe Asn Met Pro Val Asp Leu Glu Leu Val Ala Lys Gln Asn Pro Asn
   145                 150                 155                 160
   Val Lys Met Gly Gly Arg Tyr Ala Pro Arg Asp Cys Val Ser Pro His
                   165                 170                 175
   Lys Val Ala Ile Ile Ile Pro Phe Arg Asn Arg Gln Glu His Leu Lys
               180                 185                 190
   Tyr Trp Leu Tyr Tyr Leu His Pro Val Leu Gln Arg Gln Gln Leu Asp
                   195                 200                 205
   Tyr Gly Ile Tyr Val Ile Asn Gln Ala Gly Asp Thr Ile Phe Asn Arg
       210                 215                 220
   Ala Lys Leu Leu Asn Val Gly Phe Gln Glu Ala Leu Lys Asp Tyr Asp
   225                 230                 235                 240
   Tyr Thr Cys Phe Val Phe Ser Asp Val Asp Leu Ile Pro Met Asn Asp
                   245                 250                 255
   His Asn Ala Tyr Arg Cys Phe Ser Gln Pro Arg His Ile Ser Val Ala
               260                 265                 270
   Met Asp Lys Phe Gly Phe Ser Leu Pro Tyr Val Gln Tyr Phe Gly Gly
           275                 280                 285
   Val Ser Ala Leu Ser Lys Gln Gln Phe Leu Thr Ile Asn Gly Phe Pro
       290                 295                 300
   Asn Asn Tyr Trp Gly Trp Gly Gly Glu Asp Asp Asp Ile Phe Asn Arg
   305                 310                 315                 320
   Leu Val Phe Arg Gly Met Ser Ile Ser Arg Pro Asn Ala Val Val Gly
                   325                 330                 335
   Arg Cys Arg Met Ile Arg His Ser Arg Asp Lys Lys Asn Glu Pro Asn
               340                 345                 350
   Pro Gln Arg Phe Asp Arg Ile Ala His Thr Lys Glu Thr Met Leu Ser
           355                 360                 365
   Asp Gly Leu Asn Ser Leu Thr Tyr Gln Val Leu Asp Val Gln Arg Tyr
       370                 375                 380
   Pro Leu Tyr Thr Gln Ile Thr Val Asp Ile Gly Thr Pro Ser
   385                 390                 395
<210> 10
   <211> 1194
   <212> DNA
   <213> Homo sapiens
<220>
   <223> B4GALT1
<400> 10
   atgaggcttc gggagccgct cctgagcggc agcgccgcga tgccaggcgc gtccctacag      60
   cgggcctgcc gcctgctcgt ggccgtctgc gctctgcacc ttggcgtcac cctcgtttac     120
   tacctggctg gccgcgacct gagccgcctg ccccaactgg tcggagtctc cacaccgctg     180
   cagggcggct cgaacagtgc cgccgccatc gggcagtcct ccggggagct ccggaccgga     240
   ggggcccggc cgccgcctcc tctaggcgcc tcctcccagc cgcgcccggg tggcgactcc     300
   agcccagtcg tggattctgg ccctggcccc gctagcaact tgacctcggt cccagtgccc     360
   cacaccaccg cactgtcgct gcccgcctgc cctgaggagt ccccgctgct tgtgggcccc     420
   atgctgattg agtttaacat gcctgtggac ctggagctcg tggcaaagca gaacccaaat     480
   gtgaagatgg gcggccgcta tgcccccagg gactgcgtct ctcctcacaa ggtggccatc     540
   atcattccat tccgcaaccg gcaggagcac ctcaagtact ggctatatta tttgcaccca     600
   gtcctgcagc gccagcagct ggactatggc atctatgtta tcaaccaggc gggagacact     660
   atattcaatc gtgctaagct cctcaatgtt ggctttcaag aagccttgaa ggactatgac     720
   tacacctgct ttgtgtttag tgacgtggac ctcattccaa tgaatgacca taatgcgtac     780
   aggtgttttt cacagccacg gcacatttcc gttgcaatgg ataagtttgg attcagccta     840
   ccttatgttc agtattttgg aggtgtctct gctctaagta aacaacagtt tctaaccatc     900
   aatggatttc ctaataatta ttggggctgg ggaggagaag atgatgacat ttttaacaga     960
   ttagttttta gaggcatgtc tatatctcgc ccaaatgctg tggtcgggag gtgtcgcatg    1020
   atccgccact caagagacaa gaaaaatgaa cccaatcctc agaggtttga ccgaattgca    1080
   cacacaaagg agacaatgct ctctgatggt ttgaactcac tcacctacca ggtgctggat    1140
   gtacagagat acccattgta tacccaaatc acagtggaca tcgggacacc gagc          1194
<210> 11
   <211> 399
   <212> PRT
   <213> Rattus norvegicus
<220>
   <223> GALT1
<400> 11
   Met Arg Phe Arg Glu Pro Phe Leu Gly Gly Ser Ala Ala Met Pro Gly
   1               5                   10                  15
   Ala Thr Leu Gln Arg Ala Cys Arg Leu Leu Val Ala Val Cys Ala Leu
               20                  25                  30
   His Leu Gly Val Thr Leu Val Tyr Tyr Leu Ser Gly Arg Asp Leu Ser
           35                  40                  45
   Arg Leu Pro Gln Leu Val Gly Val Ser Ser Ser Leu Gln Gly Gly Thr
       50                  55                  60
   Asn Gly Ala Ala Ala Ser Lys Gln Pro Ser Gly Glu Leu Arg Pro Arg
   65                  70                  75                  80
   Gly Ala Arg Pro Pro Pro Pro Leu Gly Val Ser Pro Lys Pro Arg Pro
                   85                  90                  95
   Gly Ser Asp Ser Ser Pro Asp Ala Ala Ser Gly Pro Gly Leu Lys Ser
               100                 105                 110
   Asn Leu Thr Ser Val Pro Met Pro Thr Ser Thr Gly Leu Leu Thr Leu
           115                 120                 125
   Pro Ala Cys Pro Glu Glu Ser Pro Leu Leu Val Gly Pro Met Val Ile
       130                 135                 140
   Asp Phe Asn Ile Pro Val Asp Leu Glu Leu Leu Ala Lys Lys Asn Pro
   145                 150                 155                 160
   Glu Ile Lys Met Gly Gly Arg Tyr Phe Pro Lys Asp Cys Ile Ser Pro
                   165                 170                 175
   His Lys Val Ala Ile Ile Ile Pro Phe Arg Asn Arg Gln Glu His Leu
               180                 185                 190
   Lys Tyr Trp Leu Tyr Tyr Leu His Pro Val Leu Gln Arg Gln Gln Leu
           195                 200                 205
   Asp Tyr Gly Ile Tyr Val Ile Asn Gln Ala Gly Asp Thr Met Phe Asn
       210                 215                 220
   Arg Ala Lys Leu Leu Asn Val Gly Phe Gln Glu Ala Leu Lys Asp Tyr
   225                 230                 235                 240
   Asp Tyr Asn Cys Phe Val Phe Ser Asp Val Asp Leu Ile Pro Met Asp
                   245                 250                 255
   Asp His Asn Ala Tyr Arg Cys Phe Ser Gln Pro Arg His Ile Ser Val
               260                 265                 270
   Ala Met Asp Lys Phe Gly Phe Ser Leu Pro Tyr Val Gln Tyr Phe Gly
           275                 280                 285
   Gly Val Ser Ala Leu Ser Lys Gln Gln Phe Leu Thr Ile Asn Gly Phe
       290                 295                 300
   Pro Asn Asn Tyr Trp Gly Trp Gly Gly Glu Asp Asp Asp Ile Phe Asn
   305                 310                 315                 320
   Arg Leu Val His Lys Gly Met Ser Ile Ser Arg Pro Asn Ala Val Val
   325 330 335
   Gly Arg Cys Arg Met Ile Arg His Ser Arg Asp Lys Lys Asn Glu Pro
   340 345 350
   Asn Pro Gln Arg Phe Asp Arg Ile Ala His Thr Lys Glu Thr Met Arg
   355 360 365
   Leu Asp Gly Leu Asn Ser Leu Thr Tyr Gln Val Leu Asp Ile Gln Arg
   370 375 380
   Tyr Pro Leu Tyr Thr Lys Ile Thr Val Asp Ile Gly Thr Pro Arg
   385 390 395
<210> 12
   <211> 1197
   <212> DNA
   <213> Rattus norvegicus
<220>
   <223> B4GALT1
<400> 12
   atgaggtttc gtgagccgtt cctgggcggc agcgccgcga tgccgggcgc gaccctgcag 60
   cgggcctgcc gcctgctcgt ggcggtctgc gcgctgcacc ttggcgtcac cctggtctat 120
   tacctctccg gtcgcgatct gagccgcctg ccccaactgg tcggagtctc ctcttcactg 180
   caaggcggca cgaacggcgc cgccgccagc aagcagccct cgggagagct ccggccccgg 240
   ggcgcgcggc cgccgcctcc tttaggcgtc tccccgaagc ctcgcccggg ttctgactcc 300
   agccctgatg cggcttctgg ccccggcctg aagagcaact tgacttcggt gccaatgccc 360
   accagcactg gattgttgac tctgcctgct tgccctgagg agtccccgct gctcgttggc 420
   cccatggtga ttgactttaa tattcctgtg gatctggagc ttttggcaaa gaagaaccca 480
   gagataaaga tgggcggccg ttacttcccc aaggactgta tctcccctca caaggtggcc 540
   atcattatcc cattccgtaa ccggcaggag cacctcaaat actggctgta ttatttgcat 600
   ccagtccttc agcgccagca actcgactat ggcatctacg tcatcaatca ggctggagac 660
   accatgttta atcgagctaa gctgctcaac gttggctttc aagaggcctt gaaagactat 720
   gactacaact gctttgtgtt cagtgatgtg gacctcattc caatggatga ccataatgcc 780
   tacaggtgct tttcacagcc acggcatatt tctgtcgcaa tggacaagtt cgggtttagc 840
   ctgccttacg ttcagtattt tggaggtgtc tccgctctca gtaaacaaca gttccttacc 900
   atcaatggat ttcctaataa ttactggggc tggggaggag aagatgatga catttttaac 960
   agattagttc ataaaggcat gtctatatca cgcccaaatg ctgtggtagg caggtgtcgc 1020
   atgatccggc actcaagaga caagaaaaat gagcccaacc ctcagaggtt tgaccggatc 1080
   gcacatacaa aggaaacgat gcgccttgat ggtttgaact cacttaccta ccaggtgttg 1140
   gacatacaga gatacccgtt atataccaaa atcacagtgg acatcgggac accaaga 1197
<210> 13
   <211> 399
   <212> PRT
   <213> Mus musculus
<220>
   <223> GALT1
<400> 13
   Met Arg Phe Arg Glu Gln Phe Leu Gly Gly Ser Ala Ala Met Pro Gly
   1               5                   10                  15
   Ala Thr Leu Gln Arg Ala Cys Arg Leu Leu Val Ala Val Cys Ala Leu
               20                  25                  30
   His Leu Gly Val Thr Leu Val Tyr Tyr Leu Ser Gly Arg Asp Leu Ser
           35                  40                  45
   Arg Leu Pro Gln Leu Val Gly Val Ser Ser Thr Leu Gln Gly Gly Thr
       50                  55                  60
   Asn Gly Ala Ala Ala Ser Lys Gln Pro Pro Gly Glu Gln Arg Pro Arg
   65                  70                  75                  80
   Gly Ala Arg Pro Pro Pro Pro Leu Gly Val Ser Pro Lys Pro Arg Pro
                   85                  90                  95
   Gly Leu Asp Ser Ser Pro Gly Ala Ala Ser Gly Pro Gly Leu Lys Ser
               100                 105                 110
   Asn Leu Ser Ser Leu Pro Val Pro Thr Thr Thr Gly Leu Leu Ser Leu
           115                 120                 125
   Pro Ala Cys Pro Glu Glu Ser Pro Leu Leu Val Gly Pro Met Leu Ile
       130                 135                 140
   Asp Phe Asn Ile Ala Val Asp Leu Glu Leu Leu Ala Lys Lys Asn Pro
   145                 150                 155                 160
   Glu Ile Lys Thr Gly Gly Arg Tyr Ser Pro Lys Asp Cys Val Ser Pro
                   165                 170                 175
   His Lys Val Ala Ile Ile Ile Pro Phe Arg Asn Arg Gln Glu His Leu
                   180                 185                 190
   Lys Tyr Trp Leu Tyr Tyr Leu His Pro Ile Leu Gln Arg Gln Gln Leu
               195                 200                 205
   Asp Tyr Gly Ile Tyr Val Ile Asn Gln Ala Gly Asp Thr Met Phe Asn
       210                 215                 220
   Arg Ala Lys Leu Leu Asn Ile Gly Phe Gln Glu Ala Leu Lys Asp Tyr
   225                 230                 235                 240
   Asp Tyr Asn Cys Phe Val Phe Ser Asp Val Asp Leu Ile Pro Met Asp
                       245                 250                 255
   Asp Arg Asn Ala Tyr Arg Cys Phe Ser Gln Pro Arg His Ile Ser Val
                   260                 265                 270
   Ala Met Asp Lys Phe Gly Phe Ser Leu Pro Tyr Val Gln Tyr Phe Gly
               275                 280                 285
   Gly Val Ser Ala Leu Ser Lys Gln Gln Phe Leu Ala Ile Asn Gly Phe
       290                 295                 300
   Pro Asn Asn Tyr Trp Gly Trp Gly Gly Glu Asp Asp Asp Ile Phe Asn
   305                 310                 315                 320
   Arg Leu Val His Lys Gly Met Ser Ile Ser Arg Pro Asn Ala Val Val
                       325                 330                 335
   Gly Arg Cys Arg Met Ile Arg His Ser Arg Asp Lys Lys Asn Glu Pro
                   340                 345                 350
   Asn Pro Gln Arg Phe Asp Arg Ile Ala His Thr Lys Glu Thr Met Arg
               355                 360                 365
   Phe Asp Gly Leu Asn Ser Leu Thr Tyr Lys Val Leu Asp Val Gln Arg
       370                 375                 380
   Tyr Pro Leu Tyr Thr Gln Ile Thr Val Asp Ile Gly Thr Pro Arg
   385                 390                 395
<210> 14
   <211> 1197
   <212> DNA
   <213> Mus musculus
<220>
   <223> B4GALT1
<400> 14
   atgaggtttc gtgagcagtt cctgggcggc agcgccgcga tgccgggcgc gaccctgcag      60
   cgggcctgcc gcctgctcgt ggccgtctgc gcgctgcacc tcggcgtcac cctcgtctat     120
   tacctctctg gccgcgatct gagccgcctg ccccagttgg tcggagtctc ctctacactg     180
   cagggcggca cgaacggcgc cgcagccagc aagcagcccc caggagagca gcggccgcgg     240
   ggtgcgcggc cgccgcctcc tttaggcgtc tccccgaagc ctcgcccggg tctcgactcc     300
   agccctggtg cagcttctgg ccccggcttg aagagcaact tgtcttcgtt gccagtgccc     360
   accaccactg gactgttgtc gctgccagct tgccctgagg agtccccgct gctcgttggc     420
   cccatgctga ttgactttaa tattgctgtg gatctggagc ttttggcaaa gaagaaccca     480
   gagataaaga cgggcggccg ttactccccc aaggactgtg tctctcctca caaggtggcc     540
   atcatcatcc cattccgtaa ccggcaggag catctcaaat actggctgta ttatttgcat     600
   cccatccttc agcgccagca actcgactat ggcatctacg tcatcaatca ggctggagac     660
   accatgttca atcgagctaa gctgctcaat attggctttc aagaggcctt gaaggactat     720
   gattacaact gctttgtgtt cagtgatgtg gacctcattc cgatggacga ccgtaatgcc     780
   tacaggtgtt tttcgcagcc acggcacatt tctgttgcaa tggacaagtt cgggtttagc     840
   ctgccatatg ttcagtattt tggaggtgtc tctgctctca gtaaacaaca gtttcttgcc     900
   atcaatggat tccctaataa ttattggggt tggggaggag aagatgacga catttttaac     960
   agattagttc ataaaggcat gtctatatca cgtccaaatg ctgtagtagg gaggtgtcga    1020
   atgatccggc attcaagaga caagaaaaat gagcccaatc ctcagaggtt tgaccggatc    1080
   gcacatacaa aggaaacgat gcgcttcgat ggtttgaact cacttaccta caaggtgttg    1140
   gatgtacaga gatacccgtt atatacccaa atcacagtgg acatcgggac accgaga       1197
<210> 15
   <211> 372
   <212> PRT
   <213> Homo sapiens
<220>
   <223> GalT2
<400> 15
   Met Ser Arg Leu Leu Gly Gly Thr Leu Glu Arg Val Cys Lys Ala Val
   1               5                   10                  15
   Leu Leu Leu Cys Leu Leu His Phe Leu Val Ala Val Ile Leu Tyr Phe
               20                  25                  30
   Asp Val Tyr Ala Gln His Leu Ala Phe Phe Ser Arg Phe Ser Ala Arg
           35                  40                  45
   Gly Pro Ala His Ala Leu His Pro Ala Ala Ser Ser Ser Ser Ser Ser
       50                  55                  60
   Ser Asn Cys Ser Arg Pro Asn Ala Thr Ala Ser Ser Ser Gly Leu Pro
   65                  70                  75                  80
   Glu Val Pro Ser Ala Leu Pro Gly Pro Thr Ala Pro Thr Leu Pro Pro
                   85                  90                  95
   Cys Pro Asp Ser Pro Pro Gly Leu Val Gly Arg Leu Leu Ile Glu Phe
               100                 105                 110
   Thr Ser Pro Met Pro Leu Glu Arg Val Gln Arg Glu Asn Pro Gly Val
           115                 120                 125
   Leu Met Gly Gly Arg Tyr Thr Pro Pro Asp Cys Thr Pro Ala Gln Thr
       130                 135                 140
   Val Ala Val Ile Ile Pro Phe Arg His Arg Glu His His Leu Arg Tyr
   145                 150                 155                 160
   Trp Leu His Tyr Leu His Pro Ile Leu Arg Arg Gln Arg Leu Arg Tyr
                   165                 170                 175
   Gly Val Tyr Val Ile Asn Gln His Gly Glu Asp Thr Phe Asn Arg Ala
               180                 185                 190
   Lys Leu Leu Asn Val Gly Phe Leu Glu Ala Leu Lys Glu Asp Ala Ala
           195                 200                 205
   Tyr Asp Cys Phe Ile Phe Ser Asp Val Asp Leu Val Pro Met Asp Asp
       210                 215                 220
   Arg Asn Leu Tyr Arg Cys Gly Asp Gln Pro Arg His Phe Ala Ile Ala
   225                 230                 235                 240
   Met Asp Lys Phe Gly Phe Arg Leu Pro Tyr Ala Gly Tyr Phe Gly Gly
                   245                 250                 255
   Val Ser Gly Leu Ser Lys Ala Gln Phe Leu Arg Ile Asn Gly Phe Pro
               260                 265                 270
   Asn Glu Tyr Trp Gly Trp Gly Gly Glu Asp Asp Asp Ile Phe Asn Arg
           275                 280                 285
   Ile Ser Leu Thr Gly Met Lys Ile Ser Arg Pro Asp Ile Arg Ile Gly
   290 295 300
   Arg Tyr Arg Met Ile Lys His Asp Arg Asp Lys His Asn Glu Pro Asn
   305 310 315 320
   Pro Gln Arg Phe Thr Lys Ile Gln Asn Thr Lys Leu Thr Met Lys Arg
   325 330 335
   Asp Gly Ile Gly Ser Val Arg Tyr Gln Val Leu Glu Val Ser Arg Gln
   340 345 350
   Pro Leu Phe Thr Asn Ile Thr Val Asp Ile Gly Arg Pro Pro Ser Trp
   355 360 365
   Pro Pro Arg Gly
   370
<210> 16
   <211> 1116
   <212> DNA
   <213> Homo sapiens
<220>
   <223> B4GalT2
<400> 16
   atgagcagac tgctgggggg gacgctggag cgcgtctgca aggctgtgct ccttctctgc      60
   ctgctgcact tcctcgtggc cgtcatcctc tactttgacg tctacgccca gcacctggcc     120
   ttcttcagcc gcttcagtgc ccgaggccct gcccatgccc tccacccagc tgctagcagc     180
   agcagcagca gcagcaactg ctcccggccc aacgccaccg cctctagctc cgggctccct     240
   gaggtcccca gtgccctgcc cggtcccacg gctcccacgc tgccaccctg tcctgactcg     300
   ccacctggtc ttgtgggcag actgctgatc gagttcacct cacccatgcc cctggagcgg     360
   gtgcagaggg agaacccagg cgtgctcatg ggcggccgat acacaccgcc cgactgcacc     420
   ccagcccaga cggtggcggt catcatcccc tttagacacc gggaacacca cctgcgctac     480
   tggctccact atctacaccc catcttgagg cggcagcggc tgcgctacgg cgtctatgtc     540
   atcaaccagc atggtgagga caccttcaac cgggccaagc tgcttaacgt gggcttccta     600
   gaggcgctga aggaggatgc cgcctatgac tgcttcatct tcagcgatgt ggacctggtc     660
   cccatggatg accgcaacct ataccgctgc ggcgaccaac cccgccactt tgccattgcc     720
   atggacaagt ttggcttccg gcttccctat gctggctact ttggaggtgt gtcaggcctg     780
   agtaaggctc agtttctgag aatcaatggc ttccccaatg agtactgggg ctggggtggc     840
   gaggatgatg acatcttcaa ccggatctcc ctgactggga tgaagatctc acgcccagac     900
   atccgaatcg gccgctaccg catgatcaag cacgaccgcg acaagcataa cgaacctaac     960
   cctcagaggt ttaccaagat tcaaaacacg aagctgacca tgaagcggga cggcattggg    1020
   tcagtgcggt accaggtctt ggaggtgtct cggcaaccac tcttcaccaa tatcacagtg    1080
   gacattgggc ggcctccgtc gtggccccct cggggc                              1116
<210> 17
   <211> 369
   <212> PRT
   <213> Rattus norvegicus
<220>
   <223> GalT2
<400> 17
   Met Ser Arg Leu Leu Gly Gly Thr Leu Glu Arg Val Cys Lys Ala Val
   1               5                   10                  15
   Leu Leu Leu Cys Leu Leu His Phe Leu Val Ala Val Ile Leu Tyr Phe
               20                  25                  30
   Asp Val Tyr Ala Gln His Leu Ala Phe Phe Ser Arg Phe Ser Thr Arg
           35                  40                  45
   Ser Pro Ala His Ala Leu Tyr Pro Ala Ala Ser Ser Ser Thr Asn Cys
       50                  55                  60
   Ser Arg Pro Asn Thr Thr Ala Ala Ser Ser Gly Leu Pro Glu Val Pro
   65                  70                  75                  80
   Ser Ala Arg Pro Gly Pro Thr Ala Pro Val Ile Pro Pro Cys Pro Asp
                   85                  90                  95
   Val Pro Pro Gly Leu Val Gly Arg Val Val Ile Glu Phe Thr Ser Pro
               100                 105                 110
   Met Pro Leu Glu Arg Val Gln Arg Glu Asn Pro Gly Val Leu Leu Gly
           115                 120                 125
   Gly Arg Tyr Ser Pro Pro Asp Cys Thr Pro Ala Gln Thr Val Ala Val
       130                 135                 140
   Ile Ile Pro Phe Arg His Arg Glu His His Leu Arg Tyr Trp Leu His
   145                 150                 155                 160
   Tyr Leu His Pro Met Leu Arg Arg Gln Arg Leu Arg Tyr Gly Val Tyr
                   165                 170                 175
   Val Ile Asn Gln His Gly Glu Glu Thr Phe Asn Arg Ala Lys Leu Leu
   180 185 190
   Asn Val Gly Phe Leu Glu Ala Leu Lys Glu Asp Ala Thr Tyr Asp Cys
   195 200 205
   Phe Ile Phe Ser Asp Val Asp Leu Val Pro Met Asp Asp Arg Asn Leu
   210 215 220
   Tyr Arg Cys Gly Asp Gln Pro Arg His Phe Ala Ile Ala Met Asp Lys
   225 230 235 240
   Phe Gly Phe Arg Leu Pro Tyr Ala Ser Tyr Phe Gly Gly Val Ser Gly
   245 250 255
   Leu Ser Lys Ala Gln Phe Leu Arg Ile Asn Gly Phe Pro Asn Glu Tyr
   260 265 270
   Trp Gly Trp Gly Gly Glu Asp Asp Asp Ile Phe Asn Arg Ile Ser Leu
   275 280 285
   Thr Gly Met Lys Ile Ser Arg Pro Asp Val Arg Ile Gly Arg Tyr Arg
   290 295 300
   Met Ile Lys His Asp Arg Asp Lys His Asn Glu Pro Asn Pro Gln Arg
   305 310 315 320
   Phe Asn Lys Ile Gln Asn Thr Lys Met Ser Met Lys Trp Asp Gly Ile
   325 330 335
   Gly Ser Val Arg Tyr Arg Val Leu Glu Val Ser Arg Gln Pro Leu Phe
   340 345 350
   Thr Asn Ile Thr Val Asp Ile Gly Gln Pro Met Ser Trp Leu Thr Gln
   355 360 365
   Gly
<210> 18
   <211> 1107
   <212> DNA
   <213> Rattus norvegicus
<220>
   <223> B4GalT2
<400> 18
   atgagcagac tgctgggggg tacgctggag cgggtctgta aggctgtgct ccttctctgc      60
   ctgctgcact tccttgtggc tgtcatcctc tactttgacg tctacgccca gcacctggcc     120
   tttttcagcc gcttcagtac ccgaagccca gcccatgccc tctaccctgc agccagcagc     180
   agtaccaact gctctcggcc caacaccact gctgccagct ccgggctccc tgaagtgccc     240
   agtgccaggc ctggccccac agctccagtc attccaccct gtcctgatgt gccgcctggt     300
   cttgtgggcc gagtggtcat cgaattcacc tcacccatgc ctctggaacg ggtgcagagg     360
   gagaacccag gcgtgctcct gggcggccgc tattctccac ctgactgcac cccagcccaa     420
   acggtggcag tcatcatccc ctttagacac cgggagcacc acctacgcta ttggctccac     480
   tatctgcacc ccatgttgag gcgacagcgg ctgcgctacg gtgtctatgt catcaaccag     540
   catggcgagg agaccttcaa ccgagccaag ctgctcaacg tgggcttcct agaggcactg     600
   aaagaggatg ccacctatga ctgcttcatc ttcagtgatg tggacctggt ccctatggat     660
   gaccgcaatc tgtaccgttg tggtgaccag ccccgccact tcgccattgc catggacaag     720
   tttggcttcc ggttgcccta tgccagctac tttggaggtg tgtcaggcct gagcaaggcc     780
   cagtttctga ggattaatgg ttttcccaac gagtactggg gctggggcgg cgaggacgat     840
   gacatcttca accggatctc cctgactggg atgaagatct cacgcccaga cgtccggata     900
   ggccgctacc gcatgatcaa gcacgaccgg gacaaacata acgagcccaa ccctcagagg     960
   tttaataaga ttcaaaacac aaagatgagc atgaagtggg acggcattgg atccgtgcgg    1020
   taccgcgtct tggaagtatc tcggcaacca ctcttcacca acatcacagt ggacattggg    1080
   cagcccatgt catggctcac tcaaggc                                        1107
<210> 19
   <211> 369
   <212> PRT
   <213> Mus musculus
<220>
   <223> GalT2
<400> 19
   Met Ser Arg Leu Leu Gly Gly Thr Leu Glu Arg Val Cys Lys Ala Val
   1               5                   10                  15
   Leu Leu Leu Cys Leu Leu His Phe Leu Val Ala Val Ile Leu Tyr Phe
               20                  25                  30
   Asp Val Tyr Ala Gln His Leu Ala Phe Phe Ser Arg Phe Ser Thr Arg
           35                  40                  45
   Ser Pro Ala His Ala Leu Tyr Pro Ala Ala Ser Ser Ser Thr Asn Cys
       50                  55                  60
   Ser Arg Pro Asn Ala Thr Ala Ala Ser Ser Gly Leu Pro Glu Val Pro
   65                  70                  75                  80
   Ser Ala Arg Pro Gly Pro Thr Ala Pro Val Ile Pro Pro Cys Pro Asp
                   85                  90                  95
   Val Pro Pro Gly Leu Val Gly Arg Val Val Ile Glu Phe Thr Ser Pro
               100                 105                 110
   Met Pro Leu Glu Arg Val Gln Arg Glu Asn Pro Gly Val Leu Leu Gly
           115                 120                 125
   Gly Arg Tyr Ser Pro Pro Asp Cys Thr Pro Ala Gln Thr Val Ala Val
       130                 135                 140
   Ile Ile Pro Phe Arg His Arg Glu His His Leu Arg Tyr Trp Leu His
   145                 150                 155                 160
   Tyr Leu His Pro Met Leu Arg Arg Gln Arg Leu Arg Tyr Gly Val Tyr
                   165                 170                 175
   Val Ile Asn Gln His Gly Glu Glu Thr Phe Asn Arg Ala Lys Leu Leu
               180                 185                 190
   Asn Val Gly Phe Leu Glu Ala Leu Lys Glu Asp Ala Ala Tyr Asp Cys
           195                 200                 205
   Phe Ile Phe Ser Asp Val Asp Leu Val Pro Met Asp Asp Arg Asn Leu
       210                 215                 220
   Tyr Arg Cys Gly Asp Gln Pro Arg His Phe Ala Ile Ala Met Asp Lys
   225                 230                 235                 240
   Phe Gly Phe Arg Leu Pro Tyr Ala Ser Tyr Phe Gly Gly Val Ser Gly
                   245                 250                 255
   Leu Ser Lys Ala Gln Phe Leu Arg Ile Asn Gly Phe Pro Asn Glu Tyr
               260                 265                 270
   Trp Gly Trp Gly Gly Glu Asp Asp Asp Ile Phe Asn Arg Ile Ser Leu
           275                 280                 285
   Thr Gly Met Lys Ile Ser Arg Pro Asp Val Arg Ile Gly Arg Tyr Arg
       290                 295                 300
   Met Ile Lys His Asp Arg Asp Lys His Asn Glu Pro Asn Pro Gln Arg
   305                 310                 315                 320
   Phe Asn Lys Ile Gln Asn Thr Lys Met Ser Met Lys Trp Asp Gly Ile
   325 330 335
   Gly Ser Val Arg Tyr Arg Val Leu Glu Val Ser Arg Gln Pro Leu Phe
   340 345 350
   Thr Asn Ile Thr Val Asp Ile Gly Gln Pro Met Ser Trp Leu Thr Gln
   355 360 365
   Gly
<210> 20
   <211> 1107
   <212> DNA
   <213> Mus musculus
<220>
   <223> B4GalT2
<400> 20
   atgagcagac tgctgggggg gacgctggag cgggtctgca aggctgtgct ccttctctgc 60
   ctgctgcact tccttgtggc cgtcatcctc tactttgacg tctatgccca gcacctggcc 120
   tttttcagcc gcttcagtac ccgaagccca gcccatgccc tctaccctgc agccagcagc 180
   agtaccaact gctctcggcc caacgccact gccgctagct ccgggctgcc tgaagtgccc 240
   agtgccaggc ctggtcccac agctccagtc attccgccct gtcctgacgt gccgcctggt 300
   cttgtgggcc gagtggtcat cgaattcacc tcacccatgc ctctggaacg ggtgcagagg 360
   gaaaatccag gcgtgctcct gggcggccgc tattctccac ccgactgcac cccagcccaa 420
   acggtggcag tcatcatccc ctttagacac cgggagcacc acctacgcta ttggctccac 480
   tatctgcacc ccatgttgag gcggcagcgg ctgcgctacg gtgtctatgt catcaaccag 540
   catggcgagg agaccttcaa ccgagccaag ctgctcaacg tgggcttcct agaggcactg 600
   aaggaggatg ctgcctatga ctgcttcatc ttcagtgatg tggacctggt ccctatggat 660
   gaccgcaatc tgtaccgttg tggtgaccag ccccgccact ttgccatcgc catggacaag 720
   tttggcttcc ggttgcccta tgctagctac tttggaggtg tgtcgggcct gagcaaggcc 780
   cagtttctga ggatcaatgg ctttcccaac gagtactggg gctggggtgg cgaggatgat 840
   gacatcttca accggatctc cctgactggg atgaagatct cacgcccgga tgtccggatt 900
   ggccgctacc gcatgatcaa gcacgaccgg gacaaacata acgagcccaa ccctcagagg 960
   tttaataaga ttcaaaacac aaagatgagc atgaagtggg acggcattgg atccgtgcgg 1020
   taccgagtct tggaagtgtc tcggcaaccg ctcttcacca acatcacagt ggacattgga 1080
   cagcccatgt cgtggctcac tcaaggc 1107
<210> 21
   <211> 393
   <212> PRT
   <213> Homo sapiens
<220>
   <223> GalT3
<400> 21
   Met Leu Arg Arg Leu Leu Glu Arg Pro Cys Thr Leu Ala Leu Leu Val
   1               5                   10                  15
   Gly Ser Gln Leu Ala Val Met Met Tyr Leu Ser Leu Gly Gly Phe Arg
               20                  25                  30
   Ser Leu Ser Ala Leu Phe Gly Arg Asp Gln Gly Pro Thr Phe Asp Tyr
           35                  40                  45
   Ser His Pro Arg Asp Val Tyr Ser Asn Leu Ser His Leu Pro Gly Ala
       50                  55                  60
   Pro Gly Gly Pro Pro Ala Pro Gln Gly Leu Pro Tyr Cys Pro Glu Arg
   65                  70                  75                  80
   Ser Pro Leu Leu Val Gly Pro Val Ser Val Ser Phe Ser Pro Val Pro
                   85                  90                  95
   Ser Leu Ala Glu Ile Val Glu Arg Asn Pro Arg Val Glu Pro Gly Gly
               100                 105                 110
   Arg Tyr Arg Pro Ala Gly Cys Glu Pro Arg Ser Arg Thr Ala Ile Ile
           115                 120                 125
   Val Pro His Arg Ala Arg Glu His His Leu Arg Leu Leu Leu Tyr His
       130                 135                 140
   Leu His Pro Phe Leu Gln Arg Gln Gln Leu Ala Tyr Gly Ile Tyr Val
   145                 150                 155                 160
   Ile His Gln Ala Gly Asn Gly Thr Phe Asn Arg Ala Lys Leu Leu Asn
                   165                 170                 175
   Val Gly Val Arg Glu Ala Leu Arg Asp Glu Glu Trp Asp Cys Leu Phe
               180                 185                 190
   Leu His Asp Val Asp Leu Leu Pro Glu Asn Asp His Asn Leu Tyr Val
           195                 200                 205
   Cys Asp Pro Arg Gly Pro Arg His Val Ala Val Ala Met Asn Lys Phe
       210                 215                 220
   Gly Tyr Ser Leu Pro Tyr Pro Gln Tyr Phe Gly Gly Val Ser Ala Leu
   225                 230                 235                 240
   Thr Pro Asp Gln Tyr Leu Lys Met Asn Gly Phe Pro Asn Glu Tyr Trp
                   245                 250                 255
   Gly Trp Gly Gly Glu Asp Asp Asp Ile Ala Thr Arg Val Arg Leu Ala
               260                 265                 270
   Gly Met Lys Ile Ser Arg Pro Pro Thr Ser Val Gly His Tyr Lys Met
           275                 280                 285
   Val Lys His Arg Gly Asp Lys Gly Asn Glu Glu Asn Pro His Arg Phe
       290                 295                 300
   Asp Leu Leu Val Arg Thr Gln Asn Ser Trp Thr Gln Asp Gly Met Asn
   305                 310                 315                 320
   Ser Leu Thr Tyr Gln Leu Leu Ala Arg Glu Leu Gly Pro Leu Tyr Thr
                   325                 330                 335
   Asn Ile Thr Ala Asp Ile Gly Thr Asp Pro Arg Gly Pro Arg Ala Pro
               340                 345                 350
   Ser Gly Pro Arg Tyr Pro Pro Gly Ser Ser Gln Ala Phe Arg Gln Glu
           355                 360                 365
   Met Leu Gln Arg Arg Pro Pro Ala Arg Pro Gly Pro Leu Ser Thr Ala
       370                 375                 380
   Asn His Thr Ala Leu Arg Gly Ser His
   385                 390
<210> 22
   <211> 1179
   <212> DNA
   <213> Homo sapiens
<220>
   <223> B4GalT3
<400> 22
   atgttgcgga ggctgctgga gcggccttgc acgctggccc tgcttgtggg ctcccagctg        60
   gctgtcatga tgtacctgtc actggggggc ttccgaagtc tcagtgccct atttggccga        120
   gatcagggac cgacatttga ctattctcac cctcgtgatg tctacagtaa cctcagtcac       180
   ctgcctgggg ccccaggggg tcctccagct cctcaaggtc tgccctactg tccagaacga       240
   tctcctctct tagtgggtcc tgtgtcggtg tcctttagcc cagtgccatc actggcagag       300
   attgtggagc ggaatccccg ggtagaacca gggggccggt accgccctgc aggttgtgag       360
   ccccgctccc gaacagccat cattgtgcct catcgtgccc gggagcacca cctgcgcctg       420
   ctgctctacc acctgcaccc cttcttgcag cgccagcagc ttgcttatgg catctatgtc       480
   atccaccagg ctggaaatgg aacatttaac agggcaaaac tgttgaacgt tggggtgcga       540
   gaggccctgc gtgatgaaga gtgggactgc ctgttcttgc acgatgtgga cctcttgcca       600
   gaaaatgacc acaatctgta tgtgtgtgac ccccggggac cccgccatgt tgccgttgct        660
   atgaacaagt ttggatacag cctcccgtac ccccagtact tcggaggagt ctcagcactt        720
   actcctgacc agtacctgaa gatgaatggc ttccccaatg aatactgggg ctggggtggt       780
   gaggatgacg acattgctac cagggtgcgc ctggctggga tgaagatctc tcggcccccc       840
   acatctgtag gacactataa gatggtgaag caccgaggag ataagggcaa tgaggaaaat       900
   ccccacagat ttgacctcct ggtccgtacc cagaattcct ggacgcaaga tgggatgaac       960
   tcactgacat accagttgct ggctcgagag ctggggcctc tttataccaa catcacagca      1020
   gacattggga ctgaccctcg gggtcctcgg gctccttctg ggccacgtta cccacctggt      1080
   tcctcccaag ccttccgtca agagatgctg caacgccggc ccccagccag gcctgggcct       1140
   ctatctactg ccaaccacac agccctccga ggttcacac                             1179
<210> 23
   <211> 395
   <212> PRT
   <213> Rattus norvegicus
<220>
   <223> GalT3
<400> 23
   Met Leu Arg Arg Leu Leu Glu Arg Pro Cys Thr Leu Ala Leu Leu Val
   1               5                   10                  15
   Gly Ser Gln Leu Ala Val Met Met Tyr Leu Ser Leu Gly Gly Phe Arg
               20                  25                  30
   Ser Leu Ser Ala Leu Phe Gly Arg Asp Pro Gly Pro Thr Phe Asp Tyr
           35                  40                  45
   Ser His Pro His Asp Val Tyr Ser Asn Leu Ser His Leu Pro Gly Ala
       50                  55                  60
   Pro Gly Ala Ala Gly Ala Pro Leu Ala Gln Val Leu Pro Asp Cys Pro
   65                  70                  75                  80
   Glu Arg Ser Pro Phe Leu Val Gly Pro Val Ser Val Ser Phe Ser Pro
                   85                  90                  95
   Val Pro Ser Leu Ala Glu Ile Val Glu Arg Asn Pro Arg Val Glu Ser
               100                 105                 110
   Gly Gly Arg Tyr Arg Pro Ala Gly Cys Glu Pro Arg Ser Arg Thr Ala
           115                 120                 125
   Ile Ile Val Pro His Arg Ala Arg Glu His His Leu Arg Leu Leu Leu
       130                 135                 140
   Tyr His Leu His Pro Phe Leu Gln Arg Gln Gln Leu Ala Tyr Gly Ile
   145                 150                 155                 160
   Tyr Val Ile His Gln Ala Gly Asn Gly Thr Phe Asn Arg Ala Lys Leu
                   165                 170                 175
   Leu Asn Val Gly Val Arg Glu Ala Leu Arg Asp Glu Glu Trp Asp Cys
               180                 185                 190
   Leu Phe Leu His Asp Val Asp Leu Leu Pro Glu Asn Asp His Asn Leu
           195                 200                 205
   Tyr Val Cys Asp Pro Arg Gly Pro Arg His Val Ala Val Ala Met Asn
       210                 215                 220
   Lys Phe Gly Tyr Ser Leu Pro Tyr Pro Gln Tyr Phe Gly Gly Val Ser
   225                 230                 235                 240
   Ala Leu Thr Pro Asp Gln Tyr Leu Lys Met Asn Gly Phe Pro Asn Glu
                   245                 250                 255
   Tyr Trp Gly Trp Gly Gly Glu Asp Asp Asp Ile Ala Thr Arg Val Arg
               260                 265                 270
   Leu Ala Gly Met Lys Ile Ser Arg Pro Pro Thr Ser Val Gly His Tyr
           275                 280                 285
   Lys Met Val Lys His Arg Gly Asp Lys Gly Asn Glu Glu Asn Pro His
       290                 295                 300
   Arg Phe Asp Leu Leu Val Arg Thr Gln Asn Ser Trp Thr Gln Asp Gly
   305                 310                 315                 320
   Met Asn Ser Leu Thr Tyr Arg Leu Leu Ala Arg Glu Leu Gly Pro Leu
                   325                 330                 335
   Tyr Thr Asn Ile Thr Ala Asp Ile Gly Thr Asp Pro Arg Gly Pro Arg
               340                 345                 350
   Ala Pro Ser Gly Pro Arg Tyr Pro Pro Gly Ser Ser Gln Ala Phe Arg
           355                 360                 365
   Gln Glu Met Leu Gln Arg Arg Pro Pro Ala Arg Pro Gly Pro Leu Pro
       370                 375                 380
   Thr Ala Asn His Thr Ala Pro His Gly Ser His
   385                 390                 395
<210> 24
   <211> 1185
   <212> DNA
   <213> Rattus norvegicus
<220>
   <223> B4GalT3
<400> 24
   atgttgcgga ggctgctgga gaggccctgc acactggccc tgcttgtggg ctcccaactg      60
   gcagttatga tgtatctgtc actagggggc ttccgaagtc ttagtgccct gtttggtcga     120
   gatccaggcc caacattcga ctattctcat ccccatgatg tctatagtaa cctcagtcac     180
   ctgcctggag cccctggagc tgcaggggct cctctagctc aagttctgcc tgactgtcca     240
   gaaagatcgc ctttcttagt gggtcctgtg tcagtatcct ttagcccagt gccatcacta     300
   gcagagatcg tggagcggaa tccccgggtg gaatcagggg gccggtaccg tcctgcagga     360
   tgtgagcctc gctcccgaac agccataatt gtgccccacc gtgcccggga gcaccacctc     420
   cgactgctac tctatcacct gcaccccttc ctgcagcgcc agcagcttgc ttatggcatc     480
   tatgtcatcc accaggctgg aaatggaacg tttaacaggg ccaaactgct gaacgtaggg     540
   gtcagggaag cccttcgtga cgaagagtgg gactgcttgt ttttacacga tgtggacctc     600
   cttccagaaa acgaccataa cctgtatgtg tgtgaccccc ggggaccccg ccatgttgct     660
   gtcgccatga acaagtttgg atacagcctt ccgtaccccc agtactttgg cggagtttca     720
   gcactcactc ctgaccagta cctgaagatg aatggcttcc ccaatgaata ctggggctgg     780
   ggtggcgagg atgacgacat tgctaccagg gttcgcctgg ctgggatgaa gatttcccga     840
   ccacccacct ctgtgggaca ctacaagatg gtgaagcaca gaggggataa aggaaatgag     900
   gaaaaccccc acagatttga cctcctggtc cgtacccaga attcttggac acaagatgga     960
   atgaactcac taacataccg actgctggca agagagctgg gtcctctcta caccaacatc    1020
   actgcagaca ttgggactga ccctcggggt ccccgggctc cctctggtcc ccgataccca    1080
   ccaggttcct cccaggcctt ccgtcaggag atgctgcaac gccggccccc agctaggcct    1140
   ggccctctgc ctactgccaa ccacacagct ccccatggtt cacac                    1185
<210> 25
   <211> 395
   <212> PRT
   <213> Mus musculus
<220>
   <223> GalT3
<400> 25
   Met Leu Arg Arg Leu Leu Glu Arg Pro Cys Thr Leu Ala Leu Leu Val
   1               5                   10                  15
   Gly Ser Gln Leu Ala Val Met Met Tyr Leu Ser Leu Gly Gly Phe Arg
               20                  25                  30
   Ser Leu Ser Ala Leu Phe Gly Arg Asp Pro Gly Pro Thr Phe Asp Tyr
           35                  40                  45
   Ser His Pro His Asp Val Tyr Ser Asn Leu Ser His Leu Pro Ala Ala
       50                  55                  60
   Pro Gly Ala Ala Gly Ala Pro Pro Ala Gln Ala Leu Pro Tyr Cys Pro
   65                  70                  75                  80
   Glu Arg Ser Pro Phe Leu Val Gly Pro Val Ser Val Ser Phe Ser Pro
                   85                  90                  95
   Val Pro Ser Leu Ala Glu Ile Val Glu Arg Asn Pro Arg Val Glu Ser
               100                 105                 110
   Gly Gly Arg Tyr Arg Pro Ala Gly Cys Glu Pro Arg Ser Arg Thr Ala
           115                 120                 125
   Ile Ile Val Pro His Arg Ala Arg Glu His His Leu Arg Leu Leu Leu
       130                 135                 140
   Tyr His Leu His Pro Phe Leu Gln Arg Gln Gln Leu Ala Tyr Gly Ile
   145                 150                 155                 160
   Tyr Val Ile His Gln Ala Gly Asn Gly Thr Phe Asn Arg Ala Lys Leu
                   165                 170                 175
   Leu Asn Val Gly Val Arg Glu Ala Leu Arg Asp Glu Glu Trp Asp Cys
               180                 185                 190
   Leu Phe Leu His Asp Val Asp Leu Leu Pro Glu Asn Asp His Asn Leu
           195                 200                 205
   Tyr Val Cys Asp Pro Arg Gly Pro Arg His Val Ala Val Ala Met Asn
       210                 215                 220
   Lys Phe Gly Tyr Ser Leu Pro Tyr Pro Gln Tyr Phe Gly Gly Val Ser
   225                 230                 235                 240
   Ala Leu Thr Pro Asp Gln Tyr Leu Lys Met Asn Gly Phe Pro Asn Glu
                   245                 250                 255
   Tyr Trp Gly Trp Gly Gly Glu Asp Asp Asp Ile Ala Thr Arg Val Arg
               260                 265                 270
   Leu Ala Gly Met Lys Ile Ser Arg Pro Pro Thr Ser Val Gly His Tyr
           275                 280                 285
   Lys Met Val Lys His Arg Gly Asp Lys Gly Asn Glu Glu Asn Pro His
       290                 295                 300
   Arg Phe Asp Leu Leu Val Arg Thr Gln Asn Ser Trp Thr Gln Asp Gly
   305                 310                 315                 320
   Met Asn Ser Leu Thr Tyr Arg Leu Leu Ala Arg Glu Leu Gly Pro Leu
                   325                 330                 335
   Tyr Thr Asn Ile Thr Ala Asp Ile Gly Thr Asp Pro Arg Gly Pro Arg
               340                 345                 350
   Ala Pro Ser Gly Pro Arg Tyr Pro Pro Gly Ser Ser Gln Ala Phe Arg
           355                 360                 365
   Gln Glu Met Leu Gln Arg Arg Pro Pro Ala Arg Pro Gly Pro Leu Pro
       370                 375                 380
   Thr Ala Asn His Thr Ala Pro Arg Gly Ser His
   385                 390                 395
<210> 26
   <211> 1185
   <212> DNA
   <213> Mus musculus
<220>
   <223> B4GalT3
<400> 26
   atgttgcgga ggctgctgga gagaccctgt acattggccc tgcttgtggg ctcccaactg        60
   gcagtgatga tgtatctgtc actagggggc ttccgaagtc ttagtgccct gtttggtcga       120
   gatccgggcc caacattcga ctattctcat ccccacgatg tctatagtaa cctcagtcac       180
   ctgcccgcag cccctggagc tgcaggggct cctccggctc aagcattgcc ctactgtcca       240
   gaaagatcac ccttcttagt gggtcctgtg tcagtatcct ttagcccggt gccgtcacta       300
   gcagagattg tggagcggaa tccccgggtg gaatcagggg gccggtaccg tcctgcaggg       360
   tgtgagcctc gctcccgaac agccataatt gtgccccatc gtgcccggga gcaccatctt       420
   cggctgctgc tctatcacct gcaccctttc ctgcagcgcc agcagcttgc gtacggcatt       480
   tatgtcatcc accaggctgg aaatggaacg tttaacaggg caaagctgct gaacgtaggg       540
   gtgagggaag cccttcgtga tgaagaatgg gactgcttgt tcttacacga cgtggacctc       600
   cttccagaaa acgaccataa cctgtatgtg tgcgaccccc ggggaccccg ccacgttgct       660
   gttgccatga acaagtttgg atacagcctc ccgtaccccc agtactttgg cggagtttca       720
   gcgctcactc ccgaccagta cctgaagatg aacggcttcc ccaacgagta ctggggctgg       780
   ggtggcgagg atgacgacat tgctaccagg gtccgcctgg ctgggatgaa gatctctcga       840
   ccacctacct ctgtgggaca ctataagatg gtgaagcaca gaggggataa aggaaatgag       900
   gaaaatcccc acagatttga cctcctggtc cgtacccaga attcttggac acaagatgga       960
   atgaactcac taacgtaccg actgctggca agagagctgg gtcctctcta taccaacatc      1020
   actgcagaca tcgggactga ccctcggggt ccccgggctc cctctggtcc ccgataccca      1080
   ccaggttcct cccaggcctt ccgtcaagag atgctgcaac gccggccccc agctaggcct      1140
   ggccctctgc ccactgccaa ccacacagct ccccgtggct cacac                      1185
<210> 27
   <211> 722
   <212> PRT
   <213> Homo sapiens
<220>
   <223> GNE
<400> 27
   Met Glu Lys Asn Gly Asn Asn Arg Lys Leu Arg Val Cys Val Ala Thr
   1               5                   10                  15
   Cys Asn Arg Ala Asp Tyr Ser Lys Leu Ala Pro Ile Met Phe Gly Ile
               20                  25                  30
   Lys Thr Glu Pro Glu Phe Phe Glu Leu Asp Val Val Val Leu Gly Ser
           35                  40                  45
   His Leu Ile Asp Asp Tyr Gly Asn Thr Tyr Arg Met Ile Glu Gln Asp
       50                  55                  60
   Asp Phe Asp Ile Asn Thr Arg Leu His Thr Ile Val Arg Gly Glu Asp
   65                  70                  75                  80
   Glu Ala Ala Met Val Glu Ser Val Gly Leu Ala Leu Val Lys Leu Pro
                   85                  90                  95
   Asp Val Leu Asn Arg Leu Lys Pro Asp Ile Met Ile Val His Gly Asp
               100                 105                 110
   Arg Phe Asp Ala Leu Ala Leu Ala Thr Ser Ala Ala Leu Met Asn Ile
           115                 120                 125
   Arg Ile Leu His Ile Glu Gly Gly Glu Val Ser Gly Thr Ile Asp Asp
       130                 135                 140
   Ser Ile Arg His Ala Ile Thr Lys Leu Ala His Tyr His Val Cys Cys
   145                 150                 155                 160
   Thr Arg Ser Ala Glu Gln His Leu Ile Ser Met Cys Glu Asp His Asp
                   165                 170                 175
   Arg Ile Leu Leu Ala Gly Cys Pro Ser Tyr Asp Lys Leu Leu Ser Ala
               180                 185                 190
   Lys Asn Lys Asp Tyr Met Ser Ile Ile Arg Met Trp Leu Gly Asp Asp
           195                 200                 205
   Val Lys Ser Lys Asp Tyr Ile Val Ala Leu Gln His Pro Val Thr Thr
       210                 215                 220
   Asp Ile Lys His Ser Ile Lys Met Phe Glu Leu Thr Leu Asp Ala Leu
   225                 230                 235                 240
   Ile Ser Phe Asn Lys Arg Thr Leu Val Leu Phe Pro Asn Ile Asp Ala
                   245                 250                 255
   Gly Ser Lys Glu Met Val Arg Val Met Arg Lys Lys Gly Ile Glu His
               260                 265                 270
   His Pro Asn Phe Arg Ala Val Lys His Val Pro Phe Asp Gln Phe Ile
           275                 280                 285
   Gln Leu Val Ala His Ala Gly Cys Met Ile Gly Asn Ser Ser Cys Gly
       290                 295                 300
   Val Arg Glu Val Gly Ala Phe Gly Thr Pro Val Ile Asn Leu Gly Thr
   305                 310                 315                 320
   Arg Gln Ile Gly Arg Glu Thr Gly Glu Asn Val Leu His Val Arg Asp
                   325                 330                 335
   Ala Asp Thr Gln Asp Lys Ile Leu Gln Ala Leu His Leu Gln Phe Gly
               340                 345                 350
   Lys Gln Tyr Pro Cys Ser Lys Ile Tyr Gly Asp Gly Asn Ala Val Pro
           355                 360                 365
   Arg Ile Leu Lys Phe Leu Lys Ser Ile Asp Leu Gln Glu Pro Leu Gln
       370                 375                 380
   Lys Lys Phe Cys Phe Pro Pro Val Lys Glu Asn Ile Ser Gln Asp Ile
   385                 390                 395                 400
   Asp His Ile Leu Glu Thr Leu Ser Ala Leu Ala Val Asp Leu Gly Gly
                   405                 410                 415
   Thr Asn Leu Arg Val Ala Ile Val Ser Met Lys Gly Glu Ile Val Lys
               420                 425                 430
   Lys Tyr Thr Gln Phe Asn Pro Lys Thr Tyr Glu Glu Arg Ile Asn Leu
           435                 440                 445
   Ile Leu Gln Met Cys Val Glu Ala Ala Ala Glu Ala Val Lys Leu Asn
       450                 455                 460
   Cys Arg Ile Leu Gly Val Gly Ile Ser Thr Gly Gly Arg Val Asn Pro
   465                 470                 475                 480
   Arg Glu Gly Ile Val Leu His Ser Thr Lys Leu Ile Gln Glu Trp Asn
                   485                 490                 495
   Ser Val Asp Leu Arg Thr Pro Leu Ser Asp Thr Leu His Leu Pro Val
               500                 505                 510
   Trp Val Asp Asn Asp Gly Asn Cys Ala Ala Leu Ala Glu Arg Lys Phe
           515                 520                 525
   Gly Gln Gly Lys Gly Leu Glu Asn Phe Val Thr Leu Ile Thr Gly Thr
       530                 535                 540
   Gly Ile Gly Gly Gly Ile Ile His Gln His Glu Leu Ile His Gly Ser
   545                 550                 555                 560
   Ser Phe Cys Ala Ala Glu Leu Gly His Leu Val Val Ser Leu Asp Gly
                   565                 570                 575
   Pro Asp Cys Ser Cys Gly Ser His Gly Cys Ile Glu Ala Tyr Ala Ser
               580                 585                 590
   Gly Met Ala Leu Gln Arg Glu Ala Lys Lys Leu His Asp Glu Asp Leu
           595                 600                 605
   Leu Leu Val Glu Gly Met Ser Val Pro Lys Asp Glu Ala Val Gly Ala
       610                 615                 620
   Leu His Leu Ile Gln Ala Ala Lys Leu Gly Asn Ala Lys Ala Gln Ser
   625                 630                 635                 640
   Ile Leu Arg Thr Ala Gly Thr Ala Leu Gly Leu Gly Val Val Asn Ile
                   645                 650                 655
   Leu His Thr Met Asn Pro Ser Leu Val Ile Leu Ser Gly Val Leu Ala
               660                 665                 670
   Ser His Tyr Ile His Ile Val Lys Asp Val Ile Arg Gln Gln Ala Leu
           675                 680                 685
   Ser Ser Val Gln Asp Val Asp Val Val Val Ser Asp Leu Val Asp Pro
       690                 695                 700
   Ala Leu Leu Gly Ala Ala Ser Met Val Leu Asp Tyr Thr Thr Arg Arg
   705                 710                 715                 720
   Ile Tyr
<210> 28
   <211> 2166
   <212> DNA
   <213> Homo sapiens
<220>
   <223> GNE
<400> 28
   atggagaaga atggaaataa ccgaaagctg cgggtttgtg ttgctacttg taaccgtgca 60
   gattattcta aacttgcccc gatcatgttt ggcattaaaa ccgaacctga gttctttgaa 120
   cttgatgttg tggtacttgg ctctcacctg atagatgact atggaaatac atatcgaatg 180
   attgaacaag atgactttga cattaacacc aggctacaca caattgtgag gggagaagat 240
   gaggcagcca tggtggagtc agtaggcctg gccctagtga agctgccaga tgtccttaat 300
   cgcctgaagc ctgatatcat gattgttcat ggagacaggt ttgatgccct ggctctggcc 360
   acatctgctg ccttgatgaa catccgaatc cttcacattg aaggtgggga agtcagtggg 420
   accattgatg actctatcag acatgccata acaaaactgg ctcattatca tgtgtgctgc 480
   acccgcagtg cagagcagca cctgatatcc atgtgtgagg accatgatcg catccttttg 540
   gcaggctgcc cttcctatga caaacttctc tcagccaaga acaaagacta catgagcatc 600
   attcgcatgt ggctaggtga tgatgtaaaa tctaaagatt acattgttgc actacagcac 660
   cctgtgacca ctgacattaa gcattccata aaaatgtttg aattaacatt ggatgcactt 720
   atctcattta acaagcggac cctagtcctg tttccaaata ttgacgcagg gagcaaagag 780
   atggttcgag tgatgcggaa gaagggcatt gagcatcatc ccaactttcg tgcagttaaa 840
   cacgtcccat ttgaccagtt tatacagttg gttgcccatg ctggctgtat gattgggaac 900
   agcagctgtg gggttcgaga agttggagct tttggaacac ctgtgatcaa cctgggaaca 960
   cgtcagattg gaagagaaac aggggagaat gttcttcatg tccgggatgc tgacacccaa 1020
   gacaaaatat tgcaagcact gcaccttcag tttggtaaac agtacccttg ttcaaagata 1080
   tatggggatg gaaatgctgt tccaaggatt ttgaagtttc tcaaatctat cgatcttcaa 1140
   gagccactgc aaaagaaatt ctgctttcct cctgtgaagg agaatatctc tcaagatatt 1200
   gaccatattc ttgaaactct aagtgccttg gccgttgatc ttggcgggac gaacctccga 1260
   gttgcaatag tcagcatgaa gggtgaaata gttaagaagt atactcagtt caatcctaaa 1320
   acctatgaag agaggattaa tttaatccta cagatgtgtg tggaagctgc agcagaagct 1380
   gtaaaactga actgcagaat tttgggagta ggcatttcca caggtggccg tgtaaatcct 1440
   cgggaaggaa ttgtgctgca ttcaaccaaa ctgatccaag agtggaactc tgtggacctt 1500
   aggacccccc tttctgacac tttgcatctc cctgtgtggg tagacaatga tggcaactgt 1560
   gctgccctgg cggaaaggaa atttggccaa ggaaagggac tggaaaactt tgttacactt 1620
   atcacaggca caggaatcgg tggtggaatt atccatcagc atgaattgat ccacggaagc 1680
   tccttctgtg ctgcagaact gggccacctt gttgtgtctc tggatgggcc tgattgttcc 1740
   tgtggaagcc atgggtgcat tgaagcatac gcctctggaa tggccttgca gagggaggca 1800
   aaaaagctcc atgatgagga cctgctcttg gtggaaggga tgtcagtgcc aaaagatgag 1860
   gctgtgggtg cgctccatct catccaagct gcgaaacttg gcaatgcgaa ggcccagagc 1920
   atcctaagaa cagctggaac agctttgggt cttggggttg tgaacatcct ccataccatg 1980
   aatccctccc ttgtgatcct ctccggagtc ctggccagtc actatatcca cattgtcaaa 2040
   gacgtcattc gccagcaggc cttgtcctcc gtgcaggacg tggatgtggt ggtttcggat 2100
   ttggttgacc ccgccctgct gggtgctgcc agcatggttc tggactacac aacacgcagg 2160
   atctac 2166
<210> 29
   <211> 722
   <212> PRT
   <213> Rattus norvegicus
<220>
   <223> GNE
<400> 29
   Met Glu Lys Asn Gly Asn Asn Arg Lys Leu Arg Val Cys Val Ala Thr
   1               5                   10                  15
   Cys Asn Arg Ala Asp Tyr Ser Lys Leu Ala Pro Ile Met Phe Gly Ile
               20                  25                  30
   Lys Thr Glu Pro Ala Phe Phe Glu Leu Asp Val Val Val Leu Gly Ser
           35                  40                  45
   His Leu Ile Asp Asp Tyr Gly Asn Thr Tyr Arg Met Ile Glu Gln Asp
       50                  55                  60
   Asp Phe Asp Ile Asn Thr Arg Leu His Thr Ile Val Arg Gly Glu Asp
   65                  70                  75                  80
   Glu Ala Ala Met Val Glu Ser Val Gly Leu Ala Leu Val Lys Leu Pro
                   85                  90                  95
   Asp Val Leu Asn Arg Leu Lys Pro Asp Ile Met Ile Val His Gly Asp
               100                 105                 110
   Arg Phe Asp Ala Leu Ala Leu Ala Thr Ser Ala Ala Leu Met Asn Ile
           115                 120                 125
   Arg Ile Leu His Ile Glu Gly Gly Glu Val Ser Gly Thr Ile Asp Asp
       130                 135                 140
   Ser Ile Arg His Ala Ile Thr Lys Leu Ala His Tyr His Val Cys Cys
   145                 150                 155                 160
   Thr Arg Ser Ala Glu Gln His Leu Ile Ser Met Cys Glu Asp His Asp
                   165                 170                 175
   Arg Ile Leu Leu Ala Gly Cys Pro Ser Tyr Asp Lys Leu Leu Ser Ala
               180                 185                 190
   Lys Asn Lys Asp Tyr Met Ser Ile Ile Arg Met Trp Leu Gly Asp Asp
           195                 200                 205
   Val Lys Cys Lys Asp Tyr Ile Val Ala Leu Gln His Pro Val Thr Thr
       210                 215                 220
   Asp Ile Lys His Ser Ile Lys Met Phe Glu Leu Thr Leu Asp Ala Leu
   225                 230                 235                 240
   Ile Ser Phe Asn Lys Arg Thr Leu Val Leu Phe Pro Asn Ile Asp Ala
                   245                 250                 255
   Gly Ser Lys Glu Met Val Arg Val Met Arg Lys Lys Gly Ile Glu His
               260                 265                 270
   His Pro Asn Phe Arg Ala Val Lys His Val Pro Phe Asp Gln Phe Ile
           275                 280                 285
   Gln Leu Val Ala His Ala Gly Cys Met Ile Gly Asn Ser Ser Cys Gly
       290                 295                 300
   Val Arg Glu Val Gly Ala Phe Gly Thr Pro Val Ile Asn Leu Gly Thr
   305                 310                 315                 320
   Arg Gln Ile Gly Arg Glu Thr Gly Glu Asn Val Leu His Val Arg Asp
                   325                 330                 335
   Ala Asp Thr Gln Asp Lys Ile Leu Gln Ala Leu His Leu Gln Phe Gly
               340                 345                 350
   Lys Gln Tyr Pro Cys Ser Lys Ile Tyr Gly Asp Gly Asn Ala Val Pro
           355                 360                 365
   Arg Ile Leu Lys Phe Leu Lys Ser Ile Asp Leu Gln Glu Pro Leu Gln
       370                 375                 380
   Lys Lys Phe Cys Phe Pro Pro Val Lys Glu Asn Ile Ser Gln Asp Ile
   385                 390                 395                 400
   Asp His Ile Leu Glu Thr Leu Ser Ala Leu Ala Val Asp Leu Gly Gly
                   405                 410                 415
   Thr Asn Leu Arg Val Ala Ile Val Ser Met Lys Gly Glu Ile Val Lys
               420                 425                 430
   Lys Tyr Thr Gln Phe Asn Pro Lys Thr Tyr Glu Glu Arg Ile Ser Leu
           435                 440                 445
   Ile Leu Gln Met Cys Val Glu Ala Ala Ala Glu Ala Val Lys Leu Asn
   450 455 460
   Cys Arg Ile Leu Gly Val Gly Ile Ser Thr Gly Gly Arg Val Asn Pro
   465 470 475 480
   Gln Glu Gly Val Val Leu His Ser Thr Lys Leu Ile Gln Glu Trp Asn
   485 490 495
   Ser Val Asp Leu Arg Thr Pro Leu Ser Asp Thr Leu His Leu Pro Val
   500 505 510
   Trp Val Asp Asn Asp Gly Asn Cys Ala Ala Met Ala Glu Arg Lys Phe
   515 520 525
   Gly Gln Gly Lys Gly Gln Glu Asn Phe Val Thr Leu Ile Thr Gly Thr
   530 535 540
   Gly Ile Gly Gly Gly Ile Ile His Gln His Glu Leu Ile His Gly Ser
   545 550 555 560
   Ser Phe Cys Ala Ala Glu Leu Gly His Leu Val Val Ser Leu Asp Gly
   565 570 575
   Pro Asp Cys Ser Cys Gly Ser His Gly Cys Ile Glu Ala Tyr Ala Ser
   580 585 590
   Gly Met Ala Leu Gln Arg Glu Ala Lys Lys Leu His Asp Glu Asp Leu
   595 600 605
   Leu Leu Val Glu Gly Met Ser Val Pro Lys Asp Glu Ala Val Gly Ala
   610 615 620
   Leu His Leu Ile Gln Ala Ala Lys Leu Gly Asn Val Lys Ala Gln Ser
   625 630 635 640
   Ile Leu Arg Thr Ala Gly Thr Ala Leu Gly Leu Gly Val Val Asn Ile
   645 650 655
   Leu His Thr Met Asn Pro Ser Leu Val Ile Leu Ser Gly Val Leu Ala
   660 665 670
   Ser His Tyr Ile His Ile Val Arg Asp Val Ile Arg Gln Gln Ala Leu
   675 680 685
   Ser Ser Val Gln Asp Val Asp Val Val Val Ser Asp Leu Val Asp Pro
   690 695 700
   Ala Leu Leu Gly Ala Ala Ser Met Val Leu Asp Tyr Thr Thr Arg Arg
   705 710 715 720
   Ile His
<210> 30
   <211> 2166
   <212> DNA
   <213> Rattus norvegicus
<220>
   <223> GNE
<400> 30
   atggagaaga acgggaataa ccggaagctt cgggtttgcg ttgccacctg caaccgagcc      60
   gattactcca aactggcccc catcatgttc ggcattaaga cggagcctgc gttcttcgag     120
   ctcgacgtgg tggtgctggg ctctcacctg atcgacgact acggaaacac ataccgcatg     180
   attgagcagg acgactttga catcaacacc aggctacaca cgattgttag aggggaagac     240
   gaagcagcca tggtagagtc agtgggccta gcgctagtga agctaccgga tgtcctcaac     300
   cgcctgaagc ctgacatcat gattgttcac ggagaccgat ttgacgccct cgctctggct     360
   acatctgctg ccctgatgaa catccgcatc cttcacattg aaggaggaga ggtcagcggg     420
   actattgatg actctatcag acacgccata acaaaactgg ctcactacca cgtgtgctgc     480
   accaggagtg cagagcaaca cctgatctcc atgtgtgagg accacgaccg catccttttg     540
   gctggctgcc cttcctatga caaactgctc tcagccaaga ataaagacta tatgagcatc     600
   attcggatgt ggctaggtga tgatgtaaaa tgtaaagatt acattgttgc cctgcaacac     660
   ccggtgacca ccgacattaa gcattccata aagatgttcg aactgacact ggatgctctt     720
   atctcattta acaagaggac cctagttctg tttccaaata tcgatgcagg cagcaaggag     780
   atggttcgag tgatgcggaa gaagggcatc gagcatcacc ccaatttccg cgcagtcaag     840
   cacgtcccgt ttgaccagtt cattcagctg gtcgcccacg ctggctgcat gattgggaat     900
   agcagctgtg gagtgcgtga ggttggcgcc tttggaaccc ctgtgatcaa cctgggcacg     960
   cggcagatag gaagagaaac gggggagaat gttcttcatg tccgggatgc tgacacccaa    1020
   gacaaaatat tacaagcact acacctccag ttcggtaaac agtacccttg ctcaaagata    1080
   tatggggatg gaaatgctgt tccaaggatt ttaaagtttc tcaaatccat cgaccttcaa    1140
   gagccactac agaagaaatt ctgcttccct cccgtgaagg agaacatctc tcaggatatt    1200
   gaccatatcc tcgaaactct gagtgccttg gctgttgatc tcggggggac gaatctgaga    1260
   gtggcgatag ttagcatgaa gggtgaaata gttaagaagt acacccagtt caatcctaaa    1320
   acctatgagg aaaggattag tctaatcctg cagatgtgtg tggaagcggc agcagaagcc    1380
   gtgaagctca attgcagaat tctgggagta ggcatctcca caggtggccg tgtgaatccc     1440
   caggaaggag ttgtgctgca ctcgaccaag ctgatacagg agtggaactc tgtggacctc     1500
   aggacaccac tctccgacac cctgcatctc cccgtgtggg tggacaacga cggcaactgc     1560
   gctgccatgg cggagaggaa gtttggccaa ggaaaaggac aggagaactt tgtgacgctc     1620
   atcacaggga cagggatcgg tgggggaatc atccaccagc acgagctgat ccacggcagc     1680
   tccttctgtg cggcagagct tggccacctc gtggtgtctc tggatggtcc tgactgctcc     1740
   tgtggaagcc atgggtgcat tgaagcctac gcctctggaa tggccttgca gagggaagca     1800
   aagaagctcc acgacgagga cctgctcttg gtggaaggga tgtcagtgcc aaaagacgaa     1860
   gctgtgggcg ccctccatct catccaagcc gccaagctgg gcaacgtgaa ggcccagagc     1920
   atcttacgga cagctggaac tgctttggga ctcggagttg tgaatatcct ccacactatg     1980
   aatccttccc tggtgatcct gtctggagtc ctggctagtc actacatcca cattgtgagg     2040
   gacgtcatcc gccagcaagc cctgtcctcc gtgcaggatg tggatgtagt ggtttcagac     2100
   ttggttgacc cggccctgct tggtgcggcc agcatggttc tggactacac gacccgcagg     2160
   atccac                                                                2166
<210> 31
   <211> 722
   <212> PRT
   <213> Mus musculus
<220>
   <223> GNE
<400> 31
   Met Glu Lys Asn Gly Asn Asn Arg Lys Leu Arg Val Cys Val Ala Thr
   1               5                   10                  15
   Cys Asn Arg Ala Asp Tyr Ser Lys Leu Ala Pro Ile Met Phe Gly Ile
               20                  25                  30
   Lys Thr Glu Pro Ala Phe Phe Glu Leu Asp Val Val Val Leu Gly Ser
           35                  40                  45
   His Leu Ile Asp Asp Tyr Gly Asn Thr Tyr Arg Met Ile Glu Gln Asp
       50                  55                  60
   Asp Phe Asp Ile Asn Thr Arg Leu His Thr Ile Val Arg Gly Glu Asp
   65                  70                  75                  80
   Glu Ala Ala Met Val Glu Ser Val Gly Leu Ala Leu Val Lys Leu Pro
                   85                  90                  95
   Asp Val Leu Asn Arg Leu Lys Pro Asp Ile Met Ile Val His Gly Asp
               100                 105                 110
   Arg Phe Asp Ala Leu Ala Leu Ala Thr Ser Ala Ala Leu Met Asn Ile
           115                 120                 125
   Arg Ile Leu His Ile Glu Gly Gly Glu Val Ser Gly Thr Ile Asp Asp
       130                 135                 140
   Ser Ile Arg His Ala Ile Thr Lys Leu Ala His Tyr His Val Cys Cys
   145                 150                 155                 160
   Thr Arg Ser Ala Glu Gln His Leu Ile Ser Met Cys Glu Asp His Asp
                   165                 170                 175
   Arg Ile Leu Leu Ala Gly Cys Pro Ser Tyr Asp Lys Leu Leu Ser Ala
               180                 185                 190
   Lys Asn Lys Asp Tyr Met Ser Ile Ile Arg Met Trp Leu Gly Asp Asp
           195                 200                 205
   Val Lys Cys Lys Asp Tyr Ile Val Ala Leu Gln His Pro Val Thr Thr
       210                 215                 220
   Asp Ile Lys His Ser Ile Lys Met Phe Glu Leu Thr Leu Asp Ala Leu
   225                 230                 235                 240
   Ile Ser Phe Asn Lys Arg Thr Leu Val Leu Phe Pro Asn Ile Asp Ala
                   245                 250                 255
   Gly Ser Lys Glu Met Val Arg Val Met Arg Lys Lys Gly Ile Glu His
               260                 265                 270
   His Pro Asn Phe Arg Ala Val Lys His Val Pro Phe Asp Gln Phe Ile
           275                 280                 285
   Gln Leu Val Ala His Ala Gly Cys Met Ile Gly Asn Ser Ser Cys Gly
       290                 295                 300
   Val Arg Glu Val Gly Ala Phe Gly Thr Pro Val Ile Asn Leu Gly Thr
   305                 310                 315                 320
   Arg Gln Ile Gly Arg Glu Thr Gly Glu Asn Val Leu His Val Arg Asp
                   325                 330                 335
   Ala Asp Thr Gln Asp Lys Ile Leu Gln Ala Leu His Leu Gln Phe Gly
               340                 345                 350
   Lys Gln Tyr Pro Cys Ser Lys Ile Tyr Gly Asp Gly Asn Ala Val Pro
           355                 360                 365
   Arg Ile Leu Lys Phe Leu Lys Ser Ile Asp Leu Gln Glu Pro Leu Gln
       370                 375                 380
   Lys Lys Phe Cys Phe Pro Pro Val Lys Glu Asn Ile Ser Gln Asp Ile
   385                 390                 395                 400
   Asp His Ile Leu Glu Thr Leu Ser Ala Leu Ala Val Asp Leu Gly Gly
                   405                 410                 415
   Thr Asn Leu Arg Val Ala Ile Val Ser Met Lys Gly Glu Ile Val Lys
               420                 425                 430
   Lys Tyr Thr Gln Phe Asn Pro Lys Thr Tyr Glu Glu Arg Ile Ser Leu
           435                 440                 445
   Ile Leu Gln Met Cys Val Glu Ala Ala Ala Glu Ala Val Lys Leu Asn
       450                 455                 460
   Cys Arg Ile Leu Gly Val Gly Ile Ser Thr Gly Gly Arg Val Asn Pro
   465                 470                 475                 480
   Gln Glu Gly Val Val Leu His Ser Thr Lys Leu Ile Gln Glu Trp Asn
                   485                 490                 495
   Ser Val Asp Leu Arg Thr Pro Leu Ser Asp Thr Leu His Leu Pro Val
               500                 505                 510
   Trp Val Asp Asn Asp Gly Asn Cys Ala Ala Met Ala Glu Arg Lys Phe
           515                 520                 525
   Gly Gln Gly Lys Gly Gln Glu Asn Phe Val Thr Leu Ile Thr Gly Thr
       530                 535                 540
   Gly Ile Gly Gly Gly Ile Ile His Gln His Glu Leu Ile His Gly Ser
   545                 550                 555                 560
   Ser Phe Cys Ala Ala Glu Leu Gly His Leu Val Val Ser Leu Asp Gly
                   565                 570                 575
   Pro Asp Cys Ser Cys Gly Ser His Gly Cys Ile Glu Ala Tyr Ala Ser
               580                 585                 590
   Gly Met Ala Leu Gln Arg Glu Ala Lys Lys Leu His Asp Glu Asp Leu
           595                 600                 605
   Leu Leu Val Glu Gly Met Ser Val Pro Lys Asp Glu Ala Val Gly Ala
   610 615 620
   Leu His Leu Ile Gln Ala Ala Lys Leu Gly Asn Val Lys Ala Gln Ser
   625 630 635 640
   Ile Leu Arg Thr Ala Gly Thr Ala Leu Gly Leu Gly Val Val Asn Ile
   645 650 655
   Leu His Thr Met Asn Pro Ser Leu Val Ile Leu Ser Gly Val Leu Ala
   660 665 670
   Ser His Tyr Ile His Ile Val Lys Asp Val Ile Arg Gln Gln Ala Leu
   675 680 685
   Ser Ser Val Gln Asp Val Asp Val Val Val Ser Asp Leu Val Asp Pro
   690 695 700
   Ala Leu Leu Gly Ala Ala Ser Met Val Leu Asp Tyr Thr Thr Arg Arg
   705 710 715 720
   Ile His
<210> 32
   <211> 2166
   <212> DNA
   <213> Mus musculus
<220>
   <223> GNE
<400> 32
   atggagaaga acgggaacaa ccgaaagctc cgggtttgcg ttgccacctg caaccgagct 60
   gactactcca aactggcccc gatcatgttc ggcatcaaga cagagcccgc gttctttgag 120
   ttggacgtgg tggtgctcgg ctcccacctg attgacgact atggaaacac ataccgcatg 180
   attgagcaag atgactttga cattaacacc aggctccaca cgattgttag aggggaagat 240
   gaagcggcca tggtagagtc ggtaggccta gcgctcgtga agctaccgga cgtcctcaat 300
   cgcctgaagc ccgacatcat gattgttcac ggagaccgat ttgacgccct tgctctggct 360
   acgtctgctg ccttgatgaa catccgcatc cttcacattg aaggaggcga ggtcagcggg 420
   accattgatg actctatcag acacgccata acaaaactgg ctcactacca tgtgtgctgc 480
   actagaagtg cagagcagca cctgatctct atgtgcgagg accacgaccg catcctgttg 540
   gcaggctgcc cttcctatga caaactgctc tccgccaaga acaaagacta tatgagcatc 600
   attcggatgt ggctaggcga tgatgtaaaa tgtaaggatt acatcgttgc cctgcagcat      660
   cccgtgacca ctgacattaa gcattccata aagatgtttg agctaacact ggatgccctg      720
   atctcgttta acaagaggac cctagttctg tttccaaata tcgatgcagg cagcaaggag      780
   atggttcgag tgatgcggaa gaagggcatc gagcatcacc ccaatttccg tgcagtcaag      840
   cacgtcccgt ttgaccagtt catacagctg gtcgcccacg ctggctgcat gattgggaat      900
   agcagctgcg gcgtgcgaga ggttggcgct ttcggaacac ccgtgatcaa cctgggcaca      960
   aggcagatag gaagagaaac cggggagaat gttcttcatg tcagggatgc tgacacccaa     1020
   gataaaatat tgcaagcact acacctccag ttcggcaaac agtacccttg ctcaaagata     1080
   tatggggatg ggaatgctgt tccaaggatt ttaaagtttc tcaaatccat tgaccttcaa     1140
   gagccactac agaagaaatt ctgcttcccc cctgtaaagg agaacatctc tcaagacatt     1200
   gaccacatcc tggaaactct gagtgccttg gctgttgatc ttggcgggac aaacctgagg     1260
   gtggcaatag ttagcatgaa gggtgaaatc gttaagaagt acactcagtt caaccctaaa     1320
   acctatgaag aaaggattag tttaatcctg cagatgtgtg tggaagctgc cgcggaagct     1380
   gtgaaactca attgcagaat tctgggagta ggcatctcca caggtggccg cgtgaatccc     1440
   caggaaggag ttgtgctgca ttcaaccaag ctgatccagg aatggaactc cgtggacctc     1500
   aggacacccc tctccgacac cctgcatctc cccgtgtggg tggacaatga cggcaactgt     1560
   gccgccatgg cagagaggaa gttcggccaa ggaaaaggac aggagaactt cgtgacgctc     1620
   atcacgggga cagggatcgg tggggggatc atccaccagc acgaactgat ccacggcagc     1680
   tccttctgcg cggcggagct cggccatctc gtggtgtccc tggacggtcc tgactgctcc     1740
   tgtggaagcc atgggtgcat cgaagcgtac gcctctggaa tggccttgca gagggaagca     1800
   aagaaactcc atgatgagga cctgctcttg gtggaaggga tgtcagtacc aaaagacgaa     1860
   gctgtgggtg ccctccatct catccaggct gccaagctgg gcaacgtgaa ggcccagagc     1920
   atcttacgaa cagctggaac tgctttggga cttggggttg tgaacatcct ccacactatg     1980
   aatccttccc tggtgatcct gtctggagtc ctggccagtc actacatcca catcgtgaag     2040
   gacgtcatcc gccagcaagc cttgtcctcc gtgcaggatg tggacgtggt ggtctcagac     2100
   ttggtggacc cggccctgct tggcgcagcc agcatggttc tggactacac aacgcgcagg     2160
   atccac                                                                2166
<210> 33
   <211> 359
   <212> PRT
   <213> Homo sapiens
<220>
   <223> NANS
<400> 33
   Met Pro Leu Glu Leu Glu Leu Cys Pro Gly Arg Trp Val Gly Gly Gln
   1               5                   10                  15
   His Pro Cys Phe Ile Ile Ala Glu Ile Gly Gln Asn His Gln Gly Asp
               20                  25                  30
   Leu Asp Val Ala Lys Arg Met Ile Arg Met Ala Lys Glu Cys Gly Ala
           35                  40                  45
   Asp Cys Ala Lys Phe Gln Lys Ser Glu Leu Glu Phe Lys Phe Asn Arg
       50                  55                  60
   Lys Ala Leu Glu Arg Pro Tyr Thr Ser Lys His Ser Trp Gly Lys Thr
   65                  70                  75                  80
   Tyr Gly Glu His Lys Arg His Leu Glu Phe Ser His Asp Gln Tyr Arg
                   85                  90                  95
   Glu Leu Gln Arg Tyr Ala Glu Glu Val Gly Ile Phe Phe Thr Ala Ser
               100                 105                 110
   Gly Met Asp Glu Met Ala Val Glu Phe Leu His Glu Leu Asn Val Pro
           115                 120                 125
   Phe Phe Lys Val Gly Ser Gly Asp Thr Asn Asn Phe Pro Tyr Leu Glu
       130                 135                 140
   Lys Thr Ala Lys Lys Gly Arg Pro Met Val Ile Ser Ser Gly Met Gln
   145                 150                 155                 160
   Ser Met Asp Thr Met Lys Gln Val Tyr Gln Ile Val Lys Pro Leu Asn
                   165                 170                 175
   Pro Asn Phe Cys Phe Leu Gln Cys Thr Ser Ala Tyr Pro Leu Gln Pro
               180                 185                 190
   Glu Asp Val Asn Leu Arg Val Ile Ser Glu Tyr Gln Lys Leu Phe Pro
           195                 200                 205
   Asp Ile Pro Ile Gly Tyr Ser Gly His Glu Thr Gly Ile Ala Ile Ser
       210                 215                 220
   Val Ala Ala Val Ala Leu Gly Ala Lys Val Leu Glu Arg His Ile Thr
   225                 230                 235                 240
   Leu Asp Lys Thr Trp Lys Gly Ser Asp His Ser Ala Ser Leu Glu Pro
                   245                 250                 255
   Gly Glu Leu Ala Glu Leu Val Arg Ser Val Arg Leu Val Glu Arg Ala
               260                 265                 270
   Leu Gly Ser Pro Thr Lys Gln Leu Leu Pro Cys Glu Met Ala Cys Asn
           275                 280                 285
   Glu Lys Leu Gly Lys Ser Val Val Ala Lys Val Lys Ile Pro Glu Gly
       290                 295                 300
   Thr Ile Leu Thr Met Asp Met Leu Thr Val Lys Val Gly Glu Pro Lys
   305                 310                 315                 320
   Gly Tyr Pro Pro Glu Asp Ile Phe Asn Leu Val Gly Lys Lys Val Leu
                   325                 330                 335
   Val Thr Val Glu Glu Asp Asp Thr Ile Met Glu Glu Leu Val Asp Asn
               340                 345                 350
   His Gly Lys Lys Ile Lys Ser
           355
<210> 34
   <211> 1077
   <212> DNA
   <213> Homo sapiens
<220>
   <223> NANS
<400> 34
   atgccgctgg agctggagct gtgtcccggg cgctgggtgg gcgggcaaca cccgtgcttc      60
   atcattgccg agatcggcca gaaccaccag ggcgacctgg atgtagccaa gcgcatgatc     120
   cgcatggcca aggagtgtgg ggctgattgt gccaagttcc agaagagtga gctagaattc     180
   aagtttaatc ggaaagcctt ggagaggcca tacacctcga agcattcctg ggggaagacg     240
   tacggggagc acaaacgaca tctggagttc agccatgacc agtacaggga gctgcagagg     300
   tacgccgagg aggttgggat cttcttcact gcctctggca tggatgagat ggcagttgaa     360
   ttcctgcatg aactgaatgt tccatttttc aaagttggat ctggagacac taataatttt     420
   ccttatctgg aaaagacagc caaaaaaggt cgcccaatgg tgatctccag tgggatgcag     480
   tcaatggaca ccatgaagca agtttatcag atcgtgaagc ccctcaaccc caacttctgc     540
   ttcttgcagt gtaccagcgc atacccgctc cagcctgagg acgtcaacct gcgggtcatc     600
   tcggaatatc agaagctctt tcctgacatt cccatagggt attctgggca tgaaacaggc     660
   atagcgatat ctgtggccgc agtggctctg ggggccaagg tgttggaacg tcacataact     720
   ttggacaaga cctggaaggg gagtgaccac tcggcctcgc tggagcctgg agaactggcc     780
   gagctggtgc ggtcagtgcg tcttgtggag cgtgccctgg gctccccaac caagcagctg     840
   ctgccctgtg agatggcctg caatgagaag ctgggcaagt ctgtggtggc caaagtgaaa     900
   attccggaag gcaccattct aacaatggac atgctcaccg tgaaggtggg tgagcccaaa     960
   ggctatcctc ctgaagacat ctttaatcta gtgggcaaga aggtcctggt cactgttgaa    1020
   gaggatgaca ccatcatgga agaattggta gataatcatg gcaaaaaaat caagtct       1077
<210> 35
   <211> 359
   <212> PRT
   <213> Rattus norvegicus
<220>
   <223> NANS
<400> 35
   Met Pro Leu Glu Leu Glu Leu Cys Pro Gly Arg Trp Val Gly Gly Gln
   1               5                   10                  15
   His Pro Cys Phe Ile Ile Ala Glu Ile Gly Gln Asn His Gln Gly Asp
               20                  25                  30
   Leu Asp Val Ala Lys Arg Met Ile Arg Thr Ala Lys Glu Cys Gly Ala
           35                  40                  45
   Asp Cys Ala Lys Phe Gln Lys Ser Glu Leu Glu Phe Lys Phe Asn Arg
       50                  55                  60
   Lys Ala Leu Glu Arg Pro Tyr Thr Ser Lys His Ser Trp Gly Lys Thr
   65                  70                  75                  80
   Tyr Gly Glu His Lys Arg His Leu Glu Phe Ser His Asp Gln Tyr Lys
                   85                  90                  95
   Glu Leu Gln Ser Tyr Ala Gln Glu Ile Gly Ile Phe Phe Thr Ala Ser
               100                 105                 110
   Gly Met Asp Glu Met Ala Val Glu Phe Leu His Glu Leu Asn Val Pro
           115                 120                 125
   Phe Phe Lys Val Gly Ser Gly Asp Thr Asn Asn Phe Pro Tyr Leu Glu
       130                 135                 140
   Lys Thr Ala Lys Lys Gly Arg Pro Met Val Ile Ser Ser Gly Met Gln
   145                 150                 155                 160
   Ser Met Asp Thr Met Lys Gln Val Tyr Gln Ile Val Lys Pro Leu Asn
   165 170 175
   Pro Asn Phe Cys Phe Leu Gln Cys Thr Ser Ala Tyr Pro Leu Gln Pro
   180 185 190
   Glu Asp Ala Asn Leu Arg Val Ile Ser Glu Tyr Gln Lys Leu Phe Pro
   195 200 205
   Asp Ile Pro Ile Gly Tyr Ser Gly His Glu Thr Gly Ile Ala Ile Ser
   210 215 220
   Val Ala Ala Val Ala Leu Gly Ala Lys Val Leu Glu Arg His Ile Thr
   225 230 235 240
   Leu Asp Lys Thr Trp Lys Gly Ser Asp His Leu Ala Ser Leu Glu Pro
   245 250 255
   Gly Glu Leu Ala Glu Leu Val Arg Ser Val Arg Leu Val Glu Arg Ala
   260 265 270
   Leu Gly Ser Pro Ala Lys Gln Leu Leu Pro Cys Glu Met Ala Cys Asn
   275 280 285
   Glu Lys Leu Gly Lys Ser Val Val Ala Lys Val Lys Ile Pro Ala Gly
   290 295 300
   Thr Ile Leu Thr Leu Asp Met Leu Thr Val Lys Val Gly Glu Pro Lys
   305 310 315 320
   Gly Tyr Pro Pro Glu Asp Ile Phe Asn Leu Val Gly Lys Lys Val Leu
   325 330 335
   Val Thr Ile Glu Glu Asp Asp Thr Val Met Glu Glu Ser Val Glu Ser
   340 345 350
   Gln Ser Lys Lys Ile Lys Ala
   355
<210> 36
   <211> 1077
   <212> DNA
   <213> Rattus norvegicus
<220>
   <223> NANS
<400> 36
   atgccgctgg agctggagct gtgtcccggg cgctgggtgg gtggacagca cccgtgcttc        60
   atcatcgcgg agatcggcca gaaccaccaa ggagacttag atgtggccaa gcgcatgatc       120
   cgcactgcca aggagtgtgg ggccgactgc gctaagtttc agaagagtga gttggagttc       180
   aagtttaacc ggaaggccct ggagagacca tatacttcga agcattcatg ggggaagact        240
   tacggggagc acaaacgaca tctagaattc agccacgatc agtacaagga gctgcagagc       300
   tacgcgcagg agattggcat cttcttcact gcgtctggca tggatgagat ggcagttgag       360
   tttctgcatg aactgaatgt tccctttttc aaagttggat ctggagacac taacaatttt       420
   ccctacctgg aaaagacagc caagaaaggt cgtcctatgg tgatctccag tgggatgcag       480
   tccatggaca ccatgaagca agtctatcag atcgtgaagc ccctgaatcc caacttctgc       540
   ttcctccagt gcaccagtgc gtacccacta cagcctgagg atgccaacct gcgcgtcatc       600
   tcggaatacc agaagctctt tcctgacatt cccatagggt attctgggca cgagacaggc       660
   atcgccatat ctgtagctgc agtggccctg ggggccaagg tgttggaacg tcatataact        720
   ttggacaaga cctggaaggg gagtgaccac ttagcctcac tggagcctgg agaactggca       780
   gagctggtgc ggtctgtgcg tctggtggag cgggcactgg gctccccagc caagcagctc       840
   ctgccctgtg agatggcctg caacgagaag ctcggcaagt ccgtggtagc caaagtgaaa       900
   atcccagcag gcaccatcct gaccctggac atgctcactg tgaaggtggg ggaacccaaa       960
   ggctatcctc ctgaagacat cttcaacctg gtgggcaaaa aggtgctggt cactattgaa      1020
   gaagatgaca cggtcatgga ggaatccgtg gaaagtcaaa gcaagaaaat caaggct         1077
<210> 37
   <211> 359
   <212> PRT
   <213> Mus musculus
<220>
   <223> NANS
<400> 37
   Met Pro Leu Glu Leu Glu Leu Cys Pro Gly Arg Trp Val Gly Gly Lys
   1               5                   10                  15
   His Pro Cys Phe Ile Ile Ala Glu Ile Gly Gln Asn His Gln Gly Asp
               20                  25                  30
   Ile Asp Val Ala Lys Arg Met Ile Arg Thr Ala Lys Glu Cys Gly Ala
           35                  40                  45
   Asp Cys Ala Lys Phe Gln Lys Ser Glu Leu Glu Phe Lys Phe Asn Arg
       50                  55                  60
   Lys Ala Leu Glu Arg Pro Tyr Thr Ser Lys His Ser Trp Gly Lys Thr
   65 70 75 80
   Tyr Gly Glu His Lys Arg His Leu Glu Phe Ser His Asp Gln Tyr Lys
   85 90 95
   Glu Leu Gln Ser Tyr Ala Gln Glu Ile Gly Ile Phe Phe Thr Ala Ser
   100 105 110
   Gly Met Asp Glu Met Ala Val Glu Phe Leu His Glu Leu Asn Val Pro
   115 120 125
   Phe Phe Lys Val Gly Ser Gly Asp Thr Asn Asn Phe Pro Tyr Leu Glu
   130 135 140
   Lys Thr Ala Lys Lys Gly Arg Pro Met Val Ile Ser Ser Gly Met Gln
   145 150 155 160
   Ser Met Asp Thr Met Lys Gln Val Tyr Gln Ile Val Lys Pro Leu Asn
   165 170 175
   Pro Asn Phe Cys Phe Leu Gln Cys Thr Ser Ala Tyr Pro Leu Gln Pro
   180 185 190
   Glu Asp Ala Asn Leu Arg Val Ile Ser Glu Tyr Gln Lys Leu Phe Pro
   195 200 205
   Asp Ile Pro Ile Gly Tyr Ser Gly His Glu Thr Gly Ile Ala Ile Ser
   210 215 220
   Val Ala Ala Val Ala Leu Gly Ala Lys Val Leu Glu Arg His Ile Thr
   225 230 235 240
   Leu Asp Lys Thr Trp Lys Gly Ser Asp His Ser Ala Ser Leu Glu Pro
   245 250 255
   Gly Glu Leu Ala Glu Leu Val Arg Ser Val Arg Leu Val Glu Arg Ala
   260 265 270
   Leu Gly Ser Pro Thr Lys Gln Leu Leu Pro Cys Glu Met Ala Cys Asn
   275 280 285
   Glu Lys Leu Gly Lys Ser Val Val Ala Lys Val Lys Ile Pro Ala Gly
   290 295 300
   Thr Thr Leu Thr Leu Asp Met Leu Thr Val Lys Val Gly Glu Pro Lys
   305 310 315 320
   Gly Tyr Pro Pro Glu Asp Ile Phe Asn Leu Ala Gly Lys Lys Val Leu
   325 330 335
   Val Thr Ile Glu Glu Asp Asp Thr Val Met Glu Glu Ser Val Glu Ser
   340 345 350
   His Ser Lys Lys Ile Lys Ala
   355
<210> 38
   <211> 1077
   <212> DNA
   <213> Mus musculus
<220>
   <223> NANS
<400> 38
   atgccgctgg aactggagct gtgtcccggg cgctgggtgg gtggaaagca cccgtgcttc      60
   atcatcgcgg agatcggcca gaaccaccaa ggagacatag atgtggccaa gcgcatgatc     120
   cgcactgcca aggagtgtgg ggccgactgc gctaagtttc agaagagcga gttggagttc     180
   aagtttaacc ggaaggccct ggagagacca tatacttcga agcattcatg ggggaagacg      240
   tatggggagc acaagcggca tctggaattc agccacgacc agtacaagga gctgcagagc     300
   tatgcgcagg agatcggcat cttcttcact gcctctggca tggacgagat ggcagttgag     360
   tttctgcacg aactgaatgt tccctttttc aaagttggat ctggggacac taacaatttt     420
   ccctacctgg aaaagacagc caagaaaggt cgtcctatgg tgatctccag cgggatgcag     480
   tcaatggaca ccatgaagca agtctatcag atcgtgaagc cgctgaatcc caacttctgc     540
   ttcctccaat gcaccagcgc gtacccacta cagcccgagg atgccaacct gcgcgtcatc     600
   tcggaatacc agaagctctt tcccgacatt cccatcgggt attccgggca cgagacgggc     660
   atcgccatat ctgtggccgc cgtggctctg ggggccaagg tgttggaacg tcacataacg      720
   ttggacaaga cctggaaggg gagtgaccac tcagcctcgc tggagcctgg ggagctggca     780
   gagctggtgc ggtctgtgcg cctggtggag cgggccctgg gctccccaac caagcagctg     840
   ctgccctgtg agatggcctg caatgagaag ctcggcaagt ctgtggtagc caaagtgaaa     900
   atcccagcag gcaccaccct gaccctggac atgctcactg tgaaggtggg ggagcccaaa     960
   ggctatcctc ctgaagacat cttcaaccta gcgggcaaaa aggtgctggt cactatcgaa    1020
   gaagatgaca cggtcatgga agaatccgtg gaaagtcaca gcaagaaaat caaggct       1077
<210> 39
   <211> 248
   <212> PRT
   <213> Homo sapiens
<220>
   <223> NANP
<400> 39
   Met Gly Leu Ser Arg Val Arg Ala Val Phe Phe Asp Leu Asp Asn Thr
   1               5                   10                  15
   Leu Ile Asp Thr Ala Gly Ala Ser Arg Arg Gly Met Leu Glu Val Ile
               20                  25                  30
   Lys Leu Leu Gln Ser Lys Tyr His Tyr Lys Glu Glu Ala Glu Ile Ile
           35                  40                  45
   Cys Asp Lys Val Gln Val Lys Leu Ser Lys Glu Cys Phe His Pro Tyr
       50                  55                  60
   Asn Thr Cys Ile Thr Asp Leu Arg Thr Ser His Trp Glu Glu Ala Ile
   65                  70                  75                  80
   Gln Glu Thr Lys Gly Gly Ala Ala Asn Arg Lys Leu Ala Glu Glu Cys
                   85                  90                  95
   Tyr Phe Leu Trp Lys Ser Thr Arg Leu Gln His Met Thr Leu Ala Glu
               100                 105                 110
   Asp Val Lys Ala Met Leu Thr Glu Leu Arg Lys Glu Val Arg Leu Leu
           115                 120                 125
   Leu Leu Thr Asn Gly Asp Arg Gln Thr Gln Arg Glu Lys Ile Glu Ala
       130                 135                 140
   Cys Ala Cys Gln Ser Tyr Phe Asp Ala Val Val Val Gly Gly Glu Gln
   145                 150                 155                 160
   Arg Glu Glu Lys Pro Ala Pro Ser Ile Phe Tyr Tyr Cys Cys Asn Leu
                   165                 170                 175
   Leu Gly Val Gln Pro Gly Asp Cys Val Met Val Gly Asp Thr Leu Glu
               180                 185                 190
   Thr Asp Ile Gln Gly Gly Leu Asn Ala Gly Leu Lys Ala Thr Val Trp
           195                 200                 205
   Ile Asn Lys Asn Gly Ile Val Pro Leu Lys Ser Ser Pro Val Pro His
       210                 215                 220
   Tyr Met Val Ser Ser Val Leu Glu Leu Pro Ala Leu Leu Gln Ser Ile
   225                 230                 235                 240
   Asp Cys Lys Val Ser Met Ser Thr
                   245
<210> 40
   <211> 744
   <212> DNA
   <213> Homo sapiens
<220>
   <223> NANP
<400> 40
   atggggctga gccgcgtgcg ggcggttttc tttgacttgg acaacactct catcgacacg      60
   gccggggcga gcaggagagg catgttggag gtgataaaac tcttacaatc aaaataccat     120
   tataaagaag aggctgaaat catctgtgat aaagttcaag ttaaactcag caaggaatgt     180
   tttcatcctt acaatacatg cattactgat ttaaggactt cacattggga agaagcaatc     240
   caggaaacaa aaggtggtgc agccaataga aaattggctg aagaatgtta tttcctttgg     300
   aaatctacac gtttacagca tatgacacta gcagaagacg tcaaagccat gcttactgaa     360
   cttcgaaagg aggtccgcct acttctatta acgaatgggg acagacagac ccagagggag     420
   aagattgagg cttgtgcctg tcagtcctat tttgacgctg ttgttgtagg tggagagcag     480
   agagaggaga aaccagcacc gtccatattt tattactgct gcaatcttct cggagtacaa     540
   cctggggact gtgtgatggt cggtgacaca ttagaaaccg acatccaagg aggcctcaat     600
   gcaggattga aagcaacagt ctggatcaat aaaaatggaa tagtgccact gaagtcctcc     660
   ccagttccgc attacatggt ttcttctgtg ctagagttac ctgctctctt acaaagtata     720
   gactgcaaag tcagtatgtc cact                                            744
<210> 41
   <211> 248
   <212> PRT
   <213> Rattus norvegicus
<220>
   <223> NANP
<400> 41
   Met Gly Leu Ser Arg Val Arg Ala Val Phe Phe Asp Leu Asp Asn Thr
   1               5                   10                  15
   Leu Ile Asp Thr Ala Gly Ala Ser Arg Arg Gly Met Leu Glu Val Ile
               20                  25                  30
   Lys Leu Leu Gln Ser Lys Tyr His Tyr Lys Glu Glu Ala Glu Val Ile
           35                  40                  45
   Cys Asp Lys Val Gln Val Lys Leu Ser Lys Glu Cys Phe His Pro Tyr
       50                  55                  60
   Ser Thr Cys Ile Thr Asp Val Arg Thr Ser His Trp Glu Glu Ala Ile
   65                  70                  75                  80
   Gln Glu Thr Lys Gly Gly Ala Asp Asn Arg Lys Leu Ala Glu Glu Cys
                   85                  90                  95
   Tyr Phe Leu Trp Lys Ser Thr Arg Leu Gln His Met Thr Leu Glu Glu
               100                 105                 110
   Asp Val Lys Ala Met Leu Thr Glu Leu Arg Lys Glu Val Arg Leu Leu
           115                 120                 125
   Leu Leu Thr Asn Gly Asp Arg Gln Thr Gln Arg Glu Lys Ile Glu Ala
       130                 135                 140
   Cys Ala Cys Gln Ser Tyr Phe Asp Ala Ile Val Val Gly Gly Glu Gln
   145                 150                 155                 160
   Lys Glu Glu Lys Pro Ala Pro Ser Ile Phe Tyr His Cys Cys Asp Leu
                   165                 170                 175
   Leu Gly Val Gln Pro Gly Asp Cys Val Met Val Gly Asp Thr Leu Glu
               180                 185                 190
   Thr Asp Ile Gln Gly Gly Leu Asn Ala Gly Leu Lys Ala Thr Val Trp
           195                 200                 205
   Ile Asn Lys Ser Gly Gly Val Pro Leu Thr Ser Ser Pro Met Pro His
       210                 215                 220
   Tyr Met Val Ser Ser Val Leu Glu Leu Pro Ala Leu Leu Gln Ser Ile
   225                 230                 235                 240
   Asp Cys Lys Val Ser Met Ser Val
                   245
<210> 42
   <211> 744
   <212> DNA
   <213> Rattus norvegicus
<220>
   <223> NANP
<400> 42
   atggggctga gtcgggtccg cgcggtcttc tttgacctgg acaacacact catcgacacg        60
   gccggggcga gcaggagagg catgttggag gtaataaagc tcttacaatc aaaataccac       120
   tacaaagaag aggctgaagt catctgcgat aaagttcaag ttaaactgag caaggagtgc       180
   tttcatccct atagtacatg cattacagat gtgaggactt cgcactggga agaagcaatc       240
   caggaaacca aaggtggtgc cgacaatagg aaattggctg aggaatgtta tttcctgtgg       300
   aaatctacac gcttacagca catgacccta gaagaagatg tcaaagccat gctcactgaa       360
   cttcgtaaag aggtccgcct actcttgtta acaaacggtg acagacagac acagagggaa       420
   aagatcgagg cttgtgcctg ccagtcttac tttgatgcca ttgttgtagg aggagaacag       480
   aaggaagaga aaccagcacc ttccatattt tatcactgct gtgatcttct tggagtgcag       540
   ccaggggact gtgtgatggt tggtgacaca ctagaaaccg atatacaagg aggcctgaat       600
   gcaggactga aagccacggt ctggataaac aagagtggag gagtgccact gacatcgtcc       660
   cccatgcctc actatatggt ttcctctgtg ttagaattac ctgctctctt gcaaagcata       720
   gattgcaaag tcagcatgtc tgtg                                              744
<210> 43
   <211> 248
   <212> PRT
   <213> Mus musculus
<220>
   <223> NANP
<400> 43
   Met Gly Leu Ser Arg Val Arg Ala Val Phe Phe Asp Leu Asp Asn Thr
   1               5                   10                  15
   Leu Ile Asp Thr Ala Gly Ala Ser Arg Arg Gly Met Leu Glu Val Ile
               20                  25                  30
   Lys Leu Leu Gln Ser Lys Tyr His Tyr Lys Glu Glu Ala Glu Ile Ile
           35                  40                  45
   Cys Asp Lys Val Gln Val Lys Leu Ser Lys Glu Cys Phe His Pro Tyr
       50                  55                  60
   Ser Thr Cys Ile Thr Asp Val Arg Thr Ser His Trp Glu Glu Ala Ile
   65                  70                  75                  80
   Gln Glu Thr Lys Gly Gly Ala Asp Asn Arg Lys Leu Ala Glu Glu Cys
                   85                  90                  95
   Tyr Phe Leu Trp Lys Ser Thr Arg Leu Gln His Met Ile Leu Ala Asp
               100                 105                 110
   Asp Val Lys Ala Met Leu Thr Glu Leu Arg Lys Glu Val Arg Leu Leu
           115                 120                 125
   Leu Leu Thr Asn Gly Asp Arg Gln Thr Gln Arg Glu Lys Ile Glu Ala
       130                 135                 140
   Cys Ala Cys Gln Ser Tyr Phe Asp Ala Ile Val Ile Gly Gly Glu Gln
   145                 150                 155                 160
   Lys Glu Glu Lys Pro Ala Pro Ser Ile Phe Tyr His Cys Cys Asp Leu
                   165                 170                 175
   Leu Gly Val Gln Pro Gly Asp Cys Val Met Val Gly Asp Thr Leu Glu
               180                 185                 190
   Thr Asp Ile Gln Gly Gly Leu Asn Ala Gly Leu Lys Ala Thr Val Trp
           195                 200                 205
   Ile Asn Lys Ser Gly Arg Val Pro Leu Thr Ser Ser Pro Met Pro His
       210                 215                 220
   Tyr Met Val Ser Ser Val Leu Glu Leu Pro Ala Leu Leu Gln Ser Ile
   225                 230                 235                 240
   Asp Cys Lys Val Ser Met Ser Val
                   245
<210> 44
   <211> 744
   <212> DNA
   <213> Mus musculus
<220>
   <223> NANP
<400> 44
   atggggctga gtcgggtccg cgcggtcttc tttgacctgg acaacacact catcgacacg        60
   gccggggcga gcaggagagg catgttggag gtaataaagc tcttacagtc aaaataccac       120
   tacaaagaag aggctgaaat catctgtgat aaagttcaag ttaaactgag caaggagtgc       180
   tttcatccct atagtacatg cattacagat gtgaggactt cacactggga agaagcaatc       240
   caggaaacca aaggaggtgc tgacaatagg aaattggcgg aggaatgtta tttcctgtgg       300
   aaatctacac gcttacagca catgatccta gcagacgatg tcaaagccat gctcactgaa       360
   cttcgaaaag aggtccgcct gctcctgtta acaaatggtg acagacagac tcagagggaa       420
   aagatcgagg cctgcgcctg ccagtcttac tttgatgcca ttgttattgg cggagaacag       480
   aaggaagaga aaccagcacc ttccatattt tatcactgct gtgatcttct tggagtgcag       540
   ccaggtgact gtgtgatggt tggtgacaca ctggaaaccg atatacaagg aggcctcaat       600
   gcaggactga aagctacggt ctggataaac aagagtggaa gagtgccgct gacatcatca       660
   cccatgcctc actatatggt ttcttctgtg ctagaattac ctgctctctt gcaaagcata       720
   gattgcaaag tcagcatgtc tgtg                                              744
<210> 45
   <211> 434
   <212> PRT
   <213> Homo sapiens
<220>
   <223> CAMS
<400> 45
   Met Asp Ser Val Glu Lys Gly Ala Ala Thr Ser Val Ser Asn Pro Arg
   1               5                   10                  15
   Gly Arg Pro Ser Arg Gly Arg Pro Pro Lys Leu Gln Arg Asn Ser Arg
               20                  25                  30
   Gly Gly Gln Gly Arg Gly Val Glu Lys Pro Pro His Leu Ala Ala Leu
           35                  40                  45
   Ile Leu Ala Arg Gly Gly Ser Lys Gly Ile Pro Leu Lys Asn Ile Lys
       50                  55                  60
   His Leu Ala Gly Val Pro Leu Ile Gly Trp Val Leu Arg Ala Ala Leu
   65                  70                  75                  80
   Asp Ser Gly Ala Phe Gln Ser Val Trp Val Ser Thr Asp His Asp Glu
                   85                  90                  95
   Ile Glu Asn Val Ala Lys Gln Phe Gly Ala Gln Val His Arg Arg Ser
               100                 105                 110
   Ser Glu Val Ser Lys Asp Ser Ser Thr Ser Leu Asp Ala Ile Ile Glu
           115                 120                 125
   Phe Leu Asn Tyr His Asn Glu Val Asp Ile Val Gly Asn Ile Gln Ala
       130                 135                 140
   Thr Ser Pro Cys Leu His Pro Thr Asp Leu Gln Lys Val Ala Glu Met
   145                 150                 155                 160
   Ile Arg Glu Glu Gly Tyr Asp Ser Val Phe Ser Val Val Arg Arg His
                   165                 170                 175
   Gln Phe Arg Trp Ser Glu Ile Gln Lys Gly Val Arg Glu Val Thr Glu
   180 185 190
   Pro Leu Asn Leu Asn Pro Ala Lys Arg Pro Arg Arg Gln Asp Trp Asp
   195 200 205
   Gly Glu Leu Tyr Glu Asn Gly Ser Phe Tyr Phe Ala Lys Arg His Leu
   210 215 220
   Ile Glu Met Gly Tyr Leu Gln Gly Gly Lys Met Ala Tyr Tyr Glu Met
   225 230 235 240
   Arg Ala Glu His Ser Val Asp Ile Asp Val Asp Ile Asp Trp Pro Ile
   245 250 255
   Ala Glu Gln Arg Val Leu Arg Tyr Gly Tyr Phe Gly Lys Glu Lys Leu
   260 265 270
   Lys Glu Ile Lys Leu Leu Val Cys Asn Ile Asp Gly Cys Leu Thr Asn
   275 280 285
   Gly His Ile Tyr Val Ser Gly Asp Gln Lys Glu Ile Ile Ser Tyr Asp
   290 295 300
   Val Lys Asp Ala Ile Gly Ile Ser Leu Leu Lys Lys Ser Gly Ile Glu
   305 310 315 320
   Val Arg Leu Ile Ser Glu Arg Ala Cys Ser Lys Gln Thr Leu Ser Ser
   325 330 335
   Leu Lys Leu Asp Cys Lys Met Glu Val Ser Val Ser Asp Lys Leu Ala
   340 345 350
   Val Val Asp Glu Trp Arg Lys Glu Met Gly Leu Cys Trp Lys Glu Val
   355 360 365
   Ala Tyr Leu Gly Asn Glu Val Ser Asp Glu Glu Cys Leu Lys Arg Val
   370 375 380
   Gly Leu Ser Gly Ala Pro Ala Asp Ala Cys Ser Thr Ala Gln Lys Ala
   385 390 395 400
   Val Gly Tyr Ile Cys Lys Cys Asn Gly Gly Arg Gly Ala Ile Arg Glu
   405 410 415
   Phe Ala Glu His Ile Cys Leu Leu Met Glu Lys Val Asn Asn Ser Cys
   420 425 430
   Gln Lys
<210> 46
   <211> 1302
   <212> DNA
   <213> Homo sapiens
<220>
   <223> CAMS
<400> 46
   atggactcgg tggagaaggg ggccgccacc tccgtctcca acccgcgggg gcgaccgtcc      60
   cggggccggc cgccgaagct gcagcgcaac tctcgcggcg gccagggccg aggtgtggag     120
   aagcccccgc acctggcagc cctaattctg gcccggggag gcagcaaagg catccccctg     180
   aagaacatta agcacctggc gggggtcccg ctcattggct gggtcctgcg tgcggccctg     240
   gattcagggg ccttccagag tgtatgggtt tcgacagacc atgatgaaat tgagaatgtg     300
   gccaaacaat ttggtgcaca agttcatcga agaagttctg aagtttcaaa agacagctct     360
   acctcactag atgccatcat agaatttctt aattatcata atgaggttga cattgtagga     420
   aatattcaag ctacttctcc atgtttacat cctactgatc ttcaaaaagt tgcagaaatg     480
   attcgagaag aaggatatga ttctgttttc tctgttgtga gacgccatca gtttcgatgg     540
   agtgaaattc agaaaggagt tcgtgaagtg accgaacctc tgaatttaaa tccagctaaa     600
   cggcctcgtc gacaagactg ggatggagaa ttatatgaaa atggctcatt ttattttgct     660
   aaaagacatt tgatagagat gggttacttg cagggtggaa aaatggcata ctatgaaatg     720
   cgagctgaac atagtgtgga tatagatgtg gatattgatt ggcctattgc agagcaaaga     780
   gtattaagat atggctattt tggcaaagag aagcttaagg aaataaaact tttggtttgc     840
   aatattgatg gatgtctcac caatggccac atttatgtat caggagacca aaaagaaata     900
   atatcttatg atgtaaaaga tgctattggg ataagtttat taaagaaaag tggtattgag     960
   gtgaggctaa tctcagaaag ggcctgttca aagcagacgc tgtcttcttt aaaactggat    1020
   tgcaaaatgg aagtcagtgt atcagacaag ctagcagttg tagatgaatg gagaaaagaa    1080
   atgggcctgt gctggaaaga agtggcatat cttggaaatg aagtgtctga tgaagagtgc    1140
   ttgaagagag tgggcctaag tggcgctcct gctgatgcct gttctactgc ccagaaggct    1200
   gttggataca tttgcaaatg taatggtggc cgtggtgcca tccgagaatt tgcagagcac    1260
   atttgcctac taatggaaaa ggttaataat tcatgccaaa aa                        1302
<210> 47
   <211> 432
   <212> PRT
   <213> Rattus norvegicus
<220>
   <223> CAMS
<400> 47
   Met Asp Ala Leu Glu Lys Gly Ala Ala Thr Ser Gly Pro Ala Pro Arg
   1               5                   10                  15
   Gly Arg Pro Ser Arg Gly Arg Pro Pro Lys Leu Gln Arg Ser Arg Gly
               20                  25                  30
   Ala Gly Arg Gly Leu Glu Lys Pro Pro His Leu Ala Ala Leu Val Leu
           35                  40                  45
   Ala Arg Gly Gly Ser Lys Gly Ile Pro Leu Lys Asn Ile Lys Arg Leu
       50                  55                  60
   Ala Gly Val Pro Leu Ile Gly Trp Val Leu Arg Ala Ala Leu Asp Ala
   65                  70                  75                  80
   Gly Val Phe Gln Ser Val Trp Val Ser Thr Asp His Asp Glu Ile Glu
                   85                  90                  95
   Asn Val Ala Lys Gln Phe Gly Ala Gln Val His Arg Arg Ser Ser Glu
               100                 105                 110
   Thr Ser Lys Asp Ser Ser Thr Ser Leu Asp Ala Ile Val Glu Phe Leu
           115                 120                 125
   Asn Tyr His Asn Glu Val Asp Ile Val Gly Asn Ile Gln Ala Thr Ser
       130                 135                 140
   Pro Cys Leu His Pro Thr Asp Leu Gln Lys Val Ala Glu Met Ile Arg
   145                 150                 155                 160
   Glu Glu Gly Tyr Asp Ser Val Phe Ser Val Val Arg Arg His Gln Phe
                   165                 170                 175
   Arg Trp Ser Glu Ile Gln Lys Gly Val Arg Glu Val Thr Glu Pro Leu
               180                 185                 190
   Asn Leu Asn Pro Ala Lys Arg Pro Arg Arg Gln Asp Trp Asp Gly Glu
           195                 200                 205
   Leu Tyr Glu Asn Gly Ser Phe Tyr Phe Ala Lys Arg His Leu Ile Glu
       210                 215                 220
   Met Gly Tyr Leu Gln Gly Gly Lys Met Ala Tyr Tyr Glu Met Arg Ala
   225                 230                 235                 240
   Glu His Ser Val Asp Ile Asp Val Asp Ile Asp Trp Pro Ile Ala Glu
                   245                 250                 255
   Gln Arg Val Leu Arg Phe Gly Tyr Phe Gly Lys Glu Lys Leu Lys Glu
               260                 265                 270
   Ile Lys Leu Leu Val Cys Asn Ile Asp Gly Cys Leu Thr Asn Gly His
           275                 280                 285
   Ile Tyr Val Ser Gly Asp Gln Lys Glu Ile Ile Ser Tyr Asp Val Lys
       290                 295                 300
   Asp Ala Ile Gly Ile Ser Leu Leu Lys Lys Ser Gly Ile Glu Val Arg
   305                 310                 315                 320
   Leu Ile Ser Glu Arg Ala Cys Ser Lys Gln Thr Leu Ser Ala Leu Lys
                   325                 330                 335
   Leu Asp Cys Lys Thr Glu Val Ser Val Ser Asp Lys Leu Ala Ile Val
               340                 345                 350
   Asp Glu Trp Arg Lys Glu Met Gly Leu Cys Trp Lys Glu Val Ala Tyr
           355                 360                 365
   Leu Gly Asn Glu Val Ser Asp Glu Glu Cys Leu Lys Arg Ala Gly Leu
       370                 375                 380
   Ser Ala Val Pro Ala Asp Ala Cys Ser Arg Ala Gln Lys Ala Val Gly
   385                 390                 395                 400
   Tyr Ile Cys Lys Cys Asn Gly Gly Arg Gly Ala Ile Arg Glu Phe Ala
                   405                 410                 415
   Glu His Ile Phe Leu Leu Leu Glu Lys Val Asn Asn Ser Cys Gln Lys
               420                 425                 430
<210> 48
   <211> 1296
   <212> DNA
   <213> Rattus norvegicus
<220>
   <223> CAMS
<400> 48
   atggacgcgc tggagaaggg ggccgccacg tcggggcccg ccccgcgcgg acggccgtcc 60
   cggggccggc ccccgaagct acagcgcagc cggggcgcgg ggcgaggcct agagaagccg 120
   ccgcacctgg cagcgctggt gctggcccgc ggcggcagca aaggcatccc actgaagaac 180
   atcaagcgcc tggcgggggt tccgctcatt ggctgggtcc tgcgcgcagc cctggacgcg 240
   ggtgtcttcc agagtgtgtg ggtttcaaca gaccatgatg aaattgagaa cgtggccaaa 300
   cagtttggtg cccaggttca ccgaagaagt tctgaaacgt ccaaagacag ctctacctca 360
   ctagatgcca tcgtagaatt cctgaactat cacaatgagg ttgacattgt gggaaatatc 420
   caagctacat ctccatgttt acatcccacg gacctccaga aagttgcaga aatgatccga 480
   gaagaaggat atgactctgt cttctccgtt gtgaggcgcc atcagtttcg atggagtgaa 540
   attcagaaag gagttcgtga agtgaccgag cctctcaacc tgaacccagc taagcggcct 600
   cgtcgacagg actgggatgg agagctgtac gaaaacggct cgttctactt tgctaagaga 660
   cacttgatag agatgggcta cttacagggt gggaaaatgg catattatga aatgcgagct 720
   gagcacagtg tggacataga cgtggacatc gattggccga tcgcagagca aagagttctg 780
   agatttggct attttgggaa agagaagctg aaggagataa agcttttggt gtgtaatatt 840
   gatggatgtc tcaccaatgg tcacatttac gtatcaggag accaaaaaga aataatatct 900
   tacgatgtga aagacgctat tggcataagt ttactaaaga aaagtggcat tgaggtgagg 960
   ctcatctcag aaagggcctg ctccaagcag acgctctccg ccttaaagct ggactgtaaa 1020
   acggaagtca gtgtgtcaga caagctggcc atcgtggatg agtggaggaa ggagatgggc 1080
   ctgtgctgga aagaagtggc ctatctcggc aatgaggtgt ccgatgagga gtgtctgaag 1140
   agagcgggcc tgagcgccgt gcctgctgac gcctgctcca gggcccaaaa ggctgtgggc 1200
   tacatctgca aatgcaacgg gggccgtgga gccatccggg agtttgcaga acacattttc 1260
   ctcctgttag aaaaggttaa taactcatgc caaaaa 1296
<210> 49
   <211> 432
   <212> PRT
   <213> Mus musculus
<220>
   <223> CAMS
<400> 49
   Met Asp Ala Leu Glu Lys Gly Ala Ala Thr Ser Gly Pro Ala Pro Arg
   1               5                   10                  15
   Gly Arg Pro Ser Arg Gly Arg Pro Pro Lys Leu Gln Arg Ser Arg Gly
               20                  25                  30
   Ala Gly Arg Gly Leu Glu Lys Pro Pro His Leu Ala Ala Leu Val Leu
           35                  40                  45
   Ala Arg Gly Gly Ser Lys Gly Ile Pro Leu Lys Asn Ile Lys Arg Leu
       50                  55                  60
   Ala Gly Val Pro Leu Ile Gly Trp Val Leu Arg Ala Ala Leu Asp Ala
   65                  70                  75                  80
   Gly Val Phe Gln Ser Val Trp Val Ser Thr Asp His Asp Glu Ile Glu
                   85                  90                  95
   Asn Val Ala Lys Gln Phe Gly Ala Gln Val His Arg Arg Ser Ser Glu
               100                 105                 110
   Thr Ser Lys Asp Ser Ser Thr Ser Leu Asp Ala Ile Val Glu Phe Leu
           115                 120                 125
   Asn Tyr His Asn Glu Val Asp Ile Val Gly Asn Ile Gln Ala Thr Ser
       130                 135                 140
   Pro Cys Leu His Pro Thr Asp Leu Gln Lys Val Ala Glu Met Ile Arg
   145                 150                 155                 160
   Glu Glu Gly Tyr Asp Ser Val Phe Ser Val Val Arg Arg His Gln Phe
                   165                 170                 175
   Arg Trp Ser Glu Ile Gln Lys Gly Val Arg Glu Val Thr Glu Pro Leu
               180                 185                 190
   Asn Leu Asn Pro Ala Lys Arg Pro Arg Arg Gln Asp Trp Asp Gly Glu
           195                 200                 205
   Leu Tyr Glu Asn Gly Ser Phe Tyr Phe Ala Lys Arg His Leu Ile Glu
       210                 215                 220
   Met Gly Tyr Leu Gln Gly Gly Lys Met Ala Tyr Tyr Glu Met Arg Ala
   225                 230                 235                 240
   Glu His Ser Val Asp Ile Asp Val Asp Ile Asp Trp Pro Ile Ala Glu
                   245                 250                 255
   Gln Arg Val Leu Arg Phe Gly Tyr Phe Gly Lys Glu Lys Leu Lys Glu
               260                 265                 270
   Ile Lys Leu Leu Val Cys Asn Ile Asp Gly Cys Leu Thr Asn Gly His
           275                 280                 285
   Ile Tyr Val Ser Gly Asp Gln Lys Glu Ile Ile Ser Tyr Asp Val Lys
       290                 295                 300
   Asp Ala Ile Gly Ile Ser Leu Leu Lys Lys Ser Gly Ile Glu Val Arg
   305                 310                 315                 320
   Leu Ile Ser Glu Arg Ala Cys Ser Lys Gln Thr Leu Ser Ala Leu Lys
                   325                 330                 335
   Leu Asp Cys Lys Thr Glu Val Ser Val Ser Asp Lys Leu Ala Thr Val
               340                 345                 350
   Asp Glu Trp Arg Lys Glu Met Gly Leu Cys Trp Lys Glu Val Ala Tyr
           355                 360                 365
   Leu Gly Asn Glu Val Ser Asp Glu Glu Cys Leu Lys Arg Val Gly Leu
       370                 375                 380
   Ser Ala Val Pro Ala Asp Ala Cys Ser Gly Ala Gln Lys Ala Val Gly
   385                 390                 395                 400
   Tyr Ile Cys Lys Cys Ser Gly Gly Arg Gly Ala Ile Arg Glu Phe Ala
                   405                 410                 415
   Glu His Ile Phe Leu Leu Ile Glu Lys Val Asn Asn Ser Cys Gln Lys
               420                 425                 430
<210> 50
   <211> 1296
   <212> DNA
   <213> Mus musculus
<220>
   <223> CAMS
<400> 50
   atggacgcgc tggagaaggg ggccgccacg tcggggcccg ccccgcgtgg acggccgtcc      60
   cggggccggc ccccgaagct  gcagcgcagc cggggcgcgg ggcgcggcct agagaagccg     120
   ccgcacctgg cagcgctggt gctggcccgc ggcggcagca aaggcatccc actgaagaac     180
   atcaagcgcc tggcgggggt tccgctcatt ggctgggtcc tgcgcgccgc cctggatgcg     240
   ggggtcttcc agagtgtgtg ggtttcaaca gaccatgatg aaattgagaa tgtggccaaa     300
   cagtttggtg cacaggtcca tcgaagaagt tctgaaacgt ccaaagacag ctctacctca     360
   ctagacgcca ttgtagaatt cctgaattat cacaatgagg ttgacattgt ggggaatatc     420
   caagccacat ctccatgttt acatcccact gacctccaga aagttgcaga aatgatccga     480
   gaagaaggat atgactctgt cttctccgtt gtgaggcgcc atcagtttcg atggagtgaa     540
   attcagaaag gagttcgtga agtgactgag cctctgaact tgaatccagc gaaacggcct     600
   cgtcgacaag actgggatgg agagttatat gagaacggct cattttattt tgctaaaaga     660
   catttgatag agatgggtta cttacagggt gggaaaatgg catattatga aatgcgagct     720
   gagcacagtg tggatatcga cgtggacatc gattggccga tcgcagagca aagagttctg     780
   agatttggct attttggaaa agagaagctg aaggagataa agcttttggt ttgtaatatt     840
   gatggatgtc tcaccaatgg ccacatttat gtatcaggag accaaaaaga aataatatct     900
   tatgatgtaa aagacgctat tggcataagt ttattaaaga aaagcggtat tgaggtgagg     960
   ctcatctcag aacgggcctg ctccaagcag acgctctctg ccctaaagct ggactgtaaa    1020
   acagaagtca gtgtgtccga taagctggcc accgtggatg agtggaggaa ggagatgggc    1080
   ctgtgctgga aagaagtggc ctatctcggc aatgaagtgt ctgatgaaga atgcctcaag    1140
   agagtgggcc tgagcgctgt tcctgccgac gcctgctccg gggcccagaa  ggctgtgggg    1200
   tacatctgca aatgcagcgg tggccgggga gccatccgcg agtttgcaga gcacattttc    1260
   ctactgatag aaaaagttaa taactcatgc caaaaa                              1296
<210> 51
   <211> 406
   <212> PRT
   <213> Homo sapiens
<220>
   <223> ST6GAL1
<400> 51
   Met Ile His Thr Asn Leu Lys Lys Lys Phe Ser Cys Cys Val Leu Val
   1               5                   10                  15
   Phe Leu Leu Phe Ala Val Ile Cys Val Trp Lys Glu Lys Lys Lys Gly
               20                  25                  30
   Ser Tyr Tyr Asp Ser Phe Lys Leu Gln Thr Lys Glu Phe Gln Val Leu
           35                  40                  45
   Lys Ser Leu Gly Lys Leu Ala Met Gly Ser Asp Ser Gln Ser Val Ser
       50                  55                  60
   Ser Ser Ser Thr Gln Asp Pro His Arg Gly Arg Gln Thr Leu Gly Ser
   65                  70                  75                  80
   Leu Arg Gly Leu Ala Lys Ala Lys Pro Glu Ala Ser Phe Gln Val Trp
                   85                  90                  95
   Asn Lys Asp Ser Ser Ser Lys Asn Leu Ile Pro Arg Leu Gln Lys Ile
               100                 105                 110
   Trp Lys Asn Tyr Leu Ser Met Asn Lys Tyr Lys Val Ser Tyr Lys Gly
           115                 120                 125
   Pro Gly Pro Gly Ile Lys Phe Ser Ala Glu Ala Leu Arg Cys His Leu
       130                 135                 140
   Arg Asp His Val Asn Val Ser Met Val Glu Val Thr Asp Phe Pro Phe
   145                 150                 155                 160
   Asn Thr Ser Glu Trp Glu Gly Tyr Leu Pro Lys Glu Ser Ile Arg Thr
                   165                 170                 175
   Lys Ala Gly Pro Trp Gly Arg Cys Ala Val Val Ser Ser Ala Gly Ser
               180                 185                 190
   Leu Lys Ser Ser Gln Leu Gly Arg Glu Ile Asp Asp His Asp Ala Val
           195                 200                 205
   Leu Arg Phe Asn Gly Ala Pro Thr Ala Asn Phe Gln Gln Asp Val Gly
       210                 215                 220
   Thr Lys Thr Thr Ile Arg Leu Met Asn Ser Gln Leu Val Thr Thr Glu
   225                 230                 235                 240
   Lys Arg Phe Leu Lys Asp Ser Leu Tyr Asn Glu Gly Ile Leu Ile Val
                   245                 250                 255
   Trp Asp Pro Ser Val Tyr His Ser Asp Ile Pro Lys Trp Tyr Gln Asn
               260                 265                 270
   Pro Asp Tyr Asn Phe Phe Asn Asn Tyr Lys Thr Tyr Arg Lys Leu His
           275                 280                 285
   Pro Asn Gln Pro Phe Tyr Ile Leu Lys Pro Gln Met Pro Trp Glu Leu
       290                 295                 300
   Trp Asp Ile Leu Gln Glu Ile Ser Pro Glu Glu Ile Gln Pro Asn Pro
   305                 310                 315                 320
   Pro Ser Ser Gly Met Leu Gly Ile Ile Ile Met Met Thr Leu Cys Asp
                   325                 330                 335
   Gln Val Asp Ile Tyr Glu Phe Leu Pro Ser Lys Arg Lys Thr Asp Val
               340                 345                 350
   Cys Tyr Tyr Tyr Gln Lys Phe Phe Asp Ser Ala Cys Thr Met Gly Ala
           355                 360                 365
   Tyr His Pro Leu Leu Tyr Glu Lys Asn Leu Val Lys His Leu Asn Gln
   370 375 380
   Gly Thr Asp Glu Asp Ile Tyr Leu Leu Gly Lys Ala Thr Leu Pro Gly
   385 390 395 400
   Phe Arg Thr Ile His Cys
   405
<210> 52
   <211> 1218
   <212> DNA
   <213> Homo sapiens
<220>
   <223> ST6GAL1
<400> 52
   atgattcaca ccaacctgaa gaaaaagttc agctgctgcg tcctggtctt tcttctgttt 60
   gcagtcatct gtgtgtggaa ggaaaagaag aaagggagtt actatgattc ctttaaattg 120
   caaaccaagg aattccaggt gttaaagagt ctggggaaat tggccatggg gtctgattcc 180
   cagtctgtat cctcaagcag cacccaggac ccccacaggg gccgccagac cctcggcagt 240
   ctcagaggcc tagccaaggc caaaccagag gcctccttcc aggtgtggaa caaggacagc 300
   tcttccaaaa accttatccc taggctgcaa aagatctgga agaattacct aagcatgaac 360
   aagtacaaag tgtcctacaa ggggccagga ccaggcatca agttcagtgc agaggccctg 420
   cgctgccacc tccgggacca tgtgaatgta tccatggtag aggtcacaga ttttcccttc 480
   aatacctctg aatgggaggg ttatctgccc aaggagagca ttaggaccaa ggctgggcct 540
   tggggcaggt gtgctgttgt gtcgtcagcg ggatctctga agtcctccca actaggcaga 600
   gaaatcgatg atcatgacgc agtcctgagg tttaatgggg cacccacagc caacttccaa 660
   caagatgtgg gcacaaaaac taccattcgc ctgatgaact ctcagttggt taccacagag 720
   aagcgcttcc tcaaagacag tttgtacaat gaaggaatcc taattgtatg ggacccatct 780
   gtataccact cagatatccc aaagtggtac cagaatccgg attataattt ctttaacaac 840
   tacaagactt atcgtaagct gcaccccaat cagccctttt acatcctcaa gccccagatg 900
   ccttgggagc tatgggacat tcttcaagaa atctccccag aagagattca gccaaacccc 960
   ccatcctctg ggatgcttgg tatcatcatc atgatgacgc tgtgtgacca ggtggatatt 1020
   tatgagttcc tcccatccaa gcgcaagact gacgtgtgct actactacca gaagttcttc 1080
   gatagtgcct gcacgatggg tgcctaccac ccgctgctct atgagaagaa tttggtgaag 1140
   catctcaacc agggcacaga tgaggacatc tacctgcttg gaaaagccac actgcctggc 1200
   ttccggacca ttcactgc 1218
<210> 53
   <211> 403
   <212> PRT
   <213> Rattus norvegicus
<220>
   <223> ST6GAL1
<400> 53
   Met Ile His Thr Asn Leu Lys Lys Lys Phe Ser Leu Phe Ile Leu Val
   1               5                   10                  15
   Phe Leu Leu Phe Ala Val Ile Cys Val Trp Lys Lys Gly Ser Asp Tyr
               20                  25                  30
   Glu Ala Leu Thr Leu Gln Ala Lys Glu Phe Gln Met Pro Lys Ser Gln
           35                  40                  45
   Glu Lys Val Ala Met Gly Ser Ala Ser Gln Val Val Phe Ser Asn Ser
       50                  55                  60
   Lys Gln Asp Pro Lys Glu Asp Ile Pro Ile Leu Ser Tyr His Arg Val
   65                  70                  75                  80
   Thr Ala Lys Val Lys Pro Gln Pro Ser Phe Gln Val Trp Asp Lys Asp
                   85                  90                  95
   Ser Thr Tyr Ser Lys Leu Asn Pro Arg Leu Leu Lys Ile Trp Arg Asn
               100                 105                 110
   Tyr Leu Asn Met Asn Lys Tyr Lys Val Ser Tyr Lys Gly Pro Gly Pro
           115                 120                 125
   Gly Val Lys Phe Ser Val Glu Ala Leu Arg Cys His Leu Arg Asp His
       130                 135                 140
   Val Asn Val Ser Met Ile Glu Ala Thr Asp Phe Pro Phe Asn Thr Thr
   145                 150                 155                 160
   Glu Trp Glu Gly Tyr Leu Pro Lys Glu Asn Phe Arg Thr Lys Val Gly
                   165                 170                 175
   Pro Trp Gln Arg Cys Ala Val Val Ser Ser Ala Gly Ser Leu Lys Asn
               180                 185                 190
   Ser Gln Leu Gly Arg Glu Ile Asp Asn His Asp Ala Val Leu Arg Phe
           195                 200                 205
   Asn Gly Ala Pro Thr Asp Asn Phe Gln Gln Asp Val Gly Ser Lys Thr
       210                 215                 220
   Thr Ile Arg Leu Met Asn Ser Gln Leu Val Thr Thr Glu Lys Arg Phe
   225                 230                 235                 240
   Leu Lys Asp Ser Leu Tyr Thr Glu Gly Ile Leu Ile Val Trp Asp Pro
                   245                 250                 255
   Ser Val Tyr His Ala Asp Ile Pro Lys Trp Tyr Gln Lys Pro Asp Tyr
               260                 265                 270
   Asn Phe Phe Glu Thr Tyr Lys Ser Tyr Arg Arg Leu Asn Pro Ser Gln
           275                 280                 285
   Pro Phe Tyr Ile Leu Lys Pro Gln Met Pro Trp Glu Leu Trp Asp Ile
       290                 295                 300
   Ile Gln Glu Ile Ser Ala Asp Leu Ile Gln Pro Asn Pro Pro Ser Ser
   305                 310                 315                 320
   Gly Met Leu Gly Ile Ile Ile Met Met Thr Leu Cys Asp Gln Val Asp
                   325                 330                 335
   Ile Tyr Glu Phe Leu Pro Ser Lys Arg Lys Thr Asp Val Cys Tyr Tyr
               340                 345                 350
   His Gln Lys Phe Phe Asp Ser Ala Cys Thr Met Gly Ala Tyr His Pro
           355                 360                 365
   Leu Leu Phe Glu Lys Asn Met Val Lys His Leu Asn Glu Gly Thr Asp
       370                 375                 380
   Glu Asp Ile Tyr Leu Phe Gly Lys Ala Thr Leu Ser Gly Phe Arg Asn
   385                 390                 395                 400
   Ile Arg Cys
<210> 54
   <211> 1209
   <212> DNA
   <213> Rattus norvegicus
<220>
   <223> ST6GAL1
<400> 54
   atgattcata ccaacttgaa gaaaaagttc agcctcttca tcctggtctt tctcctgttc      60
   gcagtcatct gtgtttggaa gaaagggagc gactatgagg cccttacact gcaagccaag     120
   gaattccaga tgcccaagag ccaggagaaa gtggccatgg ggtctgcttc ccaggttgtg     180
   ttctcaaaca gcaagcaaga ccctaaggaa gacattccaa tcctcagtta ccacagggtc     240
   acagccaagg tcaaaccaca gccttccttc caggtgtggg acaaggactc cacatactca     300
   aaacttaacc ccaggctgct gaagatctgg agaaactatc tgaacatgaa caaatataaa     360
   gtatcctaca agggaccggg gccaggagtc aagttcagcg tagaagcact gcgttgccac     420
   cttcgagacc atgtgaacgt gtctatgata gaggccacag attttccctt caacaccact     480
   gagtgggagg gttacctgcc caaggagaac tttagaacca aggttgggcc ttggcaaagg     540
   tgtgccgtcg tctcttctgc aggatctctg aaaaactccc agcttggtcg agagattgat     600
   aatcatgatg cagttctgag gtttaatggg gcccctaccg acaacttcca acaggatgtg     660
   ggctcaaaaa ctaccattcg cctaatgaac tctcagttag tcaccacaga aaagcgcttc     720
   ctcaaggaca gtttgtacac cgaaggaatc ctaattgtat gggacccatc cgtgtatcat     780
   gcagatatcc caaagtggta tcagaaacca gactacaatt tcttcgaaac ctataagagt     840
   taccgaaggc tgaaccccag ccagccattt tatatcctca agccccagat gccatgggaa     900
   ctgtgggaca tcattcagga aatctctgca gatctgattc agccaaatcc cccatcctcc     960
   ggcatgctgg gtatcatcat catgatgacg ctgtgtgacc aggtagatat ttacgagttc    1020
   ctcccatcca agcgcaagac ggacgtgtgc tattatcacc aaaagttctt tgacagcgct    1080
   tgcacgatgg gtgcctacca cccgctcctc ttcgagaaga atatggtgaa gcatctcaat    1140
   gagggaacag atgaagacat ttatttgttt gggaaagcca ccctttctgg cttccggaac    1200
   attcgttgt                                                            1209
<210> 55
   <211> 403
   <212> PRT
   <213> Mus musculus
<220>
   <223> ST6GAL1
<400> 55
   Met Ile His Thr Asn Leu Lys Arg Lys Phe Ser Cys Phe Val Leu Val
   1               5                   10                  15
   Phe Leu Leu Phe Ala Ile Ile Cys Val Trp Lys Lys Gly Ser Asp Tyr
               20                  25                  30
   Glu Ala Leu Thr Leu Gln Ala Lys Val Phe Gln Met Pro Lys Ser Gln
           35                  40                  45
   Glu Lys Val Ala Val Gly Pro Ala Pro Gln Ala Val Phe Ser Asn Ser
       50                  55                  60
   Lys Gln Asp Pro Lys Glu Gly Val Gln Ile Leu Ser Tyr Pro Arg Val
   65                  70                  75                  80
   Thr Ala Lys Val Lys Pro Gln Pro Ser Leu Gln Val Trp Asp Lys Asp
                   85                  90                  95
   Ser Thr Tyr Ser Lys Leu Asn Pro Arg Leu Leu Lys Ile Trp Arg Asn
               100                 105                 110
   Tyr Leu Asn Met Asn Lys Tyr Lys Val Ser Tyr Lys Gly Pro Gly Pro
           115                 120                 125
   Gly Val Lys Phe Ser Val Glu Ala Leu Arg Cys His Leu Arg Asp His
       130                 135                 140
   Val Asn Val Ser Met Ile Glu Ala Thr Asp Phe Pro Phe Asn Thr Thr
   145                 150                 155                 160
   Glu Trp Glu Gly Tyr Leu Pro Lys Glu Asn Phe Arg Thr Lys Ala Gly
                   165                 170                 175
   Pro Trp His Lys Cys Ala Val Val Ser Ser Ala Gly Ser Leu Lys Asn
               180                 185                 190
   Ser Gln Leu Gly Arg Glu Ile Asp Asn His Asp Ala Val Leu Arg Phe
           195                 200                 205
   Asn Gly Ala Pro Thr Asp Asn Phe Gln Gln Asp Val Gly Thr Lys Thr
       210                 215                 220
   Thr Ile Arg Leu Val Asn Ser Gln Leu Val Thr Thr Glu Lys Arg Phe
   225                 230                 235                 240
   Leu Lys Asp Ser Leu Tyr Thr Glu Gly Ile Leu Ile Leu Trp Asp Pro
                   245                 250                 255
   Ser Val Tyr His Ala Asp Ile Pro Gln Trp Tyr Gln Lys Pro Asp Tyr
               260                 265                 270
   Asn Phe Phe Glu Thr Tyr Lys Ser Tyr Arg Arg Leu His Pro Ser Gln
           275                 280                 285
   Pro Phe Tyr Ile Leu Lys Pro Gln Met Pro Trp Glu Leu Trp Asp Ile
       290                 295                 300
   Ile Gln Glu Ile Ser Pro Asp Leu Ile Gln Pro Asn Pro Pro Ser Ser
   305                 310                 315                 320
   Gly Met Leu Gly Ile Ile Ile Met Met Thr Leu Cys Asp Gln Val Asp
                   325                 330                 335
   Ile Tyr Glu Phe Leu Pro Ser Lys Arg Lys Thr Asp Val Cys Tyr Tyr
               340                 345                 350
   His Gln Lys Phe Phe Asp Ser Ala Cys Thr Met Gly Ala Tyr His Pro
           355                 360                 365
   Leu Leu Phe Glu Lys Asn Met Val Lys His Leu Asn Glu Gly Thr Asp
       370                 375                 380
   Glu Asp Ile Tyr Leu Phe Gly Lys Ala Thr Leu Ser Gly Phe Arg Asn
   385                 390                 395                 400
   Asn Arg Cys
<210> 56
   <211> 1209
   <212> DNA
   <213> Mus musculus
<220>
   <223> ST6GAL1
<400> 56
   atgattcata ccaacttgaa gagaaagttc agctgctttg tcctggtctt tctcctgttt      60
   gccatcatct gcgtgtggaa gaaagggagc gactatgagg ctcttacatt gcaagccaag     120
   gtattccaga tgccgaagag ccaggagaaa gtggccgtgg ggcctgctcc ccaggctgtg     180
   ttctcaaaca gcaaacaaga ccctaaggaa ggcgttcaga tcctcagtta ccccagggtc     240
   acagccaagg tcaagccaca gccctccttg caggtgtggg acaaggactc cacatactca     300
   aaacttaacc ccaggctgct gaagatctgg aggaactatc tgaacatgaa taaatataaa     360
   gtgtcctaca aggggccggg accaggagtc aagttcagcg tagaggcgct gcgctgccac     420
   cttcgagacc acgtgaatgt gtctatgata gaggccacag attttccctt caacaccact     480
   gaatgggagg gttacctgcc caaggagaac ttcagaacca aggctgggcc ttggcataag     540
   tgtgccgtcg tgtcttctgc aggatctctg aagaactccc agctgggtcg agagattgat     600
   aatcatgatg cggtcctgag gtttaatggg gcacctacag acaacttcca acaggatgtg     660
   ggcacaaaaa ctaccatccg cctagtgaac tctcagttag tcaccacaga aaagcgcttc     720
   ctgaaggaca gtttgtacac cgaaggaatc ctgattctgt gggacccatc tgtgtatcat     780
   gcagacattc cgcagtggta tcagaagcca gactacaact tcttcgaaac ctataagagt     840
   taccgaaggc ttcaccccag ccagcctttt tacatcctca agccccagat gccatgggaa     900
   ctatgggaca tcattcagga aatctctcca gatctgattc agccgaatcc cccatcctcc     960
   ggcatgctgg gtatcatcat tatgatgacg ctgtgtgacc aagttgatat ttacgagttc    1020
   ctcccatcca agcgcaagac agatgtgtgc tactatcacc agaagttctt tgacagcgcc    1080
   tgcacgatgg gtgcctacca tccgctcctc ttcgagaaga atatggtgaa gcatctcaat    1140
   gagggaacag atgaagacat ttatttgttt gggaaagcta ccctgtctgg cttccggaac    1200
   aatcgctgt                                                            1209
<210> 57
   <211> 337
   <212> PRT
   <213> Homo sapiens
<220>
   <223> SLC35A1
<400> 57
   Met Ala Ala Pro Arg Asp Asn Val Thr Leu Leu Phe Lys Leu Tyr Cys
   1               5                   10                  15
   Leu Ala Val Met Thr Leu Met Ala Ala Val Tyr Thr Ile Ala Leu Arg
               20                  25                  30
   Tyr Thr Arg Thr Ser Asp Lys Glu Leu Tyr Phe Ser Thr Thr Ala Val
           35                  40                  45
   Cys Ile Thr Glu Val Ile Lys Leu Leu Leu Ser Val Gly Ile Leu Ala
       50                  55                  60
   Lys Glu Thr Gly Ser Leu Gly Arg Phe Lys Ala Ser Leu Arg Glu Asn
   65                  70                  75                  80
   Val Leu Gly Ser Pro Lys Glu Leu Leu Lys Leu Ser Val Pro Ser Leu
                   85                  90                  95
   Val Tyr Ala Val Gln Asn Asn Met Ala Phe Leu Ala Leu Ser Asn Leu
               100                 105                 110
   Asp Ala Ala Val Tyr Gln Val Thr Tyr Gln Leu Lys Ile Pro Cys Thr
           115                 120                 125
   Ala Leu Cys Thr Val Leu Met Leu Asn Arg Thr Leu Ser Lys Leu Gln
   130 135 140
   Trp Val Ser Val Phe Met Leu Cys Ala Gly Val Thr Leu Val Gln Trp
   145 150 155 160
   Lys Pro Ala Gln Ala Thr Lys Val Val Val Glu Gln Asn Pro Leu Leu
   165 170 175
   Gly Phe Gly Ala Ile Ala Ile Ala Val Leu Cys Ser Gly Phe Ala Gly
   180 185 190
   Val Tyr Phe Glu Lys Val Leu Lys Ser Ser Asp Thr Ser Leu Trp Val
   195 200 205
   Arg Asn Ile Gln Met Tyr Leu Ser Gly Ile Ile Val Thr Leu Ala Gly
   210 215 220
   Val Tyr Leu Ser Asp Gly Ala Glu Ile Lys Glu Lys Gly Phe Phe Tyr
   225 230 235 240
   Gly Tyr Thr Tyr Tyr Val Trp Phe Val Ile Phe Leu Ala Ser Val Gly
   245 250 255
   Gly Leu Tyr Thr Ser Val Val Val Lys Tyr Thr Asp Asn Ile Met Lys
   260 265 270
   Gly Phe Ser Ala Ala Ala Ala Ile Val Leu Ser Thr Ile Ala Ser Val
   275 280 285
   Met Leu Phe Gly Leu Gln Ile Thr Leu Thr Phe Ala Leu Gly Thr Leu
   290 295 300
   Leu Val Cys Val Ser Ile Tyr Leu Tyr Gly Leu Pro Arg Gln Asp Thr
   305 310 315 320
   Thr Ser Ile Gln Gln Gly Glu Thr Ala Ser Lys Glu Arg Val Ile Gly
   325 330 335
   Val
<210> 58
   <211> 1011
   <212> DNA
   <213> Homo sapiens
<220>
   <223> SLC35A1
<400> 58
   atggctgccc cgagagacaa tgtcacttta ttattcaagt tatactgctt ggcagtgatg      60
   accctgatgg ctgcagtcta taccatagct ttaagataca caaggacatc agacaaagaa     120
   ctctactttt caacgacagc cgtgtgtatc acagaagtta taaagttatt gctaagtgtg     180
   ggaattttag ctaaagaaac tggtagtctg ggtagattca aagcatcttt aagagaaaat     240
   gtcttgggga gccccaagga actgttgaag ttaagtgtgc catcgttagt gtatgctgtt     300
   cagaacaaca tggctttcct agctcttagc aatctggatg cagcagtgta ccaggtgacc     360
   taccagttga agattccgtg tactgcttta tgcactgttt taatgttaaa tcggacactc     420
   agcaaattac agtgggtttc agtttttatg ctgtgtgctg gagttacgct tgtacagtgg     480
   aaaccagccc aagctacaaa agtggtggtg gaacaaaatc cattattagg gtttggcgct     540
   atagctattg ctgtattgtg ctcaggattt gcaggagtat attttgaaaa agttttaaag     600
   agttcagata cttctctttg ggtgagaaac attcaaatgt atctatcagg gattattgtg     660
   acattagctg gcgtctactt gtcagatgga gctgaaatta aagaaaaagg atttttctat     720
   ggttacacat attatgtctg gtttgtcatc tttcttgcaa gtgttggtgg cctctacact     780
   tctgttgtgg ttaagtacac agacaacatc atgaaaggct tttctgcagc agcggccatt     840
   gtcctttcca ccattgcttc agtaatgctg tttggattac agataacact cacctttgcc     900
   ctgggtactc ttcttgtatg tgtttccata tatctctatg gattacccag acaagacact     960
   acatccatcc aacaaggaga aacagcttca aaggagagag ttattggtgt g             1011
<210> 59
   <211> 317
   <212> PRT
   <213> Rattus norvegicus
<220>
   <223> SLC35A1
<400> 59
   Met Thr Leu Val Ala Ala Ala Tyr Thr Ile Ala Leu Arg Tyr Thr Arg
   1               5                   10                  15
   Thr Thr Ala Glu Gly Leu Tyr Phe Ser Thr Thr Ala Val Cys Ile Thr
               20                  25                  30
   Glu Val Ile Lys Leu Leu Ile Ser Val Gly Leu Leu Ala Lys Glu Thr
           35                  40                  45
   Gly Ser Leu Gly Arg Phe Lys Ala Ser Leu Ser Glu Asn Val Leu Gly
       50                  55                  60
   Ser Pro Lys Glu Leu Leu Lys Leu Ser Val Pro Ser Leu Val Tyr Ala
   65 70 75 80
   Val Gln Asn Asn Met Ala Phe Leu Ala Leu Ser Asn Leu Asp Ala Ala
   85 90 95
   Val Tyr Gln Val Thr Tyr Gln Leu Lys Ile Pro Cys Thr Ala Leu Cys
   100 105 110
   Thr Val Leu Met Leu Asn Arg Ser Leu Ser Lys Leu Gln Trp Ile Ser
   115 120 125
   Val Phe Met Leu Cys Gly Gly Val Thr Leu Val Gln Trp Lys Pro Ala
   130 135 140
   Gln Ala Thr Lys Val Val Val Ala Gln Asn Pro Leu Leu Gly Phe Gly
   145 150 155 160
   Ala Ile Ala Ile Ala Val Leu Cys Ser Gly Phe Ala Gly Val Tyr Phe
   165 170 175
   Glu Lys Val Leu Lys Ser Ser Asp Thr Ser Leu Trp Val Arg Asn Ile
   180 185 190
   Gln Met Tyr Leu Ser Gly Ile Ala Val Thr Leu Ala Gly Thr Tyr Leu
   195 200 205
   Ser Asp Gly Ala Glu Ile Lys Glu Lys Gly Phe Phe Tyr Gly Tyr Thr
   210 215 220
   Tyr Tyr Val Trp Phe Val Ile Phe Leu Ala Ser Val Gly Gly Leu Tyr
   225 230 235 240
   Thr Ser Val Val Val Lys Tyr Thr Asp Asn Ile Met Lys Gly Phe Ser
   245 250 255
   Ala Ala Ala Ala Ile Val Leu Ser Thr Val Ala Ser Val Ile Leu Phe
   260 265 270
   Gly Leu Gln Ile Thr Leu Ser Phe Thr Leu Gly Ala Leu Leu Val Cys
   275 280 285
   Val Ser Ile Tyr Leu Tyr Gly Leu Pro Arg Gln Asp Thr Thr Ser Ile
   290 295 300
   Gln Gln Glu Thr Thr Ser Lys Glu Arg Ile Ile Gly Val
   305 310 315
<210> 60
   <211> 951
   <212> DNA
   <213> Rattus norvegicus
<220>
   <223> SLC35A1
<400> 60
   atgactctgg tggctgccgc ttataccata gctttaagat atacaaggac aacagcggaa      60
   ggactctact tttcaaccac agccgtgtgc atcacagaag ttataaagtt actgataagt     120
   gtcggccttc tagctaaaga aacaggcagt ttgggtagat ttaaagcctc tttgagtgaa     180
   aacgtcttgg ggagccctaa ggagctgctg aagttaagtg tgccgtcact ggtgtatgct     240
   gtgcagaaca acatggcttt cctagctctc agtaacctgg atgcagcagt gtaccaggtg     300
   acctatcaac tgaagattcc ctgcactgct ttatgtactg ttttaatgtt aaatcgatca     360
   ctcagcaaac tacagtggat ttcggtcttc atgctgtgtg gtggggtcac acttgtacag     420
   tggaaaccag cccaagctac aaaagtcgtg gtagcgcaga acccgttgtt aggctttgga     480
   gctatagcca ttgctgtgct gtgctcggga tttgcaggag tttattttga aaaagtttta     540
   aagagttcag acacttccct ttgggtgaga aacattcaga tgtatctgtc agggatcgct     600
   gtgacattag ctggtaccta cttgtcggat ggcgctgaaa ttaaagaaaa aggatttttc     660
   tatggctaca cgtattatgt ctggtttgtt atcttccttg ctagtgtggg aggcctctac     720
   acgtcagtgg tggtgaagta cacagacaac atcatgaaag gcttctctgc ggccgcagcc     780
   attgttctgt ctaccgttgc ctcagtcata ctgtttggat tgcagataac actttcattt     840
   acactgggag ctcttcttgt atgtgtttcc atttatctct atgggttacc cagacaagat     900
   accacatcca ttcaacaaga aacaacttca aaagaaagaa tcattggtgt g              951
<210> 61
   <211> 336
   <212> PRT
   <213> Mus musculus
<220>
   <223> SLC35A1
<400> 61
   Met Ala Pro Ala Arg Glu Asn Val Ser Leu Phe Phe Lys Leu Tyr Cys
   1               5                   10                  15
   Leu Thr Val Met Thr Leu Val Ala Ala Ala Tyr Thr Val Ala Leu Arg
               20                  25                  30
   Tyr Thr Arg Thr Thr Ala Glu Glu Leu Tyr Phe Ser Thr Thr Ala Val
           35                  40                  45
   Cys Ile Thr Glu Val Ile Lys Leu Leu Ile Ser Val Gly Leu Leu Ala
       50                  55                  60
   Lys Glu Thr Gly Ser Leu Gly Arg Phe Lys Ala Ser Leu Ser Glu Asn
   65                  70                  75                  80
   Val Leu Gly Ser Pro Lys Glu Leu Ala Lys Leu Ser Val Pro Ser Leu
                   85                  90                  95
   Val Tyr Ala Val Gln Asn Asn Met Ala Phe Leu Ala Leu Ser Asn Leu
               100                 105                 110
   Asp Ala Ala Val Tyr Gln Val Thr Tyr Gln Leu Lys Ile Pro Cys Thr
           115                 120                 125
   Ala Leu Cys Thr Val Leu Met Leu Asn Arg Thr Leu Ser Lys Leu Gln
       130                 135                 140
   Trp Ile Ser Val Phe Met Leu Cys Gly Gly Val Thr Leu Val Gln Trp
   145                 150                 155                 160
   Lys Pro Ala Gln Ala Thr Lys Val Val Val Ala Gln Asn Pro Leu Leu
                   165                 170                 175
   Gly Phe Gly Ala Ile Ala Ile Ala Val Leu Cys Ser Gly Phe Ala Gly
               180                 185                 190
   Val Tyr Phe Glu Lys Val Leu Lys Ser Ser Asp Thr Ser Leu Trp Val
           195                 200                 205
   Arg Asn Ile Gln Met Tyr Leu Ser Gly Ile Val Val Thr Leu Ala Gly
       210                 215                 220
   Thr Tyr Leu Ser Asp Gly Ala Glu Ile Gln Glu Lys Gly Phe Phe Tyr
   225                 230                 235                 240
   Gly Tyr Thr Tyr Tyr Val Trp Phe Val Ile Phe Leu Ala Ser Val Gly
                   245                 250                 255
   Gly Leu Tyr Thr Ser Val Val Val Lys Tyr Thr Asp Asn Ile Met Lys
               260                 265                 270
   Gly Phe Ser Ala Ala Ala Ala Ile Val Leu Ser Thr Ile Ala Ser Val
           275                 280                 285
   Leu Leu Phe Gly Leu Gln Ile Thr Leu Ser Phe Ala Leu Gly Ala Leu
       290                 295                 300
   Leu Val Cys Val Ser Ile Tyr Leu Tyr Gly Leu Pro Arg Gln Asp Thr
   305                 310                 315                 320
   Thr Ser Ile Gln Gln Glu Ala Thr Ser Lys Glu Arg Ile Ile Gly Val
                   325                 330                 335
<210> 62
   <211> 1008
   <212> DNA
   <213> Mus musculus
<220>
   <223> SLC35A1
<400> 62
   atggctccgg cgagagaaaa tgtcagttta ttcttcaagc tgtactgctt gacggtgatg      60
   actctggtgg ctgccgctta caccgtagct ttaagataca caaggacaac agctgaagaa     120
   ctctacttct caaccactgc cgtgtgtatc acagaagtga taaagttact gataagtgtt     180
   ggcctgttag ctaaggaaac tggcagtttg ggtagattta aagcctcatt aagtgaaaat     240
   gtcttgggga gccccaagga actggcgaag ttgagtgtgc catcactagt gtatgctgtg     300
   cagaacaaca tggccttcct ggctctcagt aatctggatg cagcagtgta ccaggtgacc     360
   tatcaactga agatcccctg cactgcttta tgtactgttt taatgttaaa tcgaacactc     420
   agcaaattac agtggatttc cgtcttcatg ctgtgtggtg gggtcacact cgtacagtgg     480
   aaaccagccc aagctacaaa agtcgtggta gcgcagaatc cattgttagg ctttggtgct     540
   atagctattg ctgtattgtg ctctggattt gcaggagttt attttgaaaa agtcttaaag     600
   agttccgaca cttccctttg ggtgagaaac attcagatgt atctgtcagg gatcgttgtg     660
   acgttagctg gtacctactt gtcagatgga gctgaaattc aagaaaaagg attcttctat     720
   ggctacacgt attatgtctg gtttgttatc ttccttgcta gtgtgggagg cctctacacg     780
   tcagtggtgg tgaagtatac agacaacatc atgaaaggct tctctgctgc cgcagccatt     840
   gttctttcta ccattgcttc agtcctactg tttggattac agataacact ttcatttgca     900
   ctgggagctc ttcttgtgtg tgtttccata tatctctatg ggttacccag acaagatact     960
   acatccattc aacaagaagc aacttcaaaa gagagaatca ttggtgtg                 1008
<210> 63
   <211> 27
   <212> DNA
   <213> Artificial
<220>
   <223> primer
<400> 63
   ggcgcgccct ttgtatccct ttttacg 27
<210> 64
   <211> 25
   <212> DNA
   <213> Artificial
<220>
   <223> primer
<400> 64
   ggatccgatc gcttgatcct tagag 25
<210> 65
   <211> 30
   <212> DNA
   <213> Artificial
<220>
   <223> primer
<400> 65
   gtacgtaagc ttgatcaaac ttcgttttcg 30
<210> 66
   <211> 44
   <212> DNA
   <213> Artificial
<220>
   <223> primer
<400> 66
   gcctagggag acggcagatc gtctcccatg ttggcggtct ttgg 44
<210> 67
   <211> 30
   <212> DNA
   <213> Artificial
<220>
   <223> primer
<400> 67
   gtcatgagag tcaaaacctt tgtgatcttg 30
<210> 68
   <211> 45
   <212> DNA
   <213> Artificial
<220>
   <223> primer
<400> 68
   gcctagggag acggcagatc gtctcctgca tttgtataag cgaca 45
<210> 69
   <211> 13
   <212> DNA
   <213> Artificial
<220>
   <223> primer
<400> 69
   cgcgccgcta gcc 13
<210> 70
   <211> 13
   <212> DNA
   <213> Artificial
<220>
   <223> primer
<400> 70
   tcgaggctag cgg 13
<210> 71
   <211> 35
   <212> DNA
   <213> Artificial
<220>
   <223> primer
<400> 71
   gtcatgaggt ttcgtgagca gttcctgggc ggcag 35
<210> 72
   <211> 40
   <212> DNA
   <213> Artificial
<220>
   <223> primer
<400> 72
   gcctaggtta tctcggtgtc ccgatgtcca ctgtgatttg 40
<210> 73
   <211> 40
   <212> DNA
   <213> Artificial
<220>
   <223> primer
<400> 73
   gcgtctccca tgagcagact gctggggggg acgctggagc 40
<210> 74
   <211> 40
   <212> DNA
   <213> Artificial
<220>
   <223> primer
<400> 74
   gcgtctccct aggtcagcct tgagtgagcc acgacatggg 40
<210> 75
   <211> 44
   <212> DNA
   <213> Artificial
<220>
   <223> primer
<400> 75
   gcgtctccca tgttgcggag gctgctggag agaccctgta catt 44
<210> 76
   <211> 45
   <212> DNA
   <213> Artificial
<220>
   <223> primer
<400> 76
   gcgtctccct aggttagtgt gagccacggg gagctgtgtg gttgg 45
<210> 77
   <211> 35
   <212> DNA
   <213> Artificial
<220>
   <223> primer
<400> 77
   gccatgggct gcaacccacc ttatcacctc tccta 35
<210> 78
   <211> 40
   <212> DNA
   <213> Artificial
<220>
   <223> primer
<400> 78
   gcctaggtta cgcagcagtc cagaaatcca ctgtgatgtt 40
<210> 79
   <211> 27
   <212> DNA
   <213> Artificial
<220>
   <223> primer
<400> 79
   ggctagcgca tgcaagcttg agctcga 27
<210> 80
   <211> 27
   <212> DNA
   <213> Artificial
<220>
   <223> primer
<400> 80
   gactagtaag cttatcgata ccgtcga 27
<210> 81
   <211> 30
   <212> DNA
   <213> Artificial
<220>
   <223> primer
<400> 81
   gcgtctccca tggagaagaa cgggaataac 30
<210> 82
   <211> 30
   <212> DNA
   <213> Artificial
<220>
   <223> primer
<400> 82
   ccgtctccct aggctagtgg atcctgcggg 30
<210> 83
   <211> 30
   <212> DNA
   <213> Artificial
<220>
   <223> primer
<400> 83
   gcgtctccca tgccgctgga gctggagctg 30
<210> 84
   <211> 30
   <212> DNA
   <213> Artificial
<220>
   <223> primer
<400> 84
   ccgtctccct agtcaagact tgattttttt 30
<210> 85
   <211> 30
   <212> DNA
   <213> Artificial
<220>
   <223> primer
<400> 85
   gcgtctccca tggggctgag ccgcgtgcgg 30
<210> 86
   <211> 30
   <212> DNA
   <213> Artificial
<220>
   <223> primer
<400> 86
   cgtctcccta gtcaagtgga catactgact 30
<210> 87
   <211> 30
   <212> DNA
   <213> Artificial
<220>
   <223> primer
<400> 87
   gcgtctccca tggactcggt ggagaagggg 30
<210> 88
   <211> 30
   <212> DNA
   <213> Artificial
<220>
   <223> primer
<400> 88
   ccgtctccct aggctatttt tggcatgaat 30
<210> 89
   <211> 30
   <212> DNA
   <213> Artificial
<220>
   <223> primer
<400> 89
   gcgtctccca tggctgcccc gagagacaat 30
<210> 90
   <211> 30
   <212> DNA
   <213> Artificial
<220>
   <223> primer
<400> 90
   ccgtctccct agtcacacac caataactct 30
<210> 91
   <211> 30
   <212> DNA
   <213> Artificial
<220>
   <223> primer
<400> 91
   gcgtctccca tgattcacac caacctgaag 30
<210> 92
   <211> 30
   <212> DNA
   <213> Artificial
<220>
   <223> primer
<400> 92
   ccgtctccct aggttagcag tgaatggtcc 30
<210> 93
   <211> 120
   <212> DNA
   <213> Artificial
<220>
   <223> synthetic
<400> 93
   gctagcaggc gcgccgggat gtaattacgt ccctcccccg ctagggggca gcagcgagcc 60
   gcccggggct ccgctccggt ccggcgctcc ccccgcatcc ccgagggcgc gcctactagt 120
<210> 94
   <211> 19
   <212> **DNA**
   <213> Artificial
<220>
   <223> primer
<400> 94
   gtctagaact agtgctagc 19
<210> 95
   <211> 23
   <212> DNA
   <213> Artificial
<220>
   <223> primer
<400> 95
   gctagcacta gttctagact gca 23
<210> 96
   <211> 40
   <212> DNA
   <213> Artificial
<220>
   <223> primer
<400> 96
   gcgtctcatg cacacgggag ccctgtggac atctgcacag 40
<210> 97
   <211> 45
   <212> DNA
   <213> Artificial
<220>
   <223> primer
<400> 97
   cgcgtctccc taggttagtg atgatgatgg tgatgcttaa cacaa 45
<210> 98
   <211> 39
   <212> DNA
   <213> Artificial
<220>
   <223> primer
<400> 98
   gcgtctcatg cacaggaccc atatgtaaaa gaagcagaa 39
<210> 99
   <211> 45
   <212> DNA
   <213> Artificial
<220>
   <223> primer
<400> 99
   cgcgtctccc taggttagtg atgatgatgg tgatgctggg atgct 45

## Description

### Technical Field

The present invention relates to a transgenic silk-spinning insect, particularly, a transgenic silkworm, which can attach a mammalian-type sugar chain to a recombinant protein of interest, and an expression vector for producing the transgenic form.

### Background Art

The production of recombinant proteins by use of gene recombination technology is very important for the development of new materials or materials of high value and the production industry of pharmaceuticals, cosmetics, etc. For example, pharmaceutical proteins occupied 45.9% of top 10 items of global pharmaceutical sales in 2010 (Non Patent Literature 1) and will presumably further continue to grow.

The production of recombinant proteins has conventionally employed microbes such as *E. coli* and yeast, and cultured cells such as insect cells and animal cells as hosts. The hosts are selected according to the structures, purposes, etc. of the proteins to be produced. In general, protein production systems using microbes permit highly efficient production at low cost, but are not suitable for large-scale production or the production of recombinant proteins having a complicated structure. On the other hand, protein production systems using cultured cells such as cultured mammalian cells are capable of producing recombinant proteins having a complicated structure. Hence, such protein production systems using cultured cells have heretofore been used typically in the production of biopharmaceuticals. However, the protein production systems using cultured cells cannot avoid huge capital investment and high-cost production using expensive culture media, etc. Continued use of biopharmaceuticals puts a large economic burden on patients. Therefore, it has been desired to develop a protein production system that can produce recombinant proteins having a complicated structure at low cost.

In recent years, silkworms *(Bombyx mori)* have received attention as a novel host for protein production systems that can solve the problems described above. The silkworms belonging to *Lepidoptera* insects have been industrially useful insects responsible for silk production since a long time ago. Their expectation as useful protein production systems has grown in recent years because of breakthrough in the gene recombination technology. Silk glands which produce and secrete silk at the larva stage of the silkworms are known to be able to synthesize a large amount of proteins in a short period. Accordingly, the exploitation of this ability of the silk glands to synthesize proteins enables a protein of interest to be produced in large amounts in the silk glands. In the case of using a silkworm as a protein production system, the gene recombination technology is essential which involves introducing a foreign gene encoding the protein of interest into silkworm cells to prepare transformants, i.e., transgenic silkworms. Fortunately, a technique of stably maintaining a foreign gene within the genome using transposon piggyBac has been established for the silkworms (Non Patent Literature 2). Protein production systems using the silkworms are superior to protein production systems using other hosts in that, for example: the amount of proteins produced can be easily controlled by the number of silkworms reared; even several tens of thousands of silkworms can be reared in reduced space; the period from hatching to the late stage of the fifth instar larvae or pupae is as relatively short as a little less than 1 month; the silkworms can be reared throughout the year using an artificial hatching technique and an artificial diet; and produced proteins are easily recovered as cocoons.

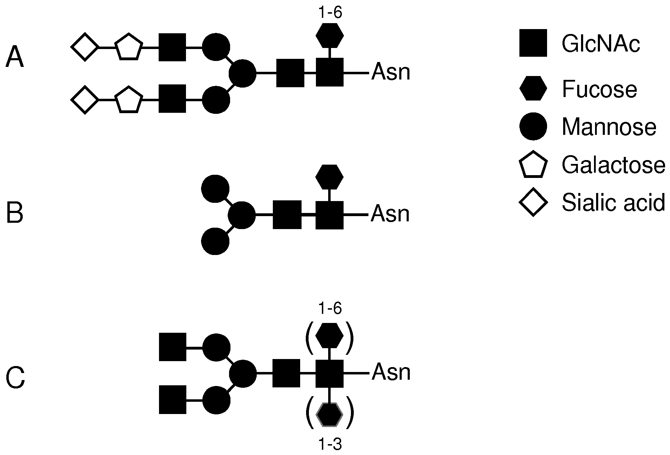

Meanwhile, proteins produced in silkworms differ in the structures of sugar chains to be attached thereto from those produced in mammals including humans. As for N-linked sugar chains attached to proteins via their asparagine residues as shown in Figure 1, proteins produced in silkworm pupae, midgut, fat body, etc. typically have a high mannose-type terminal structure where mannose is attached to a non-reducing terminal (Figure 1B). Silk gland proteins produced in the silk glands of silkworms have a GlcNAc-terminated structure where N-acetylglucosamine (in the present specification, also referred to as "GlcNAc") is further attached to the terminal mannose at the non-reducing terminal (Figure 1C). By contrast, non-reducing terminals of sugar chains typically found in mammals assume a sialic acid-terminated structure where galactose and sialic acid are further attached to the GlcNAc (Figure 1A).

Sugar chains are attached as one of the posttranslational modifications to proteins and are present in 50% or more of *in vivo* proteins. Such sugar chains play an important role in imparting various functions, such as protein stabilization, protection, physiological activity, antigen-antibody reaction, involvement in viral infection and pharmacokinetics, etc., to proteins. However, the difference in sugar chain structure between silkworms and mammals might exhibit immunogenicity and may become responsible for the onset of allergic response. Thus, in the case of producing pharmaceutical proteins with silkworms as a host, the difference in sugar chain structure is associated with the risk of influencing the activity or stability of the pharmaceutical proteins. Hence, a technique of engineering sugar chains of recombinant proteins produced in transgenic silkworms into mammalian-type sugar chains has been desired.

In the research using cultured insect cells, it has been reported that cultured cells which allow mammalian-type sugar chain modification with attached sialic acid at non-reducing terminals of recombinant proteins by introducing a human-type sugar chain modification pathway into the cultured cells (Non Patent Literature 3).

There is also a report stating that sialic acid was attached to sugar chain non-reducing terminals of recombinant proteins produced by baculovirus when an inhibitor of hexosaminidase, which decomposes N-acetylhexosamine in glycoprotein sugar chains, was added to a medium (Non Patent Literature 4).

Patent Literature 1 discloses that galactose was able to be attached to non-reducing terminals of N-linked sugar chains at an individual level of a silkworm by introducing β1,4-galactosyltransferase gene to silkworms. However, any transgenic silkworm provided with a function of attaching sialic acid to the non-reducing terminal of the galactose has not been known.

Non Patent Literature 5 discloses that sugar chains were successfully engineered by expressing human-derived β1,3-N-acetylglucosaminyltransferase 2 in silkworms. However, mammalian-type sialic acid was not attached to the non-reducing terminal.

### Citation List

### Patent Literature

Patent Literature 1: JP Patent Publication (Kokai) No. 2014-012024 A (2014)

### Non Patent Literature

Non Patent Literature 1: 2012 Documents of Kansai Branch, Development Bank of Japan Inc.
Non Patent Literature 2: Tamura T. et al., 2000, Nat Biotechnol, 18: 81-84
Non Patent Literature 3: Jarvis DL et al., Curr Opin Biotechnol. 1998 Oct; 9 (5): 528-533
Non Patent Literature 4: Watanabe et al., J Biol Chem. 2002 Feb 15; 277 (7): 5090-5093
Non Patent Literature 5: Dojima et al., J. Biotechnol. 2009, 143 (1): 27-33

### Summary of Invention

### Technical Problem

Conventional inventions have successfully attached sialic acid to non-reducing terminals of recombinant proteins by the combination of a transgenic silkworm, a baculovirus expression system and oral or transdermal administration of sialic acid. However, there has been a demand for a technique of introducing a sialic acid-attaching function by a more convenient method, i.e., by a transgenic silkworm by itself. Unfortunately, sialic acid-attached human-type sugar chains have an adverse effect on silkworm individuals and inhibit the development of silkworms by usual gene expression systems.

### Solution to Problem

In order to solve the problems described above, the present inventors have developed an expression vector that can induce the expression of a glycosylation-related gene group derived from a mammal such as a human only in a silk gland of a silkworm. A transgenic silkworm harboring the expression vector was able to efficiently attach galactose and sialic acid to a N-linked sugar chain terminal of a recombinant protein of interest produced in a silk gland, without being adversely affected in terms of development. The present inventors have also found that: the introduction of galactosyltransferase gene and three or more genes selected from a group consisting of enzyme genes related to a sialic acid synthesis system suffices for the attachment of a sugar chain with sialic acid at a non-reducing terminal to a protein via its asparagine residue; glycosylation efficiency is significantly increased by introducing CMP-Neu5Ac transporter gene to a silkworm; and regarding an isozyme of β1,4-galactosyltransferase, GalT2 has higher transfer efficiency than that of GalT1 generally used so far. The present invention is based on these results and findings and specifically provides the following aspects (1) to (20).
(1) A mammalian-type glycosylation agent comprising one to three independent expression vector(s) comprising a silk-spinning insect-derived middle and/or posterior silk gland promoter (MSG or PSG promoter) and (a) a gene encoding β1,4-galactosyltransferase (GalT) or a nucleotide encoding an active fragment of the enzyme, functionally linked downstream of the promoter, and (b) genes encoding three or more proteins selected from the group consisting of UDP-acetylglucosamine 2-epimerase/N-acetylmannosamine kinase (GNE), Neu5Ac9-phosphate synthase (NANS), Neu5Ac9-phosphate phosphatase (NANP), CMP-Neu5Ac synthase (CAMS), and α2,6-sialyltransferase (ST6GAL1) or nucleotides encoding active fragments of the proteins, wherein the genes encoding the proteins or the nucleotides encoding active fragments of the proteins are arranged so as to be under direct or indirect expression control of the MSG or PSG promoter.
(2) The mammalian-type glycosylation agent according to (1), wherein the β1,4-galactosyltransferase is GalT2.
(3) The mammalian-type glycosylation agent according to (1) or (2), wherein the middle silk gland promoter is a promoter of sericin 1 gene, sericin 2 gene, or sericin 3 gene.
(4) The mammalian-type glycosylation agent according to (1) or (2), wherein the posterior silk gland promoter is a promoter of fibroin H chain gene, fibroin L chain gene, or p25 gene.
(5) The mammalian-type glycosylation agent according to any of (1) to (4), wherein the three or more proteins selected are three or more proteins comprising GNE, CAMS, and ST6GAL1.
(6) The mammalian-type glycosylation agent according to any of (1) to (5), wherein the expression vector(s) further comprises (c) a gene encoding CMP-Neu5Ac transporter (SLC35A1) or a nucleotide encoding an active fragment of the enzyme.
(7) The mammalian-type glycosylation agent according to any of (1) to (6), wherein the expression vectors consist of a first expression vector comprising the gene or nucleotide described in the (a), and a second expression vector comprising the genes or nucleotides described in the (b).
(8) The mammalian-type glycosylation agent according to (7), wherein the gene or nucleotide (c) is comprised in the second expression vector.
(9) The mammalian-type glycosylation agent according to any of (1) to (6), wherein the genes encoding the proteins or the nucleotides encoding active fragments of the proteins are functionally linked downstream of the MSG or PSG promoter.
(10) The mammalian-type glycosylation agent according to any of (1) to (8), wherein the expression vector(s) is constituted by (i) a first subunit comprising the MSG or PSG promoter, and a gene encoding a transcriptional control element , functionally linked downstream of the promoter, and (ii) one or more second subunit(s) comprising a target promoter of the transcriptional control element , and the genes or nucleotides (a) to (c) functionally linked downstream of the promoter.
(11) The mammalian-type glycosylation agent according to (10), wherein the transcriptional control element is yeast-derived GAL4 protein, and the target promoter thereof is UAS (upstream activating sequence).
(12) The mammalian-type glycosylation agent according to any of (1) to (11), wherein the silk-spinning worm is a silkworm.
(13) The mammalian-type glycosylation agent according to any of (1) to (12), wherein the mammalian type is a human type.
(14) A transgenic silk-spinning insect capable of glycosylation with a mammalian-type sugar chain, comprising an expression vector(s) constituting a mammalian-type glycosylation agent according to any of (1) to (9).
(15) A transgenic silk-spinning insect capable of glycosylation with a mammalian-type sugar chain, comprising an expression vector(s) constituting a mammalian-type glycosylation agent according to (10) or (11).
(16) The transgenic silk-spinning insect capable of glycosylation with a mammalian-type sugar chain according to (15), wherein the first subunit and the second subunit reside on different chromosomes.
(17) The transgenic silk-spinning insect capable of glycosylation with a mammalian-type sugar chain according to any of (14) to (16), wherein the silk-spinning insect is a silkworm.
(18) The transgenic silk-spinning insect capable of glycosylation with a mammalian-type sugar chain according to any of (14) to (17), wherein the mammalian type is a human type.
(19) A line producing a transgenic silk-spinning insect capable of glycosylation with a mammalian-type sugar chain, comprising only a second subunit(s) of an expression vector(s) constituting a mammalian-type glycosylation agent according to (10) or (11).
(20) A method for preparing a transgenic silk-spinning insect capable of glycosylation with a mammalian-type sugar chain, comprising: a mating step of mating a line producing a transgenic silk-spinning insect capable of glycosylation with a mammalian-type sugar chain according to (19), and a line producing transgenic silk-spinning insect of the same species thereas having a first subunit(s) of an expression vector(s) constituting a mammalian-type glycosylation agent according to (10) or (11); and a selection step of selecting a transgenic silk-spinning insect comprising the first subunit and the second subunit as the transgenic silk-spinning insect capable of glycosylation with a mammalian-type sugar chain from a first filial generation (F1).

The present specification encompasses the contents disclosed in Japanese Patent Application No. 2016-21352 on which the priority of the present application is based.

### Advantageous Effects of Invention

According to the mammalian-type glycosylation agent of the present invention, a silk-spinning insect can be easily engineered into a transgenic silk-spinning insect capable of glycosylation with a mammalian-type sugar chain, by introducing the mammalian-type glycosylation agent to the silk-spinning insect, preferably a silkworm.

The transgenic silk-spinning insect capable of glycosylation with a mammalian-type sugar chain according to the present invention can attach a mammalian type N-linked sugar chain to a recombinant protein or peptide of interest produced in a silk gland of the transgenic silk-spinning insect.

The method for producing a transgenic silk-spinning insect capable of glycosylation with a mammalian-type sugar chain according to the present invention can produce the transgenic silk-spinning insect of interest capable of glycosylation with a mammalian-type sugar chain by administering the mammalian-type glycosylation agent of the present invention to a host silk-spinning insect and thereby introducing the expression vector(s) constituting the mammalian-type glycosylation agent to the silk-spinning insect.

### Brief Description of Drawings

[Figure 1] Figure 1 is a conceptual diagram of N-linked sugar chains of a mammal and a silkworm silk gland protein. Figure 1A shows the mammalian N-linked sugar chain structure.
Figure 1B shows the N-linked sugar chain structure of a protein present in a silkworm pupa, midgut, fat body, or the like. Figure 1C shows the N-linked sugar chain structure of a protein present in a silk gland.
[Figure 2] Figure 2 is a conceptual diagram of mammalian type and insect-type N-linked sugar chain synthesis pathways in the Golgi apparatus and a human sialic acid synthesis pathway. In the diagram, the italicized genes encoding seven proteins (β1,4-galactosyltransferase GalT and six sialic acid-related proteins GNE, NANS, NANP, CMAS, ST6GAL1 and SLC35A1) are a gene group forcedly expressed in a silkworm individual in the present invention. The pathway indicated by broken line is a conceptual diagram of a reaction pathway that occurs in the present invention.
[Figure 3] Figure 3 shows results of SDS-PAGE of purified ATIII. Lane 1 shows a middle silk gland (MSG) extract, and lanes 2 to 7 each show an eluate of Ni column purification. The arrow indicates the position of ATIII. A region excised for intra-gel digestion is boxed.
[Figure 4] Figure 4 shows results of SDS-PAGE of purified IFNγ. Lane 1 shows a MSG extract, lane 2 shows a flow-through fraction of Ni column purification, lanes 3 and 4 each show a washing solution, and lanes 5 and 6 each show an eluate. The arrows indicate three detected bands. ^{∗} and ^{∗∗} indicate bands excised for intra-gel digestion.

### Description of Embodiments

### 1. Mammalian-type glycosylation agent

### 1-1. Summary

The first aspect of the present invention is a mammalian-type glycosylation agent. The glycosylation agent of the present invention is constituted by one to three independent expression vector(s). A transgenic silk-spinning insect capable of glycosylation with a mammalian-type sugar chain can be easily prepared by introducing the glycosylation agent of the present invention into a silk-spinning insect such as a silkworm.

### 1-2. Definition

The following terms frequently used in the present specification will be defined.

In the present specification, the "mammalian-type glycosylation agent" refers to an agent that has a configuration mentioned later and is applied to a silk-spinning insect.

In the present specification, the "mammalian-type sugar chain" is a N-linked sugar chain attached to a protein via its asparagine residue and is a sugar chain having a sugar chain structure with sialic acid at a non-reducing terminal where, as shown in Figure 1A, galactose is attached to a GlcNAc non-reducing terminal and sialic acid is further attached to the galactose. In general, the mammalian-type sugar chain has a structure represented by SiaₙGalₙGlcNAcₙManₘ-Asn (Sia represents sialic acid, particularly, N-acetylneuraminic acid, Gal represents galactose, Man represents mannose, each ₘ independently represents an integer of 2 or lager, and each ₙ independently represents an integer of 1 or larger).

In the present invention, the "mammal" is not particularly limited. Preferably, a human, a chimpanzee, a rat, a mouse, a dog, a cat, cattle, a pig, a horse, a goat, sheep or the like corresponds thereto. A human is preferred. That is, a "human-type sugar chain" is preferred.

The "silk" typically refers to animal fiber that is biosynthesized in a silk gland of a silk-spinning insect.

In the present specification, the "silk-spinning insect" refers to a generic name for insects that have silk glands and can spin silk. Specifically, the silk-spinning insect typically refers to a *Lepidoptera* insect, a *Hymenoptera* insect, a *Neuroptera* insect, a *Trichoptera* insect or the like of type that can spin for nesting, cocooning or moving at the larva stage. In this context, the *Lepidoptera* insect is an insect taxonomically belonging to the order *Lepidoptera,* and various butterflies or moths correspond thereto. The *Hymenoptera* insect is an insect taxonomically belonging to the order *Hymenoptera,* and various bees or ants correspond thereto. The *Neuroptera* insect is an insect belonging to the order *Neuroptera,* and dobsonflies, owlflies, ant lions or the like correspond thereto. The *Trichoptera* insect is an insect belonging to the order *Trichoptera,* and various caddice-flies correspond thereto. The silk-spinning insect according to the present invention is preferably a *Lepidoptera* insect, which has large silk glands and can spin a large amount of silk. Among others, a species belonging to the family *Bombycidae, Saturniidae, Brahmaeidae, Eupterotidae, Lasiocampidae, Psychidae, Arctiidae, Noctuidae* or the like is preferred. A species belonging to the genus *Bombyx, Samia, Antheraea, Saturnia, Attacus,* or *Rhodinia,* specifically, a species belonging to a group called wild silkworm such as a silkworm as well as *Bombyx mandarina, Samia cynthia* (including *Samia cynthia ricini* and hybrids of *Samia cynthia* and *Samia cynthia ricini), Antheraea yamamai, Antheraea pernyi, Saturnia japonica,* and *Actias gnoma,* is particularly preferred. The silk-spinning insect is most preferably a silkworm.

The "silk gland" is a fistula that is a modified salivary gland of a silk-spinning insect, and has a function of producing, accumulating, and secreting liquid silk. The silk gland is typically present as a pair of right and left silk glands along the larval digestive tract of the silk-spinning insect. Each silk gland is constituted by 3 regions, anterior, middle and posterior silk glands. In many silk-spinning insects including silkworms, a water-soluble gelatin-like protein sericin serving as a coating component of silk is synthesized in middle silk gland (in the present specification, also referred to as "MSG") cells and secreted into the middle silk gland lumen. Also, three major proteins, fibroin H chain (in the present specification, also referred to as "Fib H"), fibroin L chain (in the present specification, also referred to as "Fib L"), and p25/FHX (hereinafter, referred to as "p25"), which constitute fiber components of silk are synthesized in posterior silk gland (in the present specification, also referred to as "PSG") cells. These three proteins form a SFEU (silk fibroin elementary unit) complex at a ratio of Fib H:Fib L:p25 = 6:6:1, which is then secreted into the posterior silk gland lumen. Then, the SFEU complex is migrated to the middle silk gland lumen, coated with sericin, and spun as silk from the anterior silk gland. Thus, in the case of using a silk-spinning insect as a protein expression system, a gene expression system specifically expressed in the middle or posterior silk gland can be used.

In the present specification, the "expression vector" refers to an expression unit that comprises a recombinant gene encoding a recombinant protein or a nucleotide encoding an active fragment thereof in an expressible state and can control the expression of the recombinant gene, etc. The expression vector of the present invention can employ various expression units capable of replicating in host cells. Examples thereof include plasmid vectors and Bacmid vectors capable of autonomously replicating, and viral vectors. In the present specification, a plasmid vector is typically used. The expression vector constituting the mammalian-type glycosylation agent of the present invention (hereinafter, in the present specification, also referred to as a "glycosylation agent expression vector") may be constituted by two or more independent subunits. In this case, all of the subunits are interpreted as one expression vector. The configuration of each subunit will be mentioned later.

In the present specification, the "recombinant protein of interest" is a protein encoded by the recombinant gene of interest and refers to a recombinant protein to be produced in a silk gland in a protein production system using a silk-spinning insect. In the present specification, the recombinant protein of interest is a recombinant protein glycosylated with a mammalian-type sugar chain in a transgenic silk-spinning insect capable of glycosylation with a mammalian-type sugar chain. The recombinant protein of interest may be derived from one gene or a gene fragment thereof, or may be derived from a chimeric gene containing linked portions of a plurality of genes. The amino acid length of the recombinant protein of interest is not particularly limited. The number of amino acid residues may be 8 to 10,000. The recombinant protein of interest also encompasses, for example, a peptide hormone consisting of only 9 amino acids, such as oxytocin. In the present specification, the type of the recombinant protein of interest is not particularly limited, and a protein of high value is preferred. Examples thereof include: peptide hormones such as insulin, calcitonin, parathormone and growth hormone; cytokines such as epidermal growth factor (EGF), fibroblast growth factor (FGF), interleukin (IL), interferon (IFN), tumor necrosis factor α (TNF-α) and transforming growth factor β (TGF-β); and immunoglobulin, antithrombin III, serum albumin, hemoglobin, various enzymes, and collagen, and their fragments (including chimeric peptides).

In the present specification, the "recombinant gene of interest" refers to a foreign gene, in principle, encoding the recombinant protein of interest described above. In the present specification, the recombinant gene of interest is present in an expression vector such that the recombinant gene of interest is functionally linked to a middle and/or posterior silk gland promoter. This expression vector may be a glycosylation agent expression vector.

### 1-3. Configuration

### 1-3-1. Component

The mammalian-type glycosylation agent of the present invention is constituted by a glycosylation agent expression vector. The glycosylation agent expression vector comprises (1) a silk-spinning insect-derived middle and/or posterior silk gland promoter and (2) glycosylation-related genes or nucleotides encoding active fragments of proteins encoded thereby (in the present specification, also referred to as "glycosylation-related genes, etc.") as essential components. When the glycosylation agent expression vector is constituted by two subunits, a first subunit and a second subunit mentioned later, the glycosylation agent expression vector can contain (3) a gene encoding a transcriptional control element and (4) a target promoter of the transcriptional control element as essential components. In addition, the glycosylation agent expression vector can comprise other components capable of contributing to the expression of the glycosylation-related genes, etc. Examples of such other components include (5) a terminator, (6) a marker gene, (7) an enhancer, (8) an insulator, and (9) an inverted terminal repeat sequence of transposon. Hereinafter, each component will be specifically described.

### (1) Middle or posterior silk gland promoter

In the present specification, the "middle or posterior silk gland promoter (MSG or PSG promoter)" is an essential component of the glycosylation agent expression vector and refers to a site-specific promoter that controls the expression of a gene specifically expressed in a middle or posterior silk gland of a silk-spinning insect.

Examples of the gene specifically expressed in the middle silk gland (MSG) of a silk-spinning insect include sericin 1 (in the present specification, also referred to as "Ser1") gene, sericin 2 (in the present specification, also referred to as "Ser2") gene, and sericin 3 (in the present specification, also referred to as "Ser3") gene. Thus, promoters that control the expression of Ser1 to Ser3 genes (in the present specification, referred to as Ser1 promoter, Ser2 promoter, and Ser3 promoter, respectively) are preferred as the silk-spinning insect-derived MSG promoter of the glycosylation agent expression vector. Specific examples of these promoters include silkworm-derived Ser1 promoter consisting of the nucleotide sequence represented by SEQ ID NO: 1, Ser2 promoter consisting of the nucleotide sequence represented by SEQ ID NO: 2, and Ser3 promoter consisting of the nucleotide sequence represented by SEQ ID NO: 3.

Examples of the gene specifically expressed in the posterior silk gland (PSG) of a silk-spinning insect include Fib H gene, Fib L gene, and p25 gene. Thus, promoters that control the expression of these genes (in the present specification, referred to as Fib H promoter, Fib L promoter, and p25 promoter, respectively) are preferred as the silk-spinning insect-derived PSG promoter of the glycosylation agent expression vector. Specific examples of these promoters include silkworm-derived Fib H promoter consisting of the nucleotide sequence represented by SEQ ID NO: 4, Fib L promoter consisting of the nucleotide sequence represented by SEQ ID NO: 5, and p25 promoter consisting of the nucleotide sequence represented by SEQ ID NO: 6, and tussah-derived Fib H promoter consisting of the nucleotide sequence represented by SEQ ID NO: 7 and Fib L promoter consisting of the nucleotide sequence represented by SEQ ID NO: 8.

The nucleotide sequence of the MSG or PSG promoter is evolutionarily conserved very well among silk-spinning insects. Thus, for example, the PSG promoter is highly probably operable in PSGs of different silk-spinning insect species (Sezutsu H., et al., 2009, Journal of Insect Biotechnology and Sericology, 78: 1-10). Accordingly, the organism species from which the MSG or PSG promoter is derived is not necessarily required to be the same as the organism species of a silk-spinning insect to which the glycosylation agent expression vector is to be introduced. The promoter is preferably derived from a species belonging to the same order thereas, more preferably a species belonging to the same family thereas, further preferably a species belonging to the same genus thereas, most preferably the same species thereas.

The glycosylation agent expression vector may comprise either of the MSG or PSG promoter. As mentioned later, when the glycosylation agent expression vector is constituted by two independent expression vectors, these expression vectors may comprise different silk gland promoters. Alternatively, different silk gland promoters may control the expression of a plurality of glycosylation-related genes mentioned later comprised in one expression vector. Usually, any one of the MSG and PSG promoters suffices. The MSG promoter is preferred.

The MSG or PSG promoter in the glycosylation agent expression vector is configured such that the glycosylation-related genes, etc. or a gene encoding a transcriptional control element mentioned later can be arranged within the scope of a control region downstream thereof (on the 3'-terminal side).

### (2) Glycosylation-related gene, etc.

The glycosylation-related genes or nucleotides encoding active fragments of proteins encoded by the genes (glycosylation-related genes, etc.) are core components in the glycosylation agent expression vector together with the MSG or PSG promoter mentioned above.

In the present specification, the "glycosylation-related genes" refer to genes encoding β1,4-galactosyltransferase and six sialic acid-related proteins related to mammalian-type glycosylation. The "nucleotides encoding active fragments of proteins encoded by the genes" refer to nucleotides encoding the amino acid sequences of peptides that are partial peptides of the proteins encoded by the glycosylation-related genes and have physiological activity equivalent to or higher than the proteins. For example, nucleotides encoding functional domains of the proteins correspond thereto. The length of amino acids in the partial peptides is not particularly limited as long as the partial peptides have activity. The amino acid length can be 40 or more amino acids, 50 or more amino acids, 60 or more amino acids, or 70 or more amino acids and less than the full length. Hereinafter, each glycosylation-related gene will be specifically described.

### A. β1,4-galactosyltransferase

The "β1,4-galactosyltransferase (in the present specification, also referred to as "GalT")" is an enzyme that catalyzes the reaction of transferring galactose from a donor substrate UDP-galactose (UDP-Gal) to GlcNAcβ1-2Man of a glycoprotein. In the mammalian-type glycosylation agent of the present invention, this enzyme has a function of attaching galactose to the GlcNAc non-reducing terminal of a N-linked sugar chain in a silk gland-derived protein of a silk-spinning insect. GalT is known to have a plurality of isozymes. For example, 7 types of isozymes have been identified in mice. Among them, 4 types, GalT1, GalT2, GalT3, and GalT4, are involved in glycoproteins, and 3 types, GalT1, GalT2, and GalT3, have activity as the mammalian-type glycosylation agent of the present invention. Among them, GalT2 is particularly preferred. Thus, the term "GalT" described in the present specification means any of GalT1, GalT2, and GalT3 unless otherwise specified.

The GalT gene is an essential component in the glycosylation agent expression vector. The organism species from which the GalT gene in the glycosylation agent expression vector is derived is not particularly limited. For example, the GalT gene derived from any organism such as an invertebrate (nematode, insect, etc.), a chordate, or a vertebrate (fish, amphibian, reptile, bird, mammal, etc.) can be used. The GalT gene derived from a mammal such as a human, a rat, or a mouse is preferred. Specific examples thereof include human GalT1 gene encoding human GalT1 consisting of the amino acid sequence represented by SEQ ID NO: 9 (e.g., human GalT1 gene consisting of the nucleotide sequence represented by SEQ ID NO: 10), rat GalT1 gene encoding rat GalT1 consisting of the amino acid sequence represented by SEQ ID NO: 11 (e.g., rat GalT1 gene consisting of the nucleotide sequence represented by SEQ ID NO: 12), mouse GalT1 gene encoding mouse GalT1 consisting of the amino acid sequence represented by SEQ ID NO: 13 (e.g., mouse GalT1 gene consisting of the nucleotide sequence represented by SEQ ID NO: 14), human GalT2 gene encoding human GalT2 consisting of the amino acid sequence represented by SEQ ID NO: 15 (e.g., human GalT2 gene consisting of the nucleotide sequence represented by SEQ ID NO: 16), rat GalT2 gene encoding rat GalT2 consisting of the amino acid sequence represented by SEQ ID NO: 17 (e.g., rat GalT1 gene consisting of the nucleotide sequence represented by SEQ ID NO: 18), mouse GalT2 gene encoding mouse GalT2 consisting of the amino acid sequence represented by SEQ ID NO: 19 (e.g., mouse GalT2 gene consisting of the nucleotide sequence represented by SEQ ID NO: 20), human GalT3 gene encoding human GalT3 consisting of the amino acid sequence represented by SEQ ID NO: 21 (e.g., human GalT3 gene consisting of the nucleotide sequence represented by SEQ ID NO: 22), rat GalT3 gene encoding rat GalT3 consisting of the amino acid sequence represented by SEQ ID NO: 23 (e.g., rat GalT3 gene consisting of the nucleotide sequence represented by SEQ ID NO: 24), and mouse GalT3 gene encoding mouse GalT3 consisting of the amino acid sequence represented by SEQ ID NO: 25 (e.g., mouse GalT3 gene consisting of the nucleotide sequence represented by SEQ ID NO: 26).

### B. Six sialic acid-related proteins

The "six sialic acid-related proteins" are a series of proteins necessary for attaching sialic acid to the non-reducing terminal consisting of the galactose at a N-linked sugar chain in silk gland cells of a silk-spinning insect and consist of four α2,6-sialyltransferase substrate synthesis-related enzymes, α2,6-sialyltransferase, and sugar nucleotide transporter.

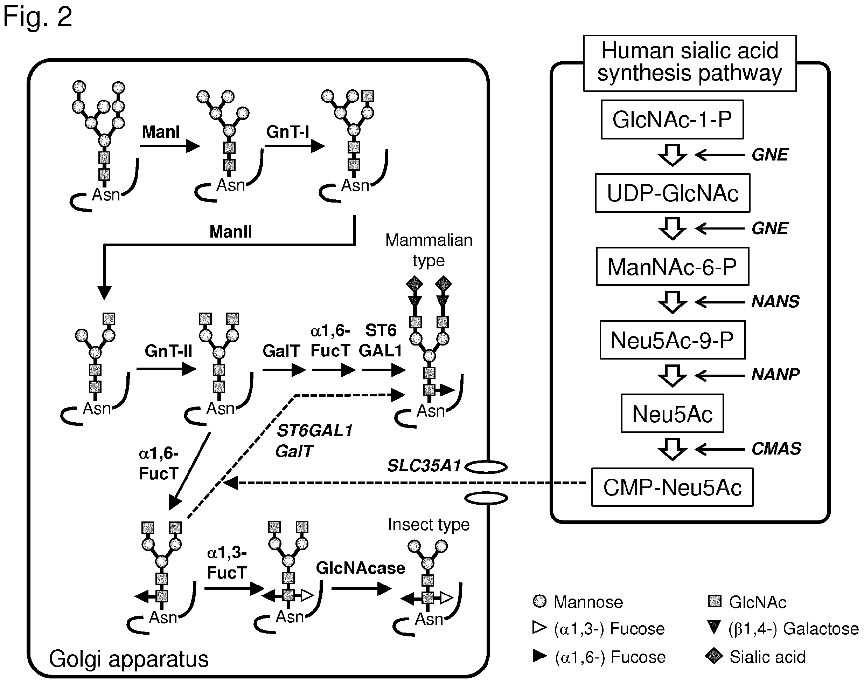

The four α2,6-sialyltransferase substrate synthesis-related enzymes are an enzyme group that functions in the sialic acid synthesis pathway of converting GlcNAc-1-P (N-acetylglucosamine-1-phosphate) to CMP-Neu5Ac (CMP-N-acetylneuraminic acid: CMP-sialic acid), as shown in Figure 2, and consist of UDP-acetylglucosamine 2-epimerase/N-acetylmannosamine kinase, Neu5Ac9-phosphate synthase, Neu5Ac9-phosphate phosphatase, and CMP-Neu5Ac synthase.

In the present specification, the genes encoding the sialic acid-related proteins are also referred to as "sialic acid-related genes". Hereinafter, the six sialic acid-related proteins will be described.

The "UDP-acetylglucosamine 2-epimerase/N-acetylmannosamine kinase (in the present specification, also referred to as "GNE")" has a catalytic effect of converting GlcNAc-1-P to UDP-GlcNAc (uridine diphosphate N-acetylglucosamine) and further the UDP-GlcNAc to ManNac-6-P (N-acetylmannosamine-6-phosphate). In the present specification, the gene encoding GNE is referred to as "GNE gene". The organism species from which the GNE gene in the glycosylation agent expression vector is derived is not particularly limited. The GEN gene derived from any organism such as an invertebrate (nematode, insect, etc.), a chordate, or a vertebrate (fish, amphibian, reptile, bird, mammal, etc.) can be used. The GNE gene derived from a mammal such as a human, a rat, or a mouse is preferred. Specific examples thereof include a gene encoding human GNE consisting of the amino acid sequence represented by SEQ ID NO: 27 (e.g., human GNE gene consisting of the nucleotide sequence represented by SEQ ID NO: 28), rat GNE gene encoding rat GNE consisting of the amino acid sequence represented by SEQ ID NO: 29 (e.g., rat GNE gene consisting of the nucleotide sequence represented by SEQ ID NO: 30), and mouse GNE gene encoding mouse GNE consisting of the amino acid sequence represented by SEQ ID NO: 31 (e.g., mouse GNE gene consisting of the nucleotide sequence represented by SEQ ID NO: 32).

The "Neu5Ac9-phosphate synthase (in the present specification, also referred to as "NANS")" has a catalytic effect of converting ManNac-6-P formed by the catalytic effect of GNE to Neu5Ac-9-P (N-acetylneuraminic acid-9-phosphate). In the present specification, the gene encoding NANS is referred to as "NANS gene". The organism species from which the NANS gene in the glycosylation agent expression vector is derived is not particularly limited. The NANS gene derived from any organism such as an invertebrate (nematode, insect, etc.), a chordate, or a vertebrate (fish, amphibian, reptile, bird, mammal, etc.) can be used. The NANS gene derived from a mammal such as a human, a rat, or a mouse is preferred. Specific examples thereof include human NANS gene encoding human NANS consisting of the amino acid sequence represented by SEQ ID NO: 33 (e.g., human NANS gene consisting of the nucleotide sequence represented by SEQ ID NO: 34), rat NANS gene encoding rat NANS consisting of the amino acid sequence represented by SEQ ID NO: 35 (e.g., rat NANS gene consisting of the nucleotide sequence represented by SEQ ID NO: 36), and mouse NANS gene encoding mouse NANS consisting of the amino acid sequence represented by SEQ ID NO: 37 (e.g., mouse NANS gene consisting of the nucleotide sequence represented by SEQ ID NO: 38).

The "Neu5Ac9-phosphate phosphatase (in the present specification, also referred to as "NANP")" has a catalytic effect of converting Neu5Ac-9-P formed by the catalytic effect of NANS to Neu5Ac (N-acetylneuraminic acid) by removing phosphoric acid. In the present specification, the gene encoding NANP is referred to as "NANP gene". The organism species from which the NANP gene in the glycosylation agent expression vector is derived is not particularly limited. The NANP gene derived from any organism such as an invertebrate (nematode, insect, etc.), a chordate, or a vertebrate (fish, amphibian, reptile, bird, mammal, etc.) can be used. The NANP gene derived from a mammal such as a human, a rat, or a mouse is preferred. Specific examples thereof include human NANP gene encoding human NANP consisting of the amino acid sequence represented by SEQ ID NO: 39 (e.g., human NANP gene consisting of the nucleotide sequence represented by SEQ ID NO: 40), rat NANP gene encoding rat NANP consisting of the amino acid sequence represented by SEQ ID NO: 41 (e.g., rat NANP gene consisting of the nucleotide sequence represented by SEQ ID NO: 42), and mouse NANP gene encoding mouse NANP consisting of the amino acid sequence represented by SEQ ID NO: 43 (e.g., mouse NANP gene consisting of the nucleotide sequence represented by SEQ ID NO: 44).

The "CMP-Neu5Ac synthase (in the present specification, also referred to as "CMAS")" has a catalytic effect of converting Neu5Ac formed by the catalytic effect of NANP to CMP-Neu5Ac. In the present specification, the gene encoding CMAS is referred to as "CMAS gene". The organism species from which the CMAS gene in the glycosylation agent expression vector is derived is not particularly limited. The CMAS gene derived from any organism such as an invertebrate (nematode, insect, etc.), a chordate, or a vertebrate (fish, amphibian, reptile, bird, mammal, etc.) can be used. The CMAS gene derived from a mammal such as a human, a rat, or a mouse is preferred. Specific examples thereof include human CMAS gene encoding human CMAS consisting of the amino acid sequence represented by SEQ ID NO: 45 (e.g., human CMAS gene consisting of the nucleotide sequence represented by SEQ ID NO: 46), rat CMAS gene encoding rat CMAS consisting of the amino acid sequence represented by SEQ ID NO: 47 (e.g., rat CMAS gene consisting of the nucleotide sequence represented by SEQ ID NO: 48), and mouse CMAS gene encoding mouse CMAS consisting of the amino acid sequence represented by SEQ ID NO: 49 (e.g., mouse CMAS gene consisting of the nucleotide sequence represented by SEQ ID NO: 50).

The "α2,6-sialyltransferase (in the present specification, also referred to as "ST6GAL1")" is a glycosyltransferase that catalyzes the reaction of transferring a sialic acid residue from the donor substrate CMP-Neu5Ac synthesized in the sialic acid synthesis pathway and transported into the Golgi apparatus by the action of SLC35A1 to a sugar chain structure in acceptor substrates glycoprotein and glycolipid. In the present specification, the gene encoding ST6GAL1 is referred to as "ST6GAL1 gene". The organism species from which the ST6GAL1 gene in the glycosylation agent expression vector is derived is not particularly limited. The ST6GAL1 gene derived from any organism such as an invertebrate (nematode, insect, etc.), a chordate, or a vertebrate (fish, amphibian, reptile, bird, mammal, etc.) can be used. The ST6GAL1 gene derived from a mammal such as a human, a rat, or a mouse is preferred. Specific examples thereof include human ST6GAL1 gene encoding human ST6GAL1 consisting of the amino acid sequence represented by SEQ ID NO: 51 (e.g., human ST6GAL1 gene consisting of the nucleotide sequence represented by SEQ ID NO: 52), rat ST6GAL1 gene encoding rat ST6GAL1 consisting of the amino acid sequence represented by SEQ ID NO: 53 (e.g., rat ST6GAL1 gene consisting of the nucleotide sequence represented by SEQ ID NO: 54), and mouse ST6GAL1 gene encoding mouse ST6GAL1 consisting of the amino acid sequence represented by SEQ ID NO: 55 (e.g., mouse ST6GAL1 gene consisting of the nucleotide sequence represented by SEQ ID NO: 56).

In the present specification, the "sugar nucleotide transporter" refers to a transporter protein having the ability to transport sialic acid to the Golgi apparatus. For example, CMP-Neu5Ac transporter corresponds thereto.

The "CMP-Neu5Ac transporter (in the present specification, also referred to as "SLC35A1")" is a membrane transport protein present on the membrane of the Golgi apparatus and has an effect of transporting CMP-Neu5Ac synthesized in the sialic acid synthesis pathway into the Golgi apparatus. In the present specification, the gene encoding SLC35A1 is referred to as "SLC35A1 gene". The organism species from which the SLC35A1 gene in the glycosylation agent expression vector is derived is not particularly limited. The SLC35A1 gene derived from any organism such as an invertebrate (nematode, insect, etc.), a chordate, or a vertebrate (fish, amphibian, mammal, etc.) can be used. The SLC35A1 gene derived from a mammal such as a human, a rat, or a mouse is preferred. Specific examples thereof include human SLC35A1 gene encoding human SLC35A1 consisting of the amino acid sequence represented by SEQ ID NO: 57 (e.g., human SLC35A1 gene consisting of the nucleotide sequence represented by SEQ ID NO: 58), rat SLC35A1 gene encoding rat SLC35A1 consisting of the amino acid sequence represented by SEQ ID NO: 59 (e.g., rat SLC35A1 gene consisting of the nucleotide sequence represented by SEQ ID NO: 60), and mouse SLC35A1 gene encoding mouse SLC35A1 consisting of the amino acid sequence represented by SEQ ID NO: 61 (e.g., mouse SLC35A1 gene consisting of the nucleotide sequence represented by SEQ ID NO: 62).

Among the six sialic acid-related genes described above, three or more genes selected from the group consisting of five genes, the GNE gene, the NANS gene, the NANP gene, the CMAS gene and the ST6GAL1 gene, excluding the SLC35A1 gene are comprised as essential components in the glycosylation agent expression vector. Examples of the three genes include a set of the GNE gene, the CMAS gene and the ST6GAL1 gene. Examples of the four genes include a set of the GNE gene, the NANP gene, the CMAS gene and the ST6GAL1 gene. Preferably, all of the five genes are selected. On the other hand, the SLC35A1 gene is an optional component in the glycosylation agent expression vector. It is particularly preferred to comprise all of the six genes because the advantageous effects of the invention are enhanced by the addition of the SLC35A1 gene.

Each gene described above is preferably a wild-type gene and may be a variant gene as long as the activity of the protein encoded by each gene is maintained. Examples thereof include variant genes based on gene polymorphism such as SNPs. Examples of such variant genes include a gene consisting of a nucleotide sequence derived from the nucleotide sequence of the wild-type gene by the deletion, substitution or addition of one to several bases, and a gene consisting of a nucleotide sequence having 70% or higher, 80% or higher, 85% or higher or 90% or higher, preferably 95% or higher, more preferably 96% or higher, 97% or higher, 98% or higher or 99% or higher base identity to the nucleotide sequence of the wild-type gene. In the present specification, the term "several" refers to, for example, 2 to 20, 2 to 15, 2 to 10, 2 to 7, 2 to 5, 2 to 4 or 2 or 3. The "base identity" refers to the ratio (%) of identical bases between two nucleotide sequences to the total number of base residues in the wild-type gene when the two nucleotide sequences are aligned, with a gap introduced, if necessary, to any of the nucleotide sequences to attain the highest base similarity therebetween.

### (3) Gene encoding transcriptional control element

In the present specification, the "gene encoding a transcriptional control element" is an essential component in a first subunit mentioned later and refers to a gene of a transcriptional control element. In the present specification, the "transcriptional control element" refers to a protein factor that can bind to a target promoter mentioned later and thereby activate the target promoter. Examples thereof include GAL4 protein which is a yeast galactose metabolism-activating protein, and tTA which is a tetracycline-controlled transcriptional activator, and variants thereof.

### (4) Target promoter of the transcriptional control element

In the present specification, the "target promoter of the transcriptional control element" is an essential component in a second subunit mentioned later and refers to a promoter that can activate gene expression under its control through the binding of the transcriptional control element encoded in the first subunit thereto. The transcriptional control element and the target promoter thereof are in a correspondence relationship. Usually, if the transcriptional control element is determined, the target promoter thereof is also determined inevitably. For example, when the transcriptional control element is GAL4 protein, UAS (upstream activating sequence) is used.

The target promoter of the transcriptional control element in the glycosylation agent expression vector is configured such that the glycosylation-related genes, etc. mentioned above can be arranged within the scope of a control region downstream thereof.

### (5) Terminator

In the present specification, the "terminator" is an optional component constituted by a nucleotide sequence that can terminate the transcription of a gene, etc. during its expression in the glycosylation agent expression vector of the present aspect.

### (6) Marker gene

In the present specification, the "marker gene" is a gene encoding a marker protein also called selection marker. The marker protein refers to a polypeptide that allows the presence or absence of the expression of the marker gene to be determined on the basis of its activity. Hence, when the glycosylation agent expression vector comprises the marker gene, a transgenic silk-spinning insect harboring the glycosylation agent expression vector can be easily determined on the basis of the activity of the marker protein. In this context, the phrase "on the basis of the activity" means "on the basis of results of detecting the activity". The detection of the activity may directly detect the activity itself of the marker protein or may be indirect detection via a metabolite, such as a dye, formed by the activity of the marker protein. The detection may be any of chemical detection (including detection through enzymatic reaction), physical detection (including detection by behavior analysis), and sensory detection by a person in charge of detection (including visual, tactile, olfactory, auditory, and gustatory detection).

The type of the marker protein is not particularly limited as long as its activity is detectable by a method known in the art. A marker protein that is low invasive to a transgenic silkworm in detection is preferred. Examples thereof include fluorescent proteins, dye-synthesizing proteins, luminescent proteins, externally secreted proteins, and proteins that control external morphology, etc. A fluorescent protein, a dye-synthesizing protein, a luminescent protein, and an externally secreted protein are particularly preferred because these proteins are visually detectable under particular conditions and therefore very low invasive to a transgenic silkworm and permit easy determination and selection.

The fluorescent protein refers to a protein that emits fluorescence having a particular wavelength when a transgenic silkworm is irradiated with excitation light having a particular wavelength. Any of natural and non-natural fluorescent proteins may be used. The excitation wavelength and the fluorescence wavelength are not particularly limited. Specific examples thereof include CFP, AmCyan, RFP, DsRed (including derivatives such as DsRed monomer and DsRed2), YFP, and GFP (including derivatives such as EGFP and EYFP).

The dye-synthesizing protein is a protein involved in the biosynthesis of a dye and is usually an enzyme. In this context, the "dye" is a low-molecular compound or a peptide that can impart the dye to a transformant and is not limited by its type. A dye that appears as an external color of an individual is preferred. Examples thereof include melanin dyes (including dopamine melanin), ommochrome dyes, and pteridine-type dyes.

The luminescent protein refers to a substrate protein that can emit light without the need of excitation light, or an enzyme that catalyzes the luminescence of the substrate protein. Examples thereof include aequorin, and luciferase as an enzyme.

In the present specification, the externally secreted protein is a protein that is secreted to the outside of cells or the outside of the body, and an exocrine enzyme or the like corresponds thereto. An enzyme that contributes to the decomposition or inactivation of a drug such as blasticidin and imparts drug resistance to a host as well as a digestive enzyme corresponds to the exocrine enzyme.

The marker gene is placed in an expressible state downstream of a promoter in the glycosylation agent expression vector. The promoter used may be the same as or different from the middle or posterior silk gland promoter.

### (7) Enhancer

The "enhancer" is a gene expression activation region that can increase the transcription level of a target gene in cooperation with a promoter and is constituted by a particular DNA sequence. Unlike a promoter, the enhancer is placed not only upstream (on the 5'-terminal side) of the target gene but downstream (on the 3'-terminal side) of or within the target gene to regulate the transcription of the target gene.

### (8) Insulator

In the present specification, the "insulator" is an optional component in the glycosylation agent expression vector and is a nucleotide sequence that can stably control the transcription of a gene flanked by the insulator sequences without being influenced by the neighboring chromatin of chromosomes. Examples thereof include a chicken cHS4 sequence and a fruit fly gypsy sequence.

### (9) Inverted terminal repeat sequence of transposon

In the present specification, the "inverted terminal repeat sequence (ITRS) of transposon" is an optional component that may be comprised when the glycosylation agent expression vector is an expression vector capable of homologous recombination. The inverted terminal repeat sequence is usually used as one set of two sequences, and piggyBac, mariner, minos, or the like can be used as the transposon (Shimizu, K. et al., 2000, Insect Mol. Biol., 9, 277-281; and Wang W. et al., 2000, Insect Mol Biol 9 (2): 145-155).

### 1-3-2. Unit configuration of glycosylation agent expression vector

In the glycosylation agent expression vector constituting the mammalian-type glycosylation agent of the present invention, the glycosylation-related genes, etc. are arranged so as to be under direct or indirect expression control of the MSG or PSG promoter. In this context, the "direct or indirect expression control" means the positional relationship between the MSG or PSG promoter and the glycosylation-related genes, etc. in the glycosylation agent expression vector. This depends on the unit configuration of the glycosylation agent expression vector. The glycosylation agent expression vector may be constituted by one unit and may be constituted by two subunits. Hereinafter, each case will be described.

### (1) Case of being constituted by one unit

The glycosylation agent expression vector comprises, in one glycosylation agent expression vector, all the components necessary for expressing the glycosylation-related genes, etc. in silk-spinning insect cells. Specifically, the glycosylation agent expression vector comprises the essential components, i.e., the MSG or PSG promoter and the glycosylation-related genes, etc. functionally linked downstream of the promoter.

In the present specification, the term "functionally linked" means that each of the glycosylation-related genes, etc. is integrated in an expressible state in the glycosylation agent expression vector. Specifically, the term means that each of the glycosylation-related genes, etc. is arranged downstream of the MSG or PSG promoter under the control of the MSG or PSG promoter in the glycosylation agent expression vector. Thus, when the glycosylation agent expression vector is constituted by one unit, the glycosylation-related genes, etc. are to be under direct expression control of the MSG or PSG promoter.

When the glycosylation agent expression vector is constituted by one unit, a transgenic silk-spinning insect having the glycosylation agent expression vector can constantly attach a mammalian-type sugar chain to a recombinant protein produced in a silk gland.

### (2) Case of being constituted by two subunits

When the glycosylation agent expression vector is constituted by two subunits, a first subunit and a second subunit, the components essential for the expression of the glycosylation-related genes, etc. are divided in these subunits. Thus, this configuration functions as one glycosylation agent expression vector only when the first and second subunits coexist with each other in a silk-spinning insect cell of a host. Specifically, in the same cell, the transcriptional control element is expressed from the first subunit by the activation of the promoter comprised in the first subunit and can activate the target promoter in the second subunit, leading to the expression of the glycosylation-related genes, etc. of interest. Thus, when the glycosylation agent expression vector is constituted by two subunits, the glycosylation-related genes, etc. are to be under indirect expression control of the MSG or PSG promoter. The first and second subunits have the following configuration.

The "first subunit" comprises the MSG or PSG promoter and the transcriptional control element gene linked in an expressible state downstream of the promoter. In this respect, two or more transcriptional control element genes may be linked under the control of one MSG or PSG promoter. Examples thereof include GAL4 and tTA linked under the control of the MSG promoter. Alternatively, the first subunit may have two or more sets each consisting of the MSG or PSG promoter and the transcriptional control element gene under the control thereof. In this case, these sets may be the same as or different from each other. Examples thereof include a first subunit comprising a set consisting of the MSG promoter and the GAL4 gene, and a set consisting of the posterior silk gland promoter and the GAL4 gene.

A known MSG or PSG promoter can be used as the promoter contained in the first subunit. Therefore, an existing gene expression vector having the MSG or PSG promoter, prepared for silk-spinning insects such as silkworms can also be used.

The "second subunit" comprises the target promoter of the transcriptional control element encoded in the first subunit, and the glycosylation-related genes, etc. functionally linked downstream of the target promoter. The target promoter comprised in the second subunit is a promoter that is activated by the transcriptional control element encoded in the first subunit. Thus, the target promoter comprised in the second subunit is unambiguously determined by the transcriptional control element encoded in the first subunit, as a rule. For example, if the transcriptional control element gene comprised in the target promoter first subunit is GAL4 gene, UAS is used as the GAL4 target promoter in the second subunit. The second subunit may comprise two or more same or different glycosylation-related genes, etc. under the control of one target promoter. For example, a second subunit corresponds thereto which comprises three genes consisting of the GNE gene, the CMAS gene and the ST6GAL1 gene, four genes consisting of the GNE gene, the CMAS gene, the ST6GAL1 gene and the NANP gene, five genes consisting of the GNE gene, the CMAS gene, the ST6GAL1 gene, the NANS gene and the NANP gene, six genes consisting of the GNE gene, the CMAS gene, the ST6GAL1 gene, the NANS gene, the NANP gene and the SLC35A1 gene, or seven genes consisting of the GNE gene, the CMAS gene, the ST6GAL1 gene, the NANS gene, the NANP gene, the SLC35A1 gene, and the GalT gene, arranged under the control of one UAS.

Alternatively, the second subunit may have two or more sets each consisting of the target promoter and the glycosylation-related genes, etc. under the control thereof. In this case, these sets may be the same as or different from each other. For example, a second subunit corresponds thereto which comprises two UASs, six genes consisting of the GNE gene, the CMAS gene, the ST6GAL1 gene, the NANS gene, the NANP gene, and the SLC35A1 gene arranged under the control of one UAS, and the GalT gene arranged under the control of the other UAS.

The second subunit may be further constituted by two or more same or different units comprising the glycosylation-related genes, etc. In this case, the transcriptional control element expressed from one first subunit can activate the target promoters in a plurality of second subunits, leading to the expression of the glycosylation-related genes, etc. comprised in the respective second subunits. For example, second subunit A comprising six genes consisting of the GNE gene, the CMAS gene, the ST6GAL1 gene, the NANS gene, the NANP gene, and the SLC35A1 gene arranged under the control of UAS, and second subunit B comprising the GalT gene arranged under the control of UAS correspond thereto.

When the glycosylation agent expression vector is constituted by two subunits, a known or existing middle or posterior silk gland-specific gene expression vector can be used as the first subunit. Therefore, an existing transgenic silk-spinning insect line comprising such a gene expression vector can be used.

The glycosylation agent expression vector of this configuration can amplify the expression of the glycosylation-related genes, etc. in the second subunit via the transcriptional control element encoded in the first subunit. Thus, the glycosylation agent expression vector of this configuration is suitable for the overexpression of the glycosylation-related genes, etc. in host cells.

### 1-3-3. Configuration of mammalian-type glycosylation agent

The mammalian-type glycosylation agent of the present invention comprises one to three independent glycosylation agent expression vector(s). In the present specification, the term "independent" means that one glycosylation agent expression vector is capable of functioning by itself as one expression unit that can express at least one glycosylation-related gene, etc. Thus, when the glycosylation agent expression vector is constituted by two subunits as mentioned above, each subunit is not regarded as being "independent", whereas a plurality of subunits together are interpreted as being "independent". On the other hand, when the glycosylation agent expression vector is constituted by one unit, this unit can be interpreted as being "independent".

When the mammalian-type glycosylation agent of the present invention is constituted by a plurality of independent glycosylation agent expression vectors, the combination of the glycosylation-related genes, etc. contained in the respective glycosylation agent expression vectors is not particularly limited. For example, a first glycosylation agent expression vector (first expression vector) may comprise only the GalT gene, and a second glycosylation agent expression vector (second expression vector) may comprise three or more sialic acid-related genes. Alternatively, the first expression vector may comprise only the GalT gene, the second expression vector may comprise three or more sialic acid-related genes except for the SLC35A1 gene, and a third glycosylation agent expression vector (third expression vector) may comprise only the SLC35A1 gene.

### 1-4. Introduction method

A method for introducing the glycosylation agent expression vector into host cells by applying the mammalian-type glycosylation agent of the present aspect to the host will be described.

The host to which the glycosylation agent expression vector is to be introduced may be any of a silk-spinning insect individual, silk-spinning insect-derived cells (including an established cell line) and silk-spinning insect-derived tissues. The individual is not limited by its developmental stage. Any of the embryo, larva, pupa, and adult stages may be used. The embryo stage is preferred which can be expected to be highly effective. The host is not limited by its sex. Likewise, the cells or the tissues are not limited by the developmental stage of an individual from which the cells or the tissues are harvested or derived.

The introduction method can be performed by a method known in the art according to the status of introduction. For example, when the host used in the introduction is a silkworm and the exogenous gene expression vector is a plasmid having an inverted terminal repeat sequence of transposon (Handler AM. et al., 1998, Proc. Natl. Acad. Sci. U.S.A. 95: 7520-5), the introduction can be performed by use of the method of Tamura et al. (Tamura T. et al., 2000, Nature Biotechnology, 18, 81-84). Briefly, a helper vector having DNA encoding transposase can be injected together with the glycosylation agent expression vector to the early embryo of the silkworm. Examples of the helper vector include pHA3PIG. When the glycosylation agent expression vector of the present aspect comprises a marker gene, a transformant can be easily selected on the basis of the expression of the gene, etc. The transgenic silkworm obtained by this method has the glycosylation agent expression vector integrated in the chromosome via the inverted terminal repeat sequence of transposon. The obtained transgenic silkworm may be sib-mated or inbred, if necessary, to obtain a homozygote of the expression vector inserted in the chromosome.

### 2. Transgenic silk-spinning insect capable of glycosylation with mammalian-type sugar chain

### 2-1. Summary

The second aspect of the present invention is a transgenic silk-spinning insect capable of glycosylation with a mammalian-type sugar chain. The transgenic silk-spinning insect capable of glycosylation with a mammalian-type sugar chain according to the present invention has the glycosylation agent expression vector of the first aspect and can attach a mammalian-type sugar chain to a recombinant protein produced in MSG and/or PSG.

### 2-2. Configuration

In the present specification, the "transgenic silk-spinning insect capable of glycosylation with a mammalian-type sugar chain (in the present specification, also referred to as a "glycosylation silk-spinning insect")" refers to a transgenic silk-spinning insect having the glycosylation agent expression vector described in the first aspect. The silk-spinning insect serving as a host may be any of the silk-spinning insects mentioned above. A silkworm, *Samia cynthia ricini* and *Antheraea pernyi* are particularly preferred whose rearing method and artificial diet have been established and which can be reared at a large scale. When the host is a silkworm, the "transgenic silk-spinning insect capable of glycosylation with a mammalian-type sugar chain" according to the present aspect is referred to as a "transgenic silkworm capable of glycosylation with a mammalian-type sugar chain (in the present specification, also referred to as a "glycosylation silkworm")". Also, the mammalian-type sugar chain is preferably a human-type sugar chain.

The glycosylation silk-spinning insect of the present invention may transiently have the glycosylation agent expression vector of the first aspect in cells or may stably have the glycosylation agent expression vector of the first aspect, for example, in a state introduced in the genome. It is preferred to stably have the glycosylation agent expression vector of the first aspect.

The glycosylation silk-spinning insect of the present invention can have two or more different glycosylation agent expression vectors of the first aspect. For example, a glycosylation silk-spinning insect having the first expression vector and the second expression vector described in the first aspect corresponds thereto. When the glycosylation-related genes, etc. essential for achieving mammalian-type glycosylation are divided in the first expression vector and the second expression vector, a glycosylation silk-spinning insect having both the first expression vector and the second expression vector can exert the advantageous effects of the present invention. When the glycosylation agent expression vector is constituted by two subunits, the first subunit and the second subunit, a glycosylation silk-spinning insect having both the subunits can also exert the advantageous effects of the present invention.

When the glycosylation agent expression vector has two or three different expression vectors (first to third expression vectors), each of which is inserted in the chromosome of the transgenic silk-spinning insect, these expression vectors may reside on the same chromosome or may reside on different chromosomes. When the expression vectors reside on different chromosomes, the glycosylation silk-spinning insect of the present invention having the first expression vector and the second expression vector can be easily obtained as a first filial generation (F1) by mating a transgenic silk-spinning insect line having only the first expression vector with a transgenic silk-spinning insect line having only the second expression vector. On the other hand, when the first expression vector and the second expression vector reside on the same chromosome, it is preferred that the subunits should be located at a close interval and linked to each other so as not to be separated by recombination during the process of passage.

The same holds true for the case where the glycosylation agent expression vector is constituted by two subunits, the first and second subunits. For example, when the first and second subunits are located on different chromosomes, the glycosylation silk-spinning insect of the present invention having both the first and second subunits can be easily obtained as F1 by mating a transgenic silk-spinning insect line having only the first expression vector with a line producing a transgenic silk-spinning insect capable of glycosylation with a mammalian-type sugar chain mentioned later, having only the second expression vector.

### 3. Line producing transgenic silk-spinning insect capable of glycosylation with a mammalian-type sugar chain

### 3-1. Summary

The third aspect of the present invention is a line producing a transgenic silk-spinning insect capable of glycosylation with a mammalian-type sugar chain. The line producing a transgenic silk-spinning insect according to the present invention is a transgenic silk-spinning insect having a portion of the glycosylation agent expression vector described in the first aspect, and progeny thereof. The glycosylation silk-spinning insect can be produced any timeand easily by using this line.

### 3-2. Configuration

In the present specification, the "line producing a transgenic silk-spinning insect capable of glycosylation with a mammalian-type sugar chain (in the present specification, also referred to as a "glycosylation silk-spinning insect-producing line")" refers to a a transgenic silk-spinning insect that has the potential to attach a mammalian-type sugar chain to a protein produced in a silk gland and is capable of passage, or progeny thereof. The silk-spinning insect serving as a host may be any of the silk-spinning insects. For the same reason as in the glycosylation silk-spinning insect of the second aspect, a silkworm, *Samia cynthia ricini* and *Antheraea pernyi* are preferred. When the host is a silkworm, the "glycosylation silk-spinning insect-producing line" of the present aspect is referred to as a "glycosylation silkworm-producing line". Also, the mammalian-type sugar chain is preferably a human-type sugar chain.

The glycosylation silk-spinning insect-producing line has a portion of the glycosylation agent expression vector described in the first aspect. When the glycosylation agent expression vector is constituted by two or three expression vectors, only one or some of the expression vectors corresponds to "a portion of the glycosylation agent expression vector". When the glycosylation agent expression vector is constituted by two subunits, only the second subunit corresponds thereto. Specifically, the glycosylation silk-spinning incest-producing line has a portion of the glycosylation agent expression vector described in the first aspect and thereby has the potential to attach a mammalian-type sugar chain to a protein produced in a silk gland. However, the glycosylation silk-spinning worm-producing line does not comprise the minimum glycosylation-related genes, etc. necessary for glycosylation and therefore cannot attach a mammalian-type sugar chain to a protein solo. On the other hand, the glycosylation silk-spinning insect of interest can be easily produced, when needed, by ensuring the minimum necessary glycosylation-related genes, etc. within one individual through mating with another glycosylation silk-spinning insect-producing line or by inducing the expression of the minimum necessary glycosylation-related genes, etc. comprised in the second subunit through mating with a transgenic silk-spinning insect comprising the first subunit. The transgenic silk-spinning insect line having only the first subunit does not have the direct potential to attach a mammalian-type sugar chain and therefore does not correspond to the glycosylation silk-spinning worm-producing line of the present invention.

### 4. Method for producing transgenic silk-spinning insect capable of glycosylation with a mammalian-type sugar chain

### 4-1. Summary

The fourth aspect of the present invention is a method for producing a transgenic silk-spinning insect capable of glycosylation with a mammalian-type sugar chain (glycosylation silk-spinning insect). The method of the present invention can produce a glycosylation silk-spinning insect that can attach a mammalian-type sugar chain to the recombinant protein of interest expressed in a silk gland.

### 4-2. Method

The method for producing a transgenic silk-spinning insect according to the present invention comprises a mating step and a selection step. Hereinafter, each step will be described.

### (1) Mating step

The "mating step" is an essential step of mating the glycosylation silk-spinning worm-producing line having the second subunit described in the third aspect with a transgenic silk-spinning insect line having the first subunit. The silk-spinning insect serving as a host may be any of the silk-spinning insects. For the same reason as in the glycosylation silk-spinning insect of the second aspect, a silkworm, *Samia cynthia ricini* and *Antheraea pernyi* are preferred. The mating can be performed between the two silk-spinning insect lines described above on the basis of a routine method. The mating is performed between a male and a female of the same species, as a rule.

For the respective transgenic silk-spinning insects having the subunit inserted in the genome, it is preferred that the silk-spinning insects of the glycosylation silk-spinning worm-producing line and the transgenic silk-spinning insect line for use in mating should be sib-mated or inbred to obtain a homozygote of each subunit in advance. As a result, all first filial generation (F1) individuals are glycosylation silk-spinning insects having both the subunits. In this case, the next selection step is not an essential step and is an optional step that is performed, if necessary, for confirmation.

### (2) Selection step

The "selection step" is the step of selecting a transgenic silk-spinning insect comprising the two subunits as the glycosylation silk-spinning insect from F1 individuals. In this step, an individual having the activity of the marker proteins encoded in the respective subunits can be selected as the glycosylation silk-spinning insect of interest on the basis of the activity of these marker proteins from F1 individuals obtained after the mating step.

### Examples

Hereinafter, embodiments of the present invention will be described with reference to examples. However, the embodiments described herein are given merely for specifically illustrating the mode for carrying out the present invention. It should be understood that the scope of the present invention is not limited by the scope of Examples given below.

### <Example 1: Construction of each expression vector>

### (Purpose)

A glycosylation agent expression vector constituting the mammalian-type glycosylation agent of the present invention, and an expression vector for a recombinant protein of interest are constructed.

In Examples of the present specification, an expression vector consisting of two subunits, the first subunit and the second subunit, was adopted as each expression vector. Specifically, the expression vector used in Examples of the present specification was constituted by a first subunit comprising MSG promoter and the GAL4 gene functionally linked downstream of the promoter, and a second subunit comprising each sugar chain-related gene functionally linked downstream of UAS promoter, and a gene encoding each recombinant protein of interest.

### (Method)

### 1. Construction of first expression unit

### (1) Construction of first subunit for expression in middle silk gland

pBacSer-pro GAL4/3xP3DsRed2 having a promoter of the sericin 1 gene specifically expressed in MSG, the transcriptional control element GAL4 gene functionally connected downstream of the promoter, and a hsp70 polyA addition sequence further connected downstream thereof was constructed as the first subunit inducing gene expression in the middle silk gland (MSG).

The sericin 1 gene promoter was prepared by PCR-amplifying a promoter-containing region, shown in SEQ ID NO: 1, corresponding to -666 to +40 (the transcription start site is defined as position 0; the same holds true for the description below) of the silkworm sericin 1 gene using genomic DNA of a silkworm Daizo line as a template and a primer pair consisting of an AscI site-containing primer shown in SEQ ID NO: 63 and a BamHI site-containing primer shown in SEQ ID NO: 64. The amplified fragment was inserted to pCR-Blunt II-TOPO vector (Thermo Fisher Scientific Inc.) and cleaved with AscI and BamHI. Then, the AscI-BamHI amplified fragment containing the promoter region was inserted to an AscI-BamHI site upstream of the GAL4 gene in pBacA3dGAL4 (Uchino K. et al., 2006, J Insect Biotechnol Sericol 75: 89-97). 3xP3-DsRed cassette excised from pBacA3GAL4/3xP3DsRed2 (Uchino K. et al., 2006, J Insect Biotechnol Sericol 75: 89-97) with BglII was inserted as a selection marker to the plasmid to construct a first subunit pBacSer-pro GAL4/3xP3DsRed2 for expression in MSG.

### 2. Construction of second expression unit

### (1) Construction of basic vector

PCR was performed using pBac[SerUAS-hr5/3xP3-EGFPinv] (Tada M. et al., 2015, MAbs. 7 (6): 1138-1150) as a template and a primer pair consisting of SerTATA-U (SEQ ID NO: 65) and BlnBsmSerK-L (SEQ ID NO: 66) to obtain an amplification product SnaBI-BsmBI fragment. Next, pBac[SerUAS_Serlintron_hr5/3xP3-EYFP_A3-Bla] (Tada M. et al., 2015, MAbs. 7(6):1138-1150) was cleaved with SnaBI and BsmBI for the removal of a short fragment, and the SnaBI-BsmBI fragment was inserted to the SnaBI-BsmBI site. The resultant was designated as pBac[SerUAS_Ser1kozak_hr5/3xP3-EYFP_A3-Bla]. Subsequently, PCR was performed using pHC-EGFP as a template and a primer pair consisting of FibHsig-U (SEQ ID NO: 67) and FibHsig-L (SEQ ID NO: 68) to obtain an amplification product BspH-BlnI fragment. The pBac[SerUAS_Serlkozak_hr5/3xP3-EYFP_A3-Bla] mentioned above was cleaved with BsmBI and BlnI for the removal of a short fragment, and the BspH-BlnI fragment was inserted to the BsmBI-BlnI site. The resultant was designated as pBac[SerUAS_FibHsigint_hr5/3xP3-EYFP_A3-Bla] and used as a basic expression vector for the second expression unit in an expression vector.

### (2) Construction of glycosylation agent expression vector

### A. Construction of GalT expression vector

### (Construction of conventional-type GalT2 expression vector)

The promoter region and the 3'UTR region of the sericin 1 gene were each amplified using silkworm genomic DNA as a template. Then, both the amplified fragments were linked by overlap-extension PCR. In this operation, a BlnI site was inserted to the boundary between the promoter region and 3'UTR. This linked fragment was inserted to pTA vector (Toyobo Co., Ltd.), and the resultant was designated as pTA2[Ser-UTR]. A GAL4Δ fragment (Kobayashi I., et al., 2011, Arch Insect Biochem Physiol, 76: 195-210) was inserted to the BlnI site of pTA2[Ser-UTR], and the resultant was designated as pTA2[Ser-GAL4Δ]. The AscI fragment of pTA2[Ser-GAL4Δ] was inserted to the AscI site of pBac[A3KMO, UAS] (Kobayashi I., et al., 2007, J. Insect Biotechnol Sericol, 76: 145-48), and the resultant was designated as pBac[A3KMO, UAS, Ser-GAL4Δ].

The mouse-derived GalT2 open reading frame was amplified by PCR such that a BlnI site was added to the terminal. This fragment was inserted to the BlnI site of pBac[A3KMO, UAS, Ser-GAL4Δ], and the resultant was designated as pBac[SerUAS-GalT2p/A3-KMO_A3-Bla] which was a conventional-type GalT2 expression vector.

### (Construction of basic vector for improved-type GalT expression vector)

The basic expression vector pBac[SerUAS_Serlkozak_hr5/3xP3-EYFP_A3-Bla] constructed in the preceding section (1) was cleaved with AscI and XhoI for the removal of a short fragment, and an adapter prepared by annealing AscI-NheI-XhoI-U (SEQ ID NO: 69) and AscI-NheI-XhoI-L (SEQ ID NO: 70) was inserted to the AscI-XhoI site. The resultant was designated as pBac[SerUAS_Ser1kozak_hr5/NheIad_A3-Bla]. The NheI fragment of pBac[A3KMO, UAS, Ser-GAL4Δ] was inserted to the NheI site of the plasmid. The resultant was designated as pBac[SerUAS_Ser1kozak_hr5/A3-KMO_A3-Bla].

### (Construction of each improved-type GalT expression vector)

The mouse-derived GalT1 to GalT4 genes were subjected to PCR using primer pairs consisting of BspHI-mGalT1 U (SEQ ID NO: 71) and mouse BlnI-mGalT1 L (SEQ ID NO: 72) for mouse GalT1, BsmBI-GalT2-U (SEQ ID NO: 73) and BsmBI-GalT2-L (SEQ ID NO: 74) for GalT2, BsmBI-mGalT3 U (SEQ ID NO: 75) and mouse BsmBI-mGalT3 L (SEQ ID NO: 76) for mouse GalT3, and NcoI-mGalT4 U (SEQ ID NO: 77) and mouse BlnI-mGalT4 L (SEQ ID NO: 78) for mouse GalT4. Each amplification product was cleaved with a restriction enzyme described in each primer name and then inserted to the BsmBI site of the basic vector pBac[SerUAS_Serlkozak_hr5/A3-KMO_A3-Bla] for an improved-type GalT vector. The obtained improved-type GalT expression vectors were designated as pBac[SerUAS-GalT1/A3-KMO_A3-Bla], pBac[SerUAS-GalT2i/A3-KMO_A3-Bla], pBac[SerUAS-GalT3/A3-KMO_A3-Bla], and pBac[SerUAS-GalT4/A3-KMO_A3-Bla], respectively. GalT2p represents conventional-type GalT2, and GalT2i represents improved-type GalT2.

### B. Construction of sialic acid-related gene expression vector

### (Construction of UAS unit vector)

PCR was performed using the basic expression vector pBac[SerUAS_Serlkozak_hr5/3xP3-EYFP_A3-Bla] constructed in the preceding section (1) as a template and a primer pair consisting of serUASUNhe (SEQ ID NO: 79) and serPolyALSpe (SEQ ID NO: 80). The obtained amplification product was inserted to the EcoRV site of pZErO2 (Thermo Fisher Scientific Inc.). The resultant was designated as SerUAS_unit/pZErO2 which was a UAS unit vector.

### (Insertion of sialic acid-related gene fragment to UAS unit vector)

ORF of each of sialic acid-related genes (GNE, NANS, NANP, CMAS, ST6GAL1, and SLC35A1 genes) was amplified by PCR using a primer pair given below to obtain each sialic acid-related gene fragment. The primer pair used for the GNE gene was r2epiU (SEQ ID NO: 81) and r2epiL (SEQ ID NO: 82). The primer pair used for the NANS gene was BsmBI_NANS_U (SEQ ID NO: 83) and BsmBI_NANS_L (SEQ ID NO: 84). The primer pair used for the NANP gene was BsmBI_NANP_U (SEQ ID NO: 85) and BsmBI_NANP_L (SEQ ID NO: 86). The primer pair used for the CMAS gene was hCSSU (SEQ ID NO: 87) and hCSSL (SEQ ID NO: 88). The primer pair used for the SLC35A1 gene was BsmBI_hCST_U (SEQ ID NO: 89) and BsmBI_hCST_L (SEQ ID NO: 90). The primer pair used for the ST6GAL1 gene was hSTU (SEQ ID NO: 91) and hSTL (SEQ ID NO: 92). Each sialic acid-related gene fragment was cleaved with BsmBI and then inserted to the BsmBI site of the UAS unit vector SerUAS_unit/pZErO2 mentioned above. The finished UAS unit vectors comprising each sialic acid-related gene were designated as UAS-GNE/pZErO2 (for GNE expression), UAS-NANS/pZErO2 (for NANS expression), UAS-NANP/pZErO2 (for NANP expression), UAS-CMAS/pZErO2 (for CMAS expression), UAS-ST6GAL1/pZErO2 (for ST6GAL1 expression), and UAS-SLC35A1/pZErO2 (for SLC35A1 expression).

### (Construction of HS4 insulator unit vector)

A HS4 insulator sequence consisting of the nucleotide sequence represented by SEQ ID NO: 93 was synthesized on a consignment basis by GenScript Japan Inc., cleaved with NheI and SpeI, and inserted to the SpeI site of a vector containing the original HS4 insulator. This operation was repeated to construct a plasmid having 4 repeats of the HS4 insulator. This plasmid was designated as HS4x4/pUC.

### (Construction of piggyBac/3xP3AmCyan vector)

An adapter prepared by annealing SpeIadaptU (SEQ ID NO: 94) and SpeIadaptL (SEQ ID NO: 95) was inserted to the EcoRV-PstI site of pBac[SerUAS/3xP3EGFP] (Tatematsu K, et al., 2010, Transgenic Res. 19 (3): 473-87). The resultant was designated as pBac[3xP3EGFP]. The EcoRI fragment of pBac[SerUAS_Serlintron_hr5/3xP3-AmCyan_A3-Bla] (Tada M. et al., 2015, MAbs. 7 (6): 1138-1150) was inserted to the EcoRI site of pBac[3xP3EGFP]. The resultant was designated as pBac[3xP3AmCyan].

### (Insertion of UAS sialic acid unit and HS4 insulator to piggyBac/3xP3AmCyan)

The UAS unit vectors UAS-GNE/pZErO2 (for GNE expression), UAS-NANS/pZErO2 (for NANS expression), UAS-NANP/pZErO2 (for NANP expression), UAS-CMAS/pZErO2 (for CMAS expression), UAS-ST6GAL1/pZErO2 (for ST6GAL1 expression), and UAS-SLC35A1/pZErO2 (for SLC35A1 expression) comprising each sialic acid-related gene, and the HS4 insulator unit vector HS4x4/pUC mentioned above were each cleaved with NheI and SpeI. Subsequently, the NheI-SpeI fragments of 3 vectors (UAS-GNE/pZErO2, UAS-CMAS/pZErO2 and UAS-ST6GAL1/pZErO2) and the NheI-SpeI fragment containing the HS4 insulator were inserted to the SpeI site of piggyBac/3xP3AmCyan such that each UAS sialic acid unit and each repeat of the HS4 insulator were alternately linked. The resultant was designated as pBac[HS4-UAS-GNE-HS4-UAS-CMAS-HS4-UAS-ST6GAL1-HS4/3xP3AmCyan] (hereinafter, abbreviated to pBac[UAS-GNE/CAMS/ST6GAL1/3xP3AmCyan]). Next, the NheI-SpeI fragments of 2 vectors represented by UAS-NANS/pZErO2 and UAS-NANP/pZErO2 were inserted again to pBac[UAS-GNE/CAMS/ST6GAL1/3xP3AmCyan] such that these two NheI-SpeI fragments were sandwiched between the NheI-SpeI fragments containing the HS4 insulator. The resultant was designated as pBac[HS4-UAS-NANS-HS4-UAS-NANP-HS4-UAS-GNE-HS4-UAS-CMAS-HS4-UAS-ST6GAL1-HS4/3xP3AmCyan] (hereinafter, abbreviated to pBac[UAS-NANS/NANP/GNE/CAMS/ST6GAL1/3xP3AmCyan]). Also, the NheI-SpeI fragments of 3 vectors represented by UAS-NANS/pZErO2, UAS-NANP/pZErO2 and UAS-SLC35A1/pZErO2 were inserted to pBac[UAS-GNE/CAMS/ST6GAL1/3xP3AmCyan] such that these three NheI-SpeI fragments were sandwiched between the NheI-SpeI fragments containing the HS4 insulator. The resultant was designated as pBac[HS4-UAS-NANS-HS4-UAS-NANP-HS4-UAS-SLC35A1-HS4-UAS-GNE-HS4-UAS-CMAS-HS4-UAS-ST6GAL1-HS4/3xP3AmCyan] (hereinafter, abbreviated to pBac[UAS-NANS/NANP/SLC35A1/GNE/CAMS/ST6GAL1/3xP3AmCyanD. As a result, sialic acid-related gene expression vectors comprising three UAS sialic acid units (GNE/CAMS/ST6GAL1), five UAS sialic acid units (NANS/NANP/GNE/CAMS/ST6GAL1), or six UAS sialic acid units (NANS/NANP/SLC35A1/GNE/CAMS/ST6GAL1) were obtained.

### (3) Construction of expression vector for recombinant protein of interest

### (Construction of human antithrombin III expression vector)

PCR was performed using vector Flexi ORF clone FHC11758 (Promega Corp.) containing ORF of the human antithrombin III (hATIII) gene as a template and a primer pair consisting of BsmBI_AT_FibHsig_U40 (SEQ ID NO: 96) and AT_C-6His_L45 (SEQ ID NO: 97) containing a His tag. The BsmBI fragment of the obtained amplification product was inserted to the BsmBI site of the basic expression vector pBac[SerUAS_FibHsigint_hr5/3xP3-EYFP_A3-Bla] constructed in the section (1). The resultant was designated as UAS-rATIII/pZErO2.

### (Construction of human interferon γ expression vector)

PCR was performed using Flexi ORF clone ORH24802 (Promega Corp.) containing ORF of the human interferon γ (hIFNγ) gene as a template and a primer pair consisting of hIFNg_FibHsig_U40 (SEQ ID NO: 98) and hIFNg_C-6His_L45 (SEQ ID NO: 99) containing a His tag. The BsmBI fragment of the obtained amplification product was inserted to the BsmBI site of the basic expression vector pBac[SerUAS_FibHsigint_hr5/3xP3-EYFP_A3-Bla] constructed in the section (1). The resultant was designated as UAS-hIFNg/pZErO2.

### <Example 2: Production of transgenic silkworm>

### (Purpose)

Various transgenic silkworms were produced using each expression vector constructed in Example 1.

### (Material and method)

### (1) Silkworm line

A w1-pnd line, which is a non-dormant line having white eyes and white eggs, maintained in National Institute of Agrobiological Sciences (NIAS) was used as a host line.

### (2) Rearing conditions

Larvae were reared with an artificial diet (SilkMate PS, Nosan Corp.) throughout the entire larval instars in a rearing room of 25 to 27°C. The artificial diet was replaced every 2 to 3 days (Uchino K. et al., 2006, J Insect Biotechnol Sericol, 75: 89-97).

### (3) Production of transgenic silkworm

Transgenic silkworms were produced according to the method of Tamura et al. (Tamura T. et al., 2000, Nature Biotechnology, 18, 81-84). The first subunit and the second subunit constructed in Example 1 were each separately mixed with a helper plasmid pHA3PIG (Tamura T. et al., 2000, Nature Biotechnology, 18, 81-84) expressing transposase at a ratio of 1:1, and the mixture was injected to silkworm eggs 2 to 8 hours after egg laying. The eggs after the injection were incubated at 25°C in a humidified state until hatching. Hatched larvae were reared by the method described above.

### (Production of transgenic silkworm line for GAL4 expression)

For the production of a transgenic silkworm line for GAL4 expression comprising the first subunit, the first subunit pBacSer-pro GAL4/3xP3DsRed2 for MSG expression was used in the injection described above. Hatched larvae after the injection were reared by the method described above. F1 individuals comprising the first subunit were selected on the basis of the presence or absence of eye fluorescence by the 3xP3DsRed2 marker to obtain a transgenic silkworm line for GAL4 expression comprising the first subunit of the transgenic silkworm of the present invention.

### (Production of GalT glycosylation silkworm producing line)

For the production of a GalT glycosylation silkworm-producing line comprising the second subunit, the GalT2p expression vector and each improved-type GalT expression vector (pBac[SerUAS-GalT1/A3-KMO_A3-Bla], pBac[SerUAS-GalT2i/A3-KMO_A3-Bla], pBac[SerUAS-GalT3/A3-KMO_A3-Bla], and pBac[SerUAS-GalT4/A3-KMO_A3-Bla]) were each used in the injection described above. Hatched larvae after the injection were reared by the method described above. F1 individuals comprising each improved-type GalT expression vector were selected on the basis of the colored body surface of the first instar larvae by the A3-KMO marker to obtain each GalT glycosylation silkworm-producing line.

### (Production of sialic acid-related protein glycosylation silkworm-producing line)

For the production of a sialic acid-related protein glycosylation silkworm-producing line comprising the second subunit, each sialic acid-related gene expression vector mentioned above was used in the injection described above. Hatched larvae after the injection were reared by the method described above. F1 individuals comprising each expression vector were selected on the basis of the presence or absence of eye fluorescence by the 3xP3-AmCyan marker to obtain each sialic acid-related protein glycosylation silkworm-producing line.

### (Production of transgenic silkworm line for expression of recombinant protein of interest)

For the production of a transgenic silkworm line for the expression of the recombinant protein of interest comprising the second subunit, the antithrombin III expression vector or the interferon γ expression vector mentioned above was used in the injection mentioned above. Hatched larvae after the injection were reared by the method described above. F1 individuals comprising each expression vector were selected on the basis of the presence or absence of eye fluorescence by the 3xP3-EYFP marker to obtain a transgenic silkworm line for ATIII expression and a transgenic silkworm line for INFγ expression. Then, the F1 individuals were sib-mated to obtain homozygotes.

### (4) Mating of lines comprising first and second subunits

The transgenic silkworm line for GAL4 expression comprising the first subunit was mated with each line comprising the second subunit (GalT glycosylation silkworm-producing line, each sialic acid-related protein glycosylation silkworm-producing line, and transgenic silkworm line for the expression of the recombinant protein of interest).

Specifically, first, the transgenic silkworm line for GAL4 expression comprising the first subunit was mated with the transgenic silkworm line for the expression of the recombinant protein of interest (ATIII or IFNγ) comprising the second subunit; the transgenic silkworm line for GAL4 expression comprising the first subunit was mated with the GalT glycosylation silkworm-producing line comprising the second subunit; and the transgenic silkworm line for GAL4 expression comprising the first subunit was mated with each sialic acid-related protein glycosylation silkworm-producing line comprising the second subunit. The expression of each gene was confirmed, and protein extraction was performed. Each first filial generation (F1) comprising the first and second subunits was selected. The [GAL4 × ATIII or IFNγ] F1 silkworm was mated with the [GAL4 × GalT] F1 silkworm; and the [GAL4 × ATIII or IFNγ] F1 silkworm was mated with the [GAL4 × sialic acid-related proteins] F1 silkworm. The expression of each gene was confirmed, and protein extraction was performed. Each second filial generation (F2) comprising the first subunit and the two second subunits was selected. Finally, the [GAL4 × ATIII or IFNγ/GalT] F2 silkworm was mated with the [GAL4 × ATIII or IFNγ/sialic acid-related proteins] F2 silkworm. The expression of each gene was confirmed, and protein extraction was performed. As a result, a F3 silkworm comprising the four subunits (first subunit and three second subunits) was produced. In addition, the [GAL4 × GalT] F1 silkworm was mated with [GAL4 × sialic acid silkworm] F1 silkworm by the same procedures as above to produce a [GAL4 × GalT/sialic acid-related proteins] F2 silkworm. Since the transgenic silkworm line for GAL4 expression, each sialic acid-related gene transgenic silkworm line, each GalT gene transgenic silkworm line, and each transgenic silkworm for the expression of the recombinant protein of interest all differed in selection marker, the order of mating is not particularly limited.

Table 1 shows the combinations of the transgenic silkworm comprising the GalT expression vector (GalT gene transgenic silkworm) and the transgenic silkworms comprising various sialic acid-related gene expression vectors (sialic acid-related gene transgenic silkworms). The respective lines having the subunits were selected from F1 individuals after mating on the basis of the marker.

**[Table 1]**

| | | Galt gene transgenic silkworm | | | | | |
|---|---|---|---|---|---|---|---|
| | | - | GalT1 | GalT2p | GalT2i | GalT3 | GalT4 |
| Sialic acid-related gene transgenic silkworm | - | ○ | ○ | ○ | ○ | ○ | ○ |
| | 3 genes | O | × | O | O | × | × |
| | 5 genes | ○ | ○ | ○ | ○ | × | × |
| | 6 genes | ○ | ○ | ○ | ○ | × | × |

| | | | | | | | |
|---|---|---|---|---|---|---|---|
| 3 genes: rGNE, hCAMS, and hST6GAL1 5 genes: rGNE, hCAMS, hST6GAL1, hNANS, and hNANP 6 genes: rGNE, hCAMS, hST6GAL1, hNANS, hNANP, and SLC35A1 | | | | | | | |

In the table, "-" represents that the transgenic silkworm comprising the GalT expression vector or the sialic acid-related gene expression vector was not used in mating. In the table, the circle represents that each transgenic silkworm having the expression vectors was obtained by mating. The x-mark represents that mating was not performed.

In this Example, transgenic silkworms were obtained which comprised pBacSer-pro GAL4/3xP3DsRed2 as the common first subunit for expression in MSG as well as only the expression vector for the recombinant protein of interest (ATIII or IFNγ), only the GalT gene expression vector, only the sialic acid-related gene expression vector (3 genes, 5 genes, or 6 genes), recombinant protein-of-interest expression vector/GalT gene expression vector, recombinant protein-of-interest expression vector/sialic acid-related gene expression vector (3 genes, 5 genes, or 6 genes), GalT gene expression vector/sialic acid-related gene expression vector (3 genes, 5 genes, or 6 genes), or recombinant protein-of-interest expression vector/GalT gene expression vector/sialic acid-related gene expression vector as the second subunit(s).

### <Example 3: Extraction of MSG lumen protein of glycosylation silkworm>

### (Purpose)

Lumen proteins including the recombinant protein are extracted from the MSG lumens of the ATIII glycosylation silkworms, the IFNγ glycosylation silkworms, and the non-ATIII/IFNγ-expressing silkworms for control produced in Example 2.

### (Method)

Each glycosylation silkworm produced in Example 2 was reared in the same way as in Example 2. The fifth instar day-6 larva was anesthetized on ice immediately before spinning. An incision was made on its back, and MSG was excised using tweezers without damage (see Yasushi Mori, ed., Kaiko ni yoru shin seibutsugaku jikken (New Biological Experiments using Silkworms in English), Sanseido Bookstore Ltd., 1970, pp. 249-255). Subsequently, the excised MSG was fixed with ethanol and separated into lumen proteins and cells using tweezers. The lumen proteins obtained from each of the ATIII glycosylation silkworms and the IFNγ glycosylation silkworms were dissolved in LiBr to prepare a MSG extract. Alternatively, the excised MSG was placed in 1 mL of a 100 mM phosphate buffer (pH 7.2) per gland and shaken at 4°C for 2 hours to extract water-soluble proteins. Then, the extract was centrifuged at 2000 × g for 10 minutes, followed by the recovery of a supernatant.

### <Example 4: Purification and preparation of recombinant protein from MSG extract>

### (Purpose)

The recombinant protein of interest obtained in Example 3 is separated and purified, and prepared for sugar chain structure analysis.

### (Method)

### (1) Measurement of protein concentration

A protein concentration in each MSG extract was measured by the Bradford method. BSA was used as a standard protein. The absorbance at OD₅₉₅ was measured as to serial dilutions (BSA concentration: 0 to 1.0 mg/mL) prepared using a Bradford reagent (Nacalai Tesque, Inc.) to prepare a calibration curve. OD₅₉₅ was also measured as to each MSG extract, and the protein concentration was determined by comparison with the calibration curve.

### (2) Protein purification

### (Human antithrombin III purification)

A column was packed with 500 µL of Ni resin (profanity IMAC Ni-charged resin) and equilibrated with an equilibrating solution (50 mM sodium phosphate buffer/0.3 M NaCl). Five hundreds (500) µL of the MSG extract was loaded in the column, which was then washed with 1.5 mL of a washing solution (50 mM sodium phosphate buffer/0.3 M NaCl/10 mM imidazole). Subsequently, the protein of interest was eluted with 300 µL of an eluent (50 mM sodium phosphate buffer/0.3 M NaCl/200 mM imidazole). The eluate was concentrated with a 50 mM sodium phosphate buffer using Amicon Ultra-0.5 mL (30K), and the protein concentration was measured. Then, 4 × sample buffer (4.0 mL of glycerol/1.67 mL of 1.5 M Tris-HCl buffer (pH 6.8)/1.0 mL of 10% SDS solution/400 µL of β-mercaptoethanol) was added to a 1 µg aliquot, and the protein was denatured at 100°C for 3 minutes and separated by SDS-PAGE.

### (Human interferon γ purification)

A column was packed with 50 µL of Ni resin (profanity IMAC Ni-charged resin) and equilibrated with an equilibrating solution (50 mM sodium phosphate buffer/0.3 M NaCl). Subsequently, 500 µL of the MSG extract was loaded in the column, which was then washed with 1.5 mL of a washing solution (50 mM sodium phosphate buffer/0.3 M NaCl/20 mM imidazole). Subsequently, the protein of interest was eluted with 300 µL of an eluent (50 mM sodium phosphate buffer/0.3 M NaCl/200 mM imidazole). The eluate was concentrated with a 50 mM sodium phosphate buffer using Amicon Ultra-0.5 mL (10K), and the protein concentration was measured. Then, 4 × sample buffer (4.0 mL of glycerol/1.67 mL of 1.5 M Tris-HCl buffer (pH 6.8)/1.0 mL of 10% SDS solution/400 µL of β-mercaptoethanol) was added to a 1 µg aliquot, and the protein was denatured at 100°C for 3 minutes and separated by SDS-PAGE.

### (3) Protein separation

### (Human antithrombin III sample preparation)

Three point three (3.3) µL of 4 × sample buffer (4.0 mL of glycerol/1.67 mL of 1.5 M Tris-HCl buffer (pH 6.8)/1.0 mL of 10% SDS solution/400 µL of β-mercaptoethanol) was added to 10 µL of each eluate from the Ni column, and the eluate was denatured at 100°C for 3 minutes. After centrifugation, the supernatant was recovered, and 1.1 µL of the MSG extract and 10 µL of the recovered supernatant were subjected to SDS-PAGE separation. The gel after the electrophoresis was stained with CBB to confirm hATIII purification.

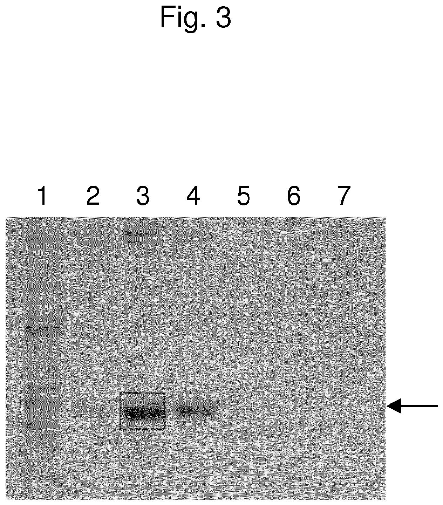

The results are shown in Figure 3. The band of hATIII was able to be confirmed in the purified fraction.

### (Human interferon γ sample preparation)

Each eluate from the Ni column was adjusted to 1.2 mL with acetone and left standing at -20°C for 3 days. Then, the supernatant was removed, followed by centrifugal drying. The precipitate was dissolved by the addition of 20 µL of 1 × sample buffer and denatured at 100°C for 3 minutes. After centrifugation, the supernatant was recovered, and 11.25 µL of the MSG extract and 10 µL of the recovered supernatant were subjected to SDS-PAGE separation. The gel after the electrophoresis was stained with CBB to confirm hIFNγ purification.

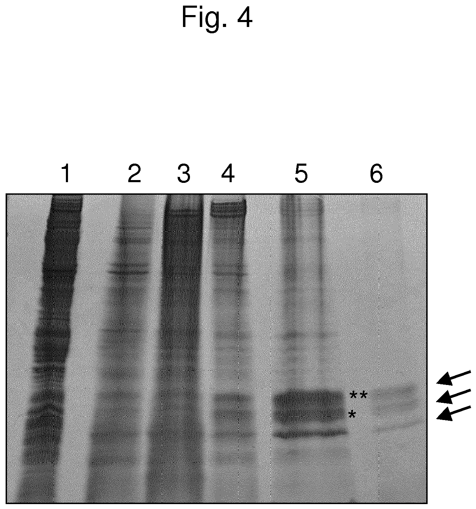

The results are shown in Figure 4. Since three bands were confirmed in the purified fraction, hIFNγ was detected by Western blotting using a mouse-derived anti-His antibody diluted 3000-fold. The hIFNγ gene fragment contained the 6-His tag at the terminal by the primers used for cloning, as mentioned above. A HRP anti-mouse IgG antibody diluted 10000-fold was used as a secondary antibody. As a result, the three bands were all confirmed to be hIFNγ. hIFNγ deglycosylated using PNGase F was detected by Western blotting. As a result, the three bands converged to one band at the smallest molecular weight. From these results, it was predicted that three divided bands were detected depending on the presence or absence of glycosylation at two glycosylation sites of hIFNγ.

### (4) In-gel digestion

### (In-gel digestion of human interferon γ)

The protein (hIFNγ) bands (two bands of sugar chain-attached proteins indicated by ^{∗} and ^{∗∗} in Figure 4) separated by SDS-PAGE were excised and destained by 15-minute vortex twice using 50 mM NH₄HCO₃/50% MeCN (acetonitrile). Then, the resulting gel was vortexed with 100% MeCN for 5 minutes and vortexed again overnight with 50 mM NH4HCO3/50% MeCN. On the next morning, 15-minute vortex was performed twice using 100% MeCN, and MeCN was removed by centrifugal drying.

### (Intra-gel digestion of human antithrombin III)

The protein (hATIII) band of interest separated by SDS-PAGE was excised and destained by 15-minute vortex twice using 50 mM NH₄HCO₃/50% MeCN (acetonitrile). Then, the resulting gel was vortexed with 100% MeCN for 5 minutes and vortexed again overnight with 50 mM NH₄HCO₃/50% MeCN. On the next morning, 15-minute vortex was performed twice using 100% MeCN, and MeCN was removed by centrifugal drying.

Reduction reaction was performed at 56°C for 20 minutes by the addition of 50 mM NH₄HCO₃ containing 25 mM DDT. Then, the DTT solution as the supernatant was removed, and the same amount thereas of 50 mM NH₄HCO₃ containing 50 mM iodoacetamide was added to the gel, followed by occasional shaking for 20 minutes in the dark. Purified water was added thereto, and the mixture was vortexed. Then, MeCN was added thereto, and 10-minute vortex was repeated twice. 50 mM NH₄HCO₃ was added thereto, and the gel was swollen for 15 minutes and then dehydrated with MeCN, followed by the removal of MeCN by centrifugal drying.

20 µL of a trypsin solution was added thereto on ice, and the gel was left standing for 30 minutes and swollen. Then, 50 mM NH₄HCO₃ containing 0.01% ProteaseMAX^{(™)} (Promega Corp.) was added thereto so as to soak the gel, followed by incubation at 50°C for 1 hour. The protease treatment may be performed using chymotrypsin. In this case, the incubation is performed at 25°C for 2 to 18 hours. The enzymatically treated solution was recovered by centrifugation at 15000 × g for 10 minutes. In order to inactivate trypsin, TFA was added thereto at 0.5%.

### <Example 5: Sugar chain structure analysis of recombinant protein>

### (Purpose)

The structures of sugar chains attached to the digested peptides obtained in Example 4 are analyzed by use of nanoLC-MS/MS.

### (Method)

6 µL or 8 µL of the enzymatically treated solution obtained in Example 4 was subjected to nanoLC-MS/MS under analysis conditions given below. A specific analysis method abided by the instruction manual attached to each instrument used.

### (Liquid chromatography (LC) conditions)

- Instrument used: Agilent Technologies 1200 series
- Eluent A: 0.1% HCOOH/Milli-Q
- Eluent B: 0.1% HCOOH/MeCN
- Column: ZORBAX 300SB-C18 (Agilent Technologies, Inc.) 150 mm × 100 µm, 3.5 µm particles
- Flow rate: 0.6 µL/min
- Time schedule concentration gradient (min): 0 → 5 → 65 → 66 → 71 → 72 → 90
   B (%): 2 → 8 → 50 → 95 → 95 → 2 → 2

### (MS/MS conditions)

- Instrument used: micrOTOF-Q Bruker
- Mass range: 50 to 4,500 m/z
- Ionization method: ESI
- Scan rate: 5 KHz
- Analysis software: Hystar

Sugar chain-attached peptides were identified from the results of nanoLC-MS/MS, and the structures of the sugar chains attached to the peptides were predicted.

### (Results)

### (1) Sugar chain structure analysis of human antithrombin III

The human antithrombin III is known to have 4 asparagine residues (N128, N167, N87, and N224; the initiation methionine is defined as M1; the same holds true for the description below) that undergo glycosylation (Zhou Z. & Smith D.L., 1990, Biomedical and environmental mass spectrometry, 19: 782-786).

### (Comparison of galactose attachment efficiency among GalT isozymes)

The sugar chain structures of proteins prepared from the silk glands of the transgenic silkworms harboring each isozyme gene of GalT were analyzed. Table 2 shows the profiles of the sugar chain structures. In the table, the numeric values indicate the ratio (%) of each sugar chain with respect to 100% in total of sugar chains detected.

**[Table 2]**

| Sugar chain structure | GalT2p | GalT2i | GalT1 | GalT3 | GalT4 |
|---|---|---|---|---|---|
| Man₅A | 17.82 | 12.75 | 24.93 | 12.54 | 31.33 |
| Man₃ | 8.40 | 6.39 | 11.31 | | 7.93 12.01 |
| Man₃F | | | | 5.83 | |
| Man₂F | 9.69 | 27.84 | 16.47 | 7.56 | 8.10 |
| _{GlcNAc}Man₃ | 18.12 | 15.42 | 5.83 | 10.92 | 7.13 |
| ^{GlcNAc}Man₃ | 3.69 | 4.72 | | 4.83 | |
| _{GlcNAc}Man₃F | | | | 4.06 | 0.78 |
| ^{GlcNAc} Man₃F | | | | 1.23 | |
| ^{GlcNAc} Man₂B | | | | 0.94 | |
| GlcNAc₂Man₃ | 0.94 | 1.04 | 8.37 | 25.66 | 16.31 |
| GlcNAc₂Man₃F | | | | 1.44 | 0.59 |
| _{Gal}GlcNAcMan₃ | 3.99 | 3.77 | - | 1.48 | - |
| ^{Gal}GlcNAcMan₃ | | | | 1.02 | |
| _{Gal}GlcNAcMan₃F | 0.81 | - | - | 1.54 | - |
| GalGlcNAc₂Man₃ | 0.63 | 2.53 | 1.42 | 1.60 | 0.74 |
| Gal₂GlcNAc₂Man₃ | 0.51 | 1.13 | 0.95 | 0.74 | - |
| GalGlcNAcMan₅A | 10.38 | 2.06 | 5.12 | 1.11 | - |
| Total Gal attachment | 16.32 | 9.49 | 7.49 | 7.49 | 0.74 |

As seen from the results of Table 2, among the GalT isozymes, GalT2p (conventional-type GalT2) had the highest galactose attachment efficiency, followed by GalT2i (improved-type GalT2). It was further revealed that: GalT1 most generally used for galactose attachment in the previous studies has the same level of attachment efficiency as that of GalT3; and the attachment efficiency of GalT4 is lowest. Accordingly, data obtained using the GalT2p, GalT2i, and GalT1 gene transgenic silkworms will be shown below.

### (Relationship between the number of sialic acid-related gene introduced and sugar chain structure)

In order to test the number of sialic acid-related genes introduced and the presence or absence of sialic acid attachment, the sugar chain structures of proteins prepared from the silk glands of the respective glycosylation silkworms obtained by mating a GalT gene transgenic silkworm with various sialic acid-related gene transgenic silkworms (3 genes, 5 genes, and 6 genes) were analyzed. Table 3 shows the profiles of the sugar chain structures. The gene of GalT2i found to have high galactose attachment efficiency was used as the GalT gene. The results are shown in Table 3. In the table, the numeric values indicate the ratio (%) of each sugar chain with respect to 100% in total of sugar chains detected. In the table, for example, Man₅ represents a structure containing 5 mannoses at a sugar chain terminal, and Man₃F represents a structure containing 3 mannoses and 1 fucose modification.

**[Table 3]**

| Galactose | GalT2i | | | |
|---|---|---|---|---|
| Sialic acid | - | 3 genes | 5 genes | 6 genes |
| Man₅ | 12.75 | 28.29 | 11.15 | 15.64 |
| Man₄ | 1.34 | 2.46 | 8.45 | 0 |
| Man₃ | 6.39 | 8.83 | 5.65 | 4.69 |
| Man₃F | 1.46 | 0 | 0 | 0 |
| Man₂ | 5.02 | 4.24 | 1.03 | 2.66 |
| Man₂F | 27.84 | 14.11 | 2.33 | 2.70 |
| _{GlcNAc}Man₃ | 15.42 | 0 | 8.19 | 11.32 |
| ^{GlcNAc}Man₃ | 4.72 | 4.16 | 0.86 | 0 |
| _{GlcNAc}Man₃F | 0 | 0.96 | 0.77 | 0 |
| GlcNAc₂Man₃ | 1.04 | 13.26 | 12.04 | 6.66 |
| _{Gal}GlcNAcMan₃ | 3.77 | 3.61 | 9.85 | 0 |
| _{Gal}GlcNAcMan₃F | 0 | 0 | 0 | 0 |
| GalGlcNAc₂Man₃ | 2.53 | 2.18 | 1.57 | 0 |
| Gal₂GlcNAc₂Man₃ | 1.13 | 0 | 0.55 | 0 |
| GalGlcNAcMan₅ | 2.06 | 0 | 3.46 | 0 |
| _{Sia}GalGlcNAcMan₃ | 0 | 1.54 | 2.17 | 19.91 |
| Sia₂Gal₂GlcNAc₂Man₃ | 0 | 0 | 0 | 1.00 |
| SiaGalGlcNAcMan₅ | 0 | 0 | 2.47 | 18.34 |

The glycoproteins from the sialic acid-related gene transgenic silkworms comprising 3, 5 or 6 sialic acid-related genes were confirmed to have a sialic acid-terminated structure. These results revealed that at least three genes selected from the GNE, NANS, NANP, CMAS, and ST6GAL1 genes suffice as the number of sialic acid-related genes introduced necessary for attaching sialic acid to a galactose non-reducing terminal. Sialic acid attachment efficiency was significantly increased for the 6 genes compared with those of 3 genes or the 5 genes. The sialic acid-related gene that differs between the sialic acid-related gene expression vector of 5 genes (NANS/NANP/GNE/CAMS/ST6GAL1) and the sialic acid-related gene expression vector of 6 genes (NANS/NANP/SLC35A1/GNE/CAMS/ST6GAL1) prepared in Example 1 is the SLC35A1 gene. Thus, it was revealed that the SLC35A1 gene is not essential for attaching sialic acid to a galactose non-reducing terminal, but has a function of enhancing the attachment efficiency.

### (Analysis of sugar chain structure at glycosylation site)

Table 4 shows the relationship between sugar chain structures at two glycosylation sites (N187 and N224) of recombinant hATIII protein produced by glycosylation silkworms obtained by mating the GalT gene transgenic silkworm (GalT1, GalT2p, or GalT2i), the sialic acid-related gene transgenic silkworm (5 genes or 6 genes), and the hATIII gene transgenic silkworm. In the table, Man₀₋₄ represents a structure containing 0 to 4 mannoses at a sugar chain terminal, and Man₂₋₃F represents a structure containing 2 to 3 mannoses and 1 fucose modification. Sugar chains having GlcNAc at a non-reducing terminal are collectively shown as a GlcNAc structure. Sugar chains having Gal at a non-reducing terminal are collectively shown as a Gal structure. Sugar chains having Sia at a non-reducing terminal are collectively shown as a Sia structure. a and b represent silkworm lines. In the table, the numeric values indicate the ratio (%) of each sugar chain with respect to 100% in total of sugar chains detected. As for the notation of ATIII/GalT/sialic acid-related genes in the table, (+/-/-) represents the hATIII gene transgenic silkworm, and (+/-/5 genes) represents a transgenic silkworm obtained by mating the hATIII gene transgenic silkworm with the sialic acid-related gene transgenic silkworm of 5 genes without mating the GalT gene transgenic silkworm. Likewise, (+/GalT2p/-) represents a transgenic silkworm obtained by mating the hATIII gene transgenic silkworm with the GalT2p gene transgenic silkworm without mating the sialic acid-related gene transgenic silkworm. (+/GalT2p/5 genes) represents a transgenic silkworm obtained by mating the hATIII gene transgenic silkworm, the GalT2p gene transgenic silkworm and the sialic acid-related gene transgenic silkworm of 5 genes. The same holds true for the other notation.

**[Table 4]**

| ATIII | | + | | | | | | | | | |
|---|---|---|---|---|---|---|---|---|---|---|---|
| GalT | | - | | | GalT2p | | | | GalT2i | GalT1 | |
| Sialic acid-related gene | | | 5 genes | 6 genes | - | 5 genes | 6 genes | | 6 genes | | 6 genes |
| | | | | | | | a | b | | | |
| N187 | Man₀₋₄ | 30.5 | 32.0 | 32.0 | 20.4 | 27.5 | 3.3 | 16.2 | 16.7 | 24.7 | 16.8 |
| | Man₅ | 24.2 | 25.7 | 24.5 | 24.2 | 20.0 | 27.8 | 25.0 | 24.2 | 28.6 | 28.7 |
| | Man₆₋₉ | 33.4 | 32.0 | 35.3 | 41.4 | 31.2 | 47.5 | 48.7 | 47.1 | 31.0 | 37.6 |
| | Man₂₋₃F | 0 | 0 | 0 | 0 | 0 | 0 | 0 | 0 | 0 | 0 |
| | GlcNAc | 11.9 | 10.5 | 8.3 | 14.0 | 14.7 | 8.5 | 4.5 | 8.1 | 15.7 | 4.5 |
| | Gal | 0 | 0 | 0 | 0 | 0.9 | 2.7 | 2.0 | 0 | 0 | 0 |
| | Sia | 0 | 0 | 0 | 0 | 5.8 | 10.1 | 3.5 | 3.9 | 0 | 12.3 |
| N224 | Man₀₋₄ | 64.3 | 72.5 | 73.3 | 28.5 | 30.4 | 7.3 | 21.5 | 26.5 | 25.4 | 22.6 |
| | Man₅ | 4.3 | 2.8 | 3.3 | 11.1 | 13.0 | 13.6 | 7.3 | 20.0 | 6.9 | 9.6 |
| | Man₆₋₉ | 8.0 | 1.8 | 1.5 | 6.0 | 3.2 | 9.4 | 3.7 | 6.6 | 0 | 0 |
| | Man₂₋₃F | 7.5 | 4.2 | 9.4 | 0 | 1.0 | 0.3 | 0 | 0 | 0 | 0 |
| | GlcNAc | 15.9 | 18.7 | 12.3 | 36.3 | 27.0 | 21.9 | 19.2 | 27.7 | 29.8 | 17.5 |
| | Gal | 0 | 0 | 0 | 18.0 | 18.1 | 19.9 | 26.7 | 12.6 | 37.7 | 18.0 |
| | Sia | 0 | 0 | 0 | 0 | 7.3 | 29.5 | 22.7 | 6.8 | 0 | 32.4 |

Both the sugar chain-attached asparagine residues N187 and N224 had sialic acid attached to a non-reducing terminal, as in mammals, only in the transgenic silkworms comprising the GalT (GalT2p, GalT2i or GalT1) expression vector and the sialic acid-related gene expression vector of 5 genes or 6 genes. The efficiency was higher when GalT2p or GalT2i was used than when GAlT1 was used.

### (2) Sugar chain structure analysis of human interferon γ

For human interferon γ (hIFNγ), the central band indicated by ^{∗} in Figure 4 was designated as a first molecular species, and the band with the slowest mobility indicated by ^{∗∗} was designated as a second molecular species.

The human interferon γ is known to have two asparagine residues (N48 and N120) that undergo glycosylation. Table 5 shows sugar chain structures at the glycosylation sites N48 and N120 in the first and second molecular species of recombinant hIFNγ protein obtained from glycosylation silkworms obtained by mating the GalT2p gene transgenic silkworm, the sialic acid-related gene transgenic silkworm (5 genes or 6 genes), and the hIFNγ gene transgenic silkworm. In the table, the numeric values indicate the ratio (%) of each sugar chain with respect to 100% in total of sugar chains detected. a and b represent silkworm lines. As for the notation of IFNy/GalT2p/sialic acid-related genes in the table, (+/-/-) represents the hIFNγ gene transgenic silkworm, and (+/-/5 genes) represents a transgenic silkworm obtained by mating the hIFNγ gene transgenic silkworm with the sialic acid-related gene transgenic silkworm of 5 genes without mating the GalT2p gene transgenic silkworm. Likewise, (+/+/-) represents a transgenic silkworm obtained by mating the hIFNγ gene transgenic silkworm with the GalT2p gene transgenic silkworm without mating the sialic acid-related gene transgenic silkworm. (+/+/5 genes or 6 genes) represents a transgenic silkworm obtained by mating the GalT2p gene transgenic silkworm, the hIFNγ gene transgenic silkworm, and the sialic acid-related gene transgenic silkworm of 5 genes or 6 genes.

Table 6 shows excerpts of the sugar chain structures directly related to the present invention and the ratios (%) thereof from Table 5.

Table 7 shows top 4 abundance ratios of the sugar chain structures detected in each molecular species. The transgenic silkworm for control in this table is a hIFNγ gene transgenic silkworm obtained without mating the GalT gene transgenic silkworm and the sialic acid-related gene transgenic silkworm. Thus, in the transgenic silkworm for control, substantially a wild-type silkworm sugar chain was attached.

**[Table 5]**

| IFNγ | | + | | | | | | | | | |
|---|---|---|---|---|---|---|---|---|---|---|---|
| GalT2p | | - | | | | + | | | | | |
| Sialic acid-related gene | | - | | 5 genes | | - | | 5 genes | | 6 genes | |
| | | a | b | a | b | a | b | a | b | a | b |
| N48 | Man₀₋₄ | 39.5 | 44.1 | 36.6 | 24.5 | 20.6 | 12.1 | 18.2 | 8.7 | 9.4 | 3.9 |
| | Man₅ | 0 | 0 | 0 | 0 | 5.9 | 5.9 | 10.7 | 15.3 | 18.0 | 13.0 |
| | Man₆₋₉ | 0 | 0 | 0 | 0 | 0 | 0 | 0 | 0 | 0 | 0 |
| | Man₂₋₃F | 16.8 | 15.4 | 19.6 | 14.8 | 0 | 1.3 | 1.4 | 1.0 | 1.8 | 0 |
| | GlcNAc | 43.6 | 40.5 | 44.0 | 60.8 | 42.9 | 42.2 | 17.5 | 20.0 | 16.6 | 7.5 |
| | Gal | 0 | 0 | 0 | 0 | 30.7 | 38.5 | 5.7 | 9.2 | 7.4 | 11.1 |
| | Sia | 0 | 0 | 0 | 0 | 0 | 0 | 46.5 | 45.8 | 46.9 | 64.5 |
| N120 | Man₀₋₄ | 57.4 | 46.2 | 34.8 | 20.1 | 15.5 | 9.8 | 14.2 | 6.6 | 15.0 | 7.8 |
| | Man₅ | 26.2 | 31.8 | 38.3 | 42.3 | 41.5 | 39.9 | 44.9 | 38.0 | 44.8 | 41.4 |
| | Man₆₋₉ | 4.3 | 7.3 | 14.3 | 23.1 | 25.3 | 29.9 | 25.6 | 40.0 | 14.1 | 32.4 |
| | Man₂₋₃F | 0.3 | 0 | 0 | 0 | 0 | 0 | 0 | 0 | 0 | 0 |
| | GlcNAc | 11.7 | 14.6 | 12.7 | 14.5 | 9.7 | 9.6 | 6.0 | 4.8 | 5.3 | 2.6 |
| | Gal | 0 | 0 | 0 | 0 | 7.8 | 10.8 | 2.8 | 4.0 | 0 | 0 |
| | Sia | 0 | 0 | 0 | 0 | 0 | 0 | 6.5 | 6.7 | 20.8 | 15.8 |

**[Table 6]**

| | Attached sugar chain | N48 | N120 |
|---|---|---|---|
| First molecular species | Sia | 46.5 | 6.5 |
| | Only Gal | 5.7 | 2.8 |
| | GlcNAc | 17.5 | 6.0 |
| Second molecular species | Sia | 45.8 | 6.7 |
| | Only Gal | 9.2 | 4.0 |
| | GlcNAc | 20.0 | 4.8 |

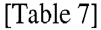

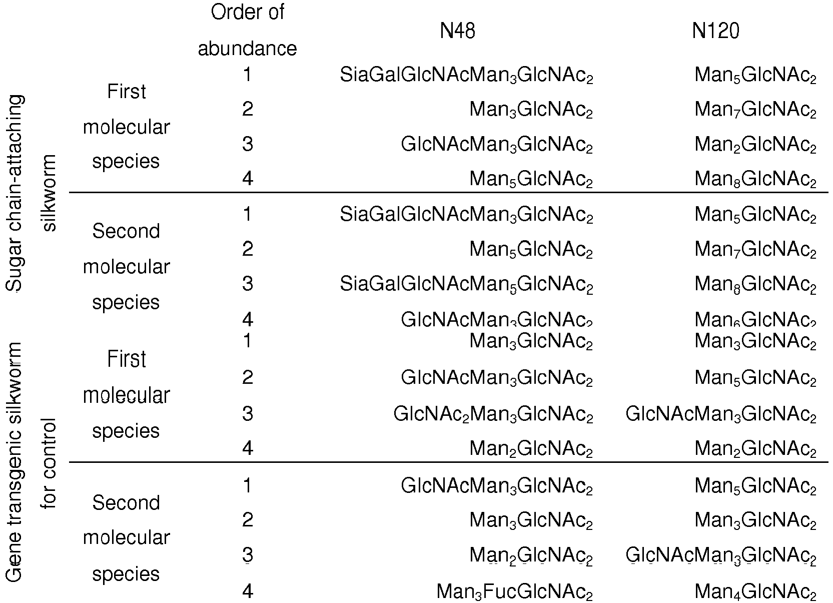

As seen from these results, the sugar chain-attached asparagine residues N48 and N120 in hIFNγ also had a sialic acid-containing mammalian-type sugar chain attached to a non-reducing terminal in the transgenic silkworms comprising both of the GalT2 expression vector and the sialic acid-related gene expression vector. Thus, use of the glycosylation silkworm of the present invention enables a mammalian-type sugar chain to be attached to the protein of interest produced in a silk gland of the silkworm.

## Claims

1. A mammalian-type glycosylation agent, which attaches a sugar chain with sialic acid at a non-reducing terminal to a protein via its asparagine residue, comprising one to three independent expression vector(s) comprising
a silk-spinning insect-derived middle and/or posterior silk gland promoter and
a gene encoding β1,4-galactosyltransferase or a nucleotide encoding an active fragment of the enzyme, functionally linked downstream of the promoter, and
genes encoding
UDP-acetylglucosamine 2-epimerase/N-acetylmannosamine kinase,
α2,6-sialyltransferase,
CMP-Neu5Ac synthase, and
Neu5Ac9-phosphate synthase and/or Neu5Ac9-phosphate phosphatase, or
nucleotides encoding active fragments of the enzymes, wherein
the genes encoding the enzymes or the nucleotides encoding active fragments of the enzymes are arranged so as to be under direct or indirect expression control of the middle and/or posterior silk gland promoter.

2. The mammalian-type glycosylation agent according to claim 1, wherein the β1,4-galactosyltransferase is GalT2.

3. The mammalian-type glycosylation agent according to claim 1 or 2, wherein the middle silk gland promoter is a promoter of sericin 1 gene, sericin 2 gene, or sericin 3 gene.

4. The mammalian-type glycosylation agent according to claim 1 or 2, wherein the posterior silk gland promoter is a promoter of fibroin H chain gene, fibroin L chain gene, or p25 gene.

5. The mammalian-type glycosylation agent according to any one of claims 1 to 4, wherein the expression vector(s) further comprises
a gene encoding CMP-Neu5Ac transporter, or
a nucleotide encoding an active fragment of the enzyme.

6. The mammalian-type glycosylation agent according to claim 5, wherein the expression vector(s) comprises
genes encoding β1,4-galactosyltransferase, UDP-acetylglucosamine 2-epimerase/N-acetylmannosamine kinase, α2,6-sialyltransferase, Neu5Ac9-phosphate synthase, Neu5Ac9-phosphate phosphatase, CMP-Neu5Ac transporter, and CMP-Neu5Ac synthase, or
nucleotides encoding active fragments of the enzymes.

7. The mammalian-type glycosylation agent according to any one of claims 1 to 4, wherein the expression vectors consist of
a first expression vector comprising the gene encoding β1,4-galactosyltransferase or the nucleotide encoding an active fragment of the enzyme, and
a second expression vector comprising the genes of UDP-acetylglucosamine 2-epimerase/N-acetylmannosamine kinase, α2,6-sialyltransferase, CMP-Neu5Ac synthase, and Neu5Ac9-phosphate synthase and/or Neu5Ac9-phosphate phosphatase, or the nucleotides encoding active fragments of the enzymes.

8. The mammalian-type glycosylation agent according to claim 7, wherein
the gene encoding CMP-Neu5Ac transporter, or
the nucleotide encoding an active fragment of the enzyme
is comprised in the second expression vector.

9. The mammalian-type glycosylation agent according to any one of claims 1 to 6, wherein the genes encoding the enzymes or the nucleotides encoding active fragments of the enzymes are functionally linked downstream of the middle and/or posterior silk gland promoter.

10. The mammalian-type glycosylation agent according to any one of claims 1 to 8, wherein the expression vector(s) is constituted by
(i) a first subunit comprising the middle and/or posterior silk gland promoter and a gene encoding a transcriptional control element, functionally linked downstream of the promoter, and
(ii) one or more second subunit(s) comprising a target promoter of the transcriptional control element and a gene(s) encoding one or more enzyme(s) selected from the group consisting of β1,4-galactosyltransferase, UDP-acetylglucosamine 2-epimerase/N-acetylmannosamine kinase, α2,6-sialyltransferase, Neu5Ac9-phosphate synthase, Neu5Ac9-phosphate phosphatase, CMP-Neu5Ac transporter, and CMP-Neu5Ac synthase, or a nucleotide(s) encoding an active fragment of the enzyme(s), functionally linked downstream of the promoter.

11. The mammalian-type glycosylation agent according to claim 10, wherein the transcriptional control element is yeast-derived GAL4 protein, and the target promoter thereof is UAS (upstream activating sequence).

12. A transgenic silk-spinning insect capable of glycosylation with a mammalian-type sugar chain with attached sialic acid at a non-reducing terminal, comprising an expression vector(s) constituting a mammalian-type glycosylation agent according to any one of claims 1 to 9.

13. The transgenic silk-spinning insect according to claim 12, wherein the expression vector(s) constitute a mammalian-type glycosylation agent according to claim 10 or 11.

14. A line producing a transgenic silk-spinning insect capable of glycosylation with a mammalian-type sugar chain with attached sialic acid at a non-reducing terminal, comprising only a second subunit(s) of an expression vector(s) constituting a mammalian-type glycosylation agent according to claim 10 or 11.

15. A transgenic silk-spinning insect capable of glycosylation with a mammalian-type sugar chain with attached sialic acid at a non-reducing terminal, prepared by the following step;
a mating step of mating a line producing a transgenic silk-spinning insect capable of glycosylation with a mammalian-type sugar chain according to claim 14, and a transgenic silk-spinning insect-producing line of the same species thereas having a first subunit(s) of an expression vector(s) constituting a mammalian-type glycosylation agent according to claim 10 or 11; and
a selection step of selecting a transgenic silk-spinning insect comprising the first subunit and the second subunit as the transgenic silk-spinning insect capable of glycosylation with a mammalian-type sugar chain from a first filial generation (F1).

## Patentansprüche

1. Glykosylierungsmittel vom Säugetiertyp, das eine Zuckerkette mit Sialinsäure an einem nichtreduzierenden Ende an ein Protein über seinen Asparaginrest bindet, umfassend einen bis drei unabhängige Expressionsvektoren, umfassend
einen von einem seidenspinnenden Insekt abgeleiteten Promotor der mittleren und/oder der hinteren Seidendrüse und
ein Gen, das für β1,4-Galactosyltransferase kodiert, oder ein Nukleotid, das für ein aktives Fragment des Enzyms kodiert, das stromabwärts des Promotors funktionell verknüpft ist, und
Gene, die kodieren für UDP-Acetylglucosamin-2-epimerase/N-Acetylmannosaminkinase,
α2,6-Sialyltransferase,
CMP-Neu5Ac-Synthase und
Neu5Ac9-Phosphatsynthase und/oder Neu5Ac9-Phosphatphosphatase, oder
Nukleotide, die für aktive Fragmente der Enzyme kodieren, wobei
die für die Enzyme kodierenden Gene oder die für aktive Fragmente der Enzyme kodierenden Nukleotide so angeordnet sind, dass sie unter direkter oder indirekter Expressionskontrolle des mittleren und/oder hinteren Seidendrüsenpromotors stehen.

2. Glykosylierungsmittel vom Säugetiertyp nach Anspruch 1, wobei die β1,4-Galactosyltransferase GalT2 ist.

3. Glykosylierungsmittel vom Säugetiertyp nach Anspruch 1 oder 2, wobei der Promotor der mittleren Seidendrüse ein Promotor des Sericin-1-Gens, des Sericin-2-Gens oder des Sericin-3-Gens ist.

4. Glykosylierungsmittel vom Säugetiertyp nach Anspruch 1 oder 2, wobei der Promotor der hinteren Seidendrüse ein Promotor des Fibroin-H-Ketten-Gens, des Fibroin-L-Ketten-Gens oder des p25-Gens ist.

5. Glykosylierungsmittel vom Säugetiertyp nach einem der Ansprüche 1 bis 4, wobei der eine oder die mehreren Expressionsvektoren ferner Folgendes umfasst:
ein Gen, das für den CMP-Neu5Ac-Transporter kodiert, oder
ein Nukleotid, das für ein aktives Fragment des Enzyms kodiert.

6. Glykosylierungsmittel vom Säugetiertyp nach Anspruch 5, wobei der eine oder die mehreren Expressionsvektoren Folgendes umfassen:
Gene, die für β1,4-Galactosyltransferase, UDP-Acetylglucosamin-2-epimerase/N-Acetylmannosaminkinase, α2,6-Sialyltransferase, Neu5Ac9-Phosphatsynthase, Neu5Ac9-Phosphatphosphatase, CMP-Neu5Ac-Transporter und CMP-Neu5Ac-Synthase kodieren oder
Nukleotide, die für aktive Fragmente der Enzyme kodieren.

7. Glykosylierungsmittel vom Säugetiertyp nach einem der Ansprüche 1 bis 4, wobei die Expressionsvektoren aus Folgendem bestehen:
aus einem ersten Expressionsvektor, umfassend das Gen, das für β1,4-Galactosyltransferase kodiert, oder das Nukleotid, das für ein aktives Fragment des Enzyms kodiert, und
aus einem zweiten Expressionsvektor, umfassend die Gene von UDP-Acetylglucosamin-2-epimerase/N-Acetylmannosaminkinase, α2,6-Sialyltransferase, CMP-Neu5Ac-Synthase und Neu5Ac9-Phosphatsynthase und/oder Neu5Ac9-Phosphatphosphatase, oder die Nukleotide, die für aktive Fragmente der Enzyme kodieren.

8. Glykosylierungsmittel vom Säugetiertyp nach Anspruch 7, wobei:
das Gen, das für den CMP-Neu5Ac-Transporter kodiert, oder
das Nukleotid, das für ein aktives Fragment des Enzyms kodiert, in dem zweiten Expressionsvektor enthalten ist.

9. Glykosylierungsmittel vom Säugetiertyp nach einem der Ansprüche 1 bis 6, wobei die Gene, die für die Enzyme kodieren, oder die Nukleotide, die für aktive Fragmente der Enzyme kodieren, stromabwärts des mittleren und/oder des hinteren Seidendrüsenpromotors funktionell verknüpft sind.

10. Glykosylierungsmittel vom Säugetiertyp nach einem der Ansprüche 1 bis 8, wobei der eine oder die mehreren Expressionsvektoren ausgebildet werden durch
(i) eine erste Untereinheit, umfassend den mittleren und/oder den hinteren Seidendrüsenpromotor und ein Gen, das für ein Transkriptionskontrollelement kodiert, stromabwärts des Promotors funktionell verknüpft, und
(ii) eine oder mehrere zweite Untereinheiten, umfassend einen Zielpromotor des Transkriptionskontrollelements und ein oder mehrere Gene, die für ein oder mehrere Enzyme kodieren, ausgewählt aus der Gruppe bestehend aus β1,4-Galactosyltransferase, UDP-Acetylglucosamin-2-epimerase/N-Acetylmannosaminkinase, α2,6-Sialyltransferase, Neu5SAc9-Phosphatsynthase, Neu5Ac9-Phosphatphosphatase, CMP-Neu5Ac-Transporter und CMP-Neu5Ac-Synthase, oder ein oder mehrere Nukleotide, die für ein aktives Fragment des einen oder der mehreren Enzyme kodieren, stromabwärts des Promotors funktionell verknüpft.

11. Glykosylierungsmittel vom Säugertyp nach Anspruch 10, wobei das Transkriptionskontrollelement von Hefe abgeleitetes GAL4-Protein ist und der Zielpromotor davon UAS (Upstream-Aktivierungssequenz) ist.

12. Transgenes seidenspinnendes Insekt, das zur Glykosylierung mit einer Zuckerkette vom Säugertyp mit angehängter Sialinsäure an einem nichtreduzierenden Ende fähig ist, umfassend einen oder mehrere Expressionsvektoren, die ein Glykosylierungsmittel vom Säugertyp nach einem der Ansprüche 1 bis 9 ausbilden.

13. Transgenes seidenspinnendes Insekt nach Anspruch 12, wobei der oder die Expressionsvektoren ein Glykosylierungsmittel vom Säugetiertyp nach Anspruch 10 oder 11 ausbilden.

14. Linie, die ein transgenes seidenspinnendes Insekt erzeugt, das zur Glykosylierung mit einer Zuckerkette vom Säugertyp mit angehängter Sialinsäure an einem nichtreduzierenden Ende fähig ist, umfassend nur einen oder mehrere zweite Untereinheiten eines oder mehrerer Expressionsvektoren, die ein Glykosylierungsmittel vom Säugertyp nach Anspruch 10 oder 11 ausbilden.

15. Transgenes seidenspinnendes Insekt, das zur Glykosylierung mit einer Zuckerkette vom Säugertyp mit angehängter Sialinsäure an einem nichtreduzierenden Ende fähig ist, erzeugt durch den folgenden Schritt;
einen Paarungsschritt zum Paaren einer Linie, die ein transgenes seidenspinnendes Insekt erzeugt, das zur Glykosylierung mit einer Zuckerkette vom Säugertyp nach Anspruch 14 fähig ist, mit einer Linie, die ein transgenes seidenspinnendes Insekt derselben Spezies erzeugt, wobei eine oder mehrere erste Untereinheiten eines oder mehrerer Expressionsvektoren ein Glykosylierungsmittel vom Säugertyp nach Anspruch 10 oder 11 ausbilden; und
einen Auswahlschritt zum Auswählen eines transgenen seidenspinnenden Insekts, das die erste Untereinheit und die zweite Untereinheit umfasst, als das transgene seidenspinnende Insekt, das zur Glykosylierung mit einer Zuckerkette vom Säugertyp aus einer ersten Filialgeneration (F1) fähig ist.

## Revendications

1. Agent de glycosylation de type mammalien qui attache une chaîne glucidique, avec un acide sialique au niveau d'un terminal non réducteur, à une protéine via son résidu d'asparagine, comprenant d'un à trois vecteur(s) d'expression indépendant(s) comprenant
un promoteur de la glande séricigène moyenne et/ou postérieure dérivée d'un insecte fileur de soie et
un gène codant pour une β1,4-galactosyl-transférase ou un nucléotide codant pour un fragment actif de l'enzyme, étant fonctionnellement lié en aval du promoteur, et
des gènes codant pour
une UDP-acétyl-glucosamine 2-épimérase/N-acétyl-mannosamine kinase,
une α2,6-sialyl-transférase,
une CMP-Neu5Ac synthase et
une Neu5Ac9-phosphate synthase et/ou une Neu5Ac9-phosphate phosphatase ou bien
des nucléotides codant pour des fragments actifs des enzymes, dans lequel
les gènes codant pour les enzymes ou les nucléotides codant pour des fragments actifs des enzymes sont arrangés de sorte à être sous un contrôle direct ou indirect de l'expression du promoteur de la glande séricigène moyenne et/ou postérieure.

2. Agent de glycosylation de type mammalien selon la revendication 1, dans lequel la β1,4-galactosyl-transférase est la GalT2.

3. Agent de glycosylation de type mammalien selon les revendications 1 ou 2, dans lequel le promoteur de la glande séricigène moyenne est un promoteur du gène de la séricine 1, du gène de la séricine 2 ou du gène de la séricine 3.

4. Agent de glycosylation de type mammalien selon les revendications 1 ou 2, dans lequel le promoteur de la glande séricigène postérieure est un promoteur du gène de la chaîne H de la fibroïne, du gène de la chaîne L de la fibroïne ou du gène p25.

5. Agent de glycosylation de type mammalien selon l'une quelconque des revendications 1 à 4, dans lequel le(s) vecteur(s) d'expression comprend/comprennent en outre
un gène codant pour un transporteur de CMP-Neu5Ac ou
un nucléotide codant pour un fragment actif de l'enzyme.

6. Agent de glycosylation de type mammalien selon la revendication 5, dans lequel le(s) vecteur(s) d'expression comprend/comprennent
des gènes codant pour les β1,4-galactosyl-transférase, UDP-acétyl-glucosamine 2-épimérase/N-acétyl-mannosamine kinase, α2,6-sialyl-transférase, Neu5Ac9-phosphate synthase, Neu5Ac9-phosphate phosphatase, transporteur de CMP-Neu5Ac et CMP-Neu5Ac synthase ou bien
des nucléotides codant pour des fragments actifs des enzymes.

7. Agent de glycosylation de type mammalien selon l'une quelconque des revendications 1 à 4, dans lequel les vecteurs d'expression sont constitués par
un premier vecteur d'expression comprenant le gène codant pour une β1,4-galactosyl-transférase, ou le nucléotide codant pour un fragment actif de l'enzyme, et
un deuxième vecteur d'expression comprenant les gènes codant pour les UDP-acétyl-glucosamine 2-épimérase/N-acétyl-mannosamine kinase, α2,6-sialyl-transférase, CMP-Neu5Ac synthase et Neu5Ac9-phosphate synthase et/ou Neu5Ac9-phosphate phosphatase ou bien les nucléotides codant pour des fragments actifs des enzymes.

8. Agent de glycosylation de type mammalien selon la revendication 7, dans lequel
le gène codant pour un transporteur de CMP-Neu5Ac, ou
le nucléotide codant pour un fragment actif de l'enzyme,
est compris dans le deuxième vecteur d'expression.

9. Agent de glycosylation de type mammalien selon l'une quelconque des revendications 1 à 6, dans lequel les gènes codant pour les enzymes, ou les nucléotides codant pour des fragments actifs des enzymes, sont fonctionnellement liés en aval du promoteur de la glande séricigène moyenne et/ou postérieure.

10. Agent de glycosylation de type mammalien selon l'une quelconque des revendications 1 à 8, dans lequel le(s) vecteur(s) d'expression est/sont constitué(s) par
(i) une première sous-unité comprenant le promoteur de la glande séricigène moyenne et/ou postérieure et un gène codant pour un élément de contrôle de la transcription, étant fonctionnellement lié en aval du promoteur, et
(ii) une ou plusieurs deuxième(s) sous-unité(s) comprenant un promoteur cible de l'élément de contrôle de la transcription et un/des gène(s) codant pour une ou plusieurs enzyme(s) choisie(s) dans le groupe constitué par les β1,4-galactosyl-transférase, UDP-acétyl-glucosamine 2-épimérase/N-acétyl-mannosamine kinase, α2,6-sialyl-transférase, Neu5Ac9-phosphate synthase, Neu5Ac9-phosphate phosphatase, transporteur de CMP-Neu5Ac et CMP-Neu5Ac synthase, ou un/des nucléotide(s) codant pour un fragment actif de l'enzyme/des enzymes, étant fonctionnellement lié(s) en aval du promoteur.

11. Agent de glycosylation de type mammalien selon la revendication 10, dans lequel l'élément de contrôle de la transcription est la protéine GAL4 dérivée de la levure et le promoteur cible de celui-ci est l'UAS (séquence d'activation en amont).

12. Insecte fileur de soie transgénique capable de réaliser une glycosylation avec une chaîne glucidique de type mammalien, avec un acide sialique attaché au niveau d'un terminal non réducteur, comprenant un/des vecteur(s) d'expression constituant un agent de glycosylation de type mammalien selon l'une quelconque des revendications 1 à 9.

13. Insecte fileur de soie transgénique selon la revendication 12, dans lequel le(s) vecteur(s) d'expression constitue/constituent un agent de glycosylation de type mammalien selon les revendications 10 ou 11.

14. Lignée produisant un insecte fileur de soie transgénique capable de réaliser une glycosylation avec une chaîne glucidique de type mammalien, avec un acide sialique attaché au niveau d'un terminal non réducteur, comprenant seulement une/des deuxième(s) sous-unité(s) d'un/de vecteur(s) d'expression constituant un agent de glycosylation de type mammalien selon les revendications 10 ou 11.

15. Insecte fileur de soie transgénique capable de réaliser une glycosylation avec une chaîne glucidique de type mammalien, avec un acide sialique attaché au niveau d'un terminal non réducteur, préparé par les étapes suivantes ;
une étape d'accouplement consistant à accoupler une lignée produisant un insecte fileur de soie transgénique capable de réaliser une glycosylation avec une chaîne glucidique de type mammalien, selon la revendication 14, et une lignée transgénique produisant un insecte fileur de soie transgénique de la même espèce que celle-là ayant une/des première(s) sous-unité(s) d'un/de vecteur(s) d'expression constituant un agent de glycosylation de type mammalien selon les revendications 10 ou 11 ; et
une étape de sélection consistant à sélectionner un insecte fileur de soie transgénique comprenant la première sous-unité et la deuxième sous-unité comme étant l'insecte transgénique fileur de soie capable de réaliser une glycosylation avec une chaîne glucidique de type mammalien provenant d'une première génération filiale (F1).
